# EUROPEAN PATENT SPECIFICATION

(11) **EP 2 044 221 B1**
(45) Date of publication and mention of the grant of the patent: **22.07.2015**
(21) Application number: 07813223.0
(22) Date of filing: 23.07.2007
(51) Int. Cl.: C12Q 1/68

(54) **METHODS AND KIT FOR ANALYSES OF METHYLATION IN COLORECTAL CELLULAR PROLIFERATIVE DISORDERS**
VERFAHREN UND KIT ZUR ANALYSE DER METHYLIERUNG IN KOLOREKTALEN PROLIFERATIVEN ZELLERKRANKUNGEN
PROCÉDÉS ET TROUSSE DESTINÉS AUX ANALYSES DE LA METHYLATION DES MALADIES ASSOCIÉES À UNE PROLIFÉRATION CELLULAIRE COLORECTALE

(30) Priority: 21.07.2006 US 832509 P; 20.10.2006 US 853097 P
(43) Date of publication of application: 08.04.2009
(60) Divisional application: 13154538.6
(73) Proprietor: Epigenomics AG, 10829 Berlin (DE)
(72) Inventor: LOFTON-DAY, Catherine, Seattle, WA 98103 (US); SLEDZIEWSKI, Andrew, Shoreline, Washington 98177 (US); MODEL, Fabian, 12683 Berlin (DE); COTTRELL, Susan, Seattle, Washington 98115 (US); DISTLER, Juergen, 12163 Berlin (DE); TETZNER, Reimo, 10439 Berlin (DE); DIETRICH, Dimo, 10437 Berlin (DE)
(74) Representative: Zwicker, Jörk
(86) International application number: PCT/US2007/074106
(87) International publication number: WO 2008/011620

(56) References cited:
- WO-A-2005/123945
- WO-A-2006/008128
- WO-A-2006/113466
- WO-A-2007/008693
- WO-A-2007/009755
- WO-A-2007/039234
- HESSON L B ET AL: "CpG island promoter hypermethylation of a novel Ras-effector gene RASSF2A is an arly event in colon carcinogenesis and correlates inversely with K-ras mutations" ONCOGENE, BASINGSTOKE, HANTS, GB, vol. 24, 4 April 2005 (2005-04-04), pages 3987-3994, XP003013793 ISSN: 0950-9232
- ENDOH M ET AL: "RASSF2, a potential tumour suppressor, is silenced by CpG island hypermethylation in gastric cancer" BRITISH JOURNAL OF CANCER, vol. 93, no. 12, December 2005 (2005-12), pages 1395-1399, XP002458486 ISSN: 0007-0920
- NOSHO KATSUHIKO ET AL: "Genetic and epigenetic profiling in early colorectal tumors and prediction of invasive potential in pT1 (early invasive) colorectal cancers." CARCINOGENESIS JUN 2007, vol. 28, no. 6, June 2007 (2007-06), pages 1364-1370, XP002458487 ISSN: 0143-3334
- BURROWS J F ET AL: "Altered expression of the septin gene, SEPT9, in ovarian neoplasia" JOURNAL OF PATHOLOGY, CHICHESTER, SUSSEX, GB, vol. 201, no. 4, December 2003 (2003-12), pages 581-588, XP002402384 ISSN: 0022-3417
- SCOTT MICHAEL ET AL: "Multimodality expression profiling shows SEPT9 to be overexpressed in a wide range of human tumours." ONCOGENE 7 JUL 2005, vol. 24, no. 29, 7 July 2005 (2005-07-07), pages 4688-4700, XP002462084 ISSN: 0950-9232
- DEVOS T ET AL: "Methylated Septin 9 DNA: a biomarker found in blood plasma, for the detection of colorectal cancer." CLINICAL CHEMISTRY, vol. 53, no. 6, Suppl. S, June 2007 (2007-06), page A109, XP001537814 & 59TH ANNUAL MEETING OF THE AMERICAN-ASSOCIATION-FOR-CLINICAL-CHEMIST RY; SAN DIEGO, CA, USA; JULY 15 -19, 2007 ISSN: 0009-9147
- Thierry Lecomte ET AL: "Detection of free-circulating tumor-associated DNA in plasma of colorectal cancer patients and its association with prognosis", International Journal of Cancer, vol. 100, no. 5, 10 August 2002 (2002-08-10), pages 542-548, XP055071717, ISSN: 0020-7136, DOI: 10.1002/ijc.10526

### SEQUENCE LISTING

<110> Epigenomics AG
<120> METHODS AND NUCLEIC ACIDS FOR ANALYSERS OF CELLULAR PROLIFERATIVE DISORDERS
<130> 0781322301404
<140> EP 2044221
   <141> 2007-07-23
<150> US 60/823,509
   <151> 2006-07-21
<150> US 60/853,097
   <151> 2006-10-20
<160> 70
<170> PatentIn version 3.5
<210> 1
<400> 1
   000
<210> 2
<400> 2
   000
<210> 3
<400> 3
   000
<210> 4
<400> 4
   000
<210> 5
<400> 5
   000
<210> 6
<400> 6
   000
<210> 7
   <211> 1920
   <212> DNA
   <213> Homo sapiens
<400> 7
<210> 13
   <211> 1405
   <212> DNA
   <213> Homo sapiens
<400> 13
   cgttacccga gttgtaaagg gcggctccct gtgtctgccc cgctgcaccg atacaccgag      60
   ctgcgcacgg tgcccagcgc agggagaaca aatgatcatc tgtccaacgc gcccatttac     120
   aggtgaggaa actaaggctc caactcaatc gacgcactct gcccttttga ttaccagaaa     180
   agtagcagga caggtgtcct gtcccgccct accccggccc actaagccgg caccccggct     240
   ccgacccccg gctgtgcccg gcgccgccgc ggtgcccggc gccgccgcct cgcccggcgg     300
   ggccgcccgg agcgcccgca cctccgcccg cttccacctg gccgggcccg ccccgcccgg     360
   actcgggact gggaagtgcg gcgactcccg gaaccagcca ttggcgccag cgcggggagc     420
   tgggggtgca gagctgcggg cgcggcgggc cacgcaggcg gcccccaccc ccggcctggc     480
   ctggtctggt ctggtctgcg ctgccgcgcg ggggcgcccc ctcccaggcc cggcgcccgc     540
   cagccccgct ccgccaggtg cagcgcagcg caggggtggg cgggggtggg gctcggcgcg     600
   cacgttcacg gggcggggag ggggcgggtc aggggcggga ccacagccgg ctgggccggg     660
   gttctatgcg catctccggg gaggggcggg gcgggggcgg ggccggggcg gggcccggtc     720
   ggtgcactcc agacggcggg ccgccccctc ttcccgcctt cctactaccg gcccaggatt     780
   agcgccctgg gagcgcgcgc cccgctgcct cgccgccaca ctttcctggg agcggcggcc     840
   acggaggcac catgaagaag tcttactcag gtgggcttcg cgcccggggt ggggaggggt     900
   cggtgtcccg ggaccagcgc tgctcacctg agtgcctgcg gccgggagtg gcgaggcgcc     960
   cccggagctg agcgagtccc cgcggcgggc acactgcagg tcgagttcct cccaggacag    1020
   ggccgctgtc gggccgcttt cgacctgagc cgaccgtccc ctgcgctgtc tccagccctt    1080
   gctcgagtgt cggaggggct gccctggggg acgctccctc ttcctcgccc cttgcaccct    1140
   cgcaggaatc gctgactttc caggtcggcc gggtgctttg ggtccctgtg cgtctgtgtg    1200
   ggtgaatggg gtcggggcta ggtggagggg tgtccttggg ttcagcctct agggctggtg     1260
   gtccaggccg cagcatcctt tcttcggatt ctcttcggtt tctcctctac ttagtggggc    1320
   acgggacggc ctccagatgg gaccgtccag cagcgcccaa acttggcgac tcgggttcac    1380
   gttttgcgct caggacgccg cccgc                                           1405
<210> 14
   <211> 1818
   <212> DNA
   <213> Homo sapiens
<400> 14
   cgtcgttcgt ttttggtttt tttgatagtc gtgttttatt ttcgttttgt gttttttttt      60
   tcggatagtg ttttttttag ggtttattta ggagggtgta gcggtggttt tcggggcggt     120
   ggtcgtggtg ggggtgttag ttgtaggggt gttttcggtg ggtgggagtt ggtggttttt     180
   cgttggtgtt atgggattcg tatgttcgtt ttgcgttttt cggtttttga gtttataggt     240
   cgggattttg tttgttagtc gcgtgcgttg tcgtttaatt tttgtaggcg tagagcgcgc     300
   ggcggcggtg atagagaatt ttgtttggtt gtttaaatat agttttttgt agaaggattt      360
   tgcgttcggg gaaggggagg aatttttttt tttttgggcg ttcgtttttt tcgttatggt     420
   tcggttttta tattcgttta tatttggtcg tagcggggcg ttcgggggga ggggttgagg     480
   tcgcgttttt cgtcgttttt tgggcgcggg ttaggcgggg aggagggggg cgtttcggtc      540
   gtgtgtttag gattgttttt tagcggttat tcgggtttta gttttttagg tttggttttg     600
   ataggcgggc ggagtagtta gtgcgagata gggaggtcgg tgcgggtgcg ggaatttgat     660
   tcgttcggga ggcgggggcg gggcgggggc gtagcgcgcg gggaggggtc ggcgttcgtt       720
   tttttttttt atttatttag ttgagttagg gggtttaggg gttttttcgg cggttagttt     780
   tgtattgtag gagcgcgggc gcggcgtttt agttagcgcg tagggttcgg gtttcgtcgg     840
   gggcgttttt tcgtcgttgt ttttcgcgcg attcgttgtt tattagttat tatgtcggat     900
   ttcgcggtta acgcgtagtt ggatgggatt atttcggatt tcgaaggtgg gtgttgggtt     960
   ggttgttgcg gtcgcggacg tgttggagag gattttgcgg gtgggtttgg cgcgggacgg    1020
   gggtgcgttg aggggagacg ggagtgcgtt gaggggagac gggattttta atttaggcgt     1080
   tttttcgttg agagcgtcgc gcgttttcgg tttcgtgttc gcgtcgttta cgtgggggat    1140
   tttgttaggg gtattcgcgt agattttgcg cgtttttata ggattttgtg ttcgttttgc    1200
   gtattgtcgt ttgggttttt ttttttttat tgttgtttgt gtttgttaag cgatagcgat    1260
   tttttcgagg gttcgcgagg ttgtttcgga attttttagg acgtatagtt ttattttggg    1320
   aaatttatcg gttttttttt tttggttttt ttcggcggtt ttcgggtttc gtttggattc    1380
   ggtaacggga tagggaggtc gttttttatt ttcgattgag tggatagtcg cgttttgttc   1440
   gggtggatag tttttttttt ttttacgtta gtttcggggt cgttaagttg tgtagttcgt    1500
   gggtcgggag tatcgaacgg atatagttta ggtcgtggta gggtttagag tgggatgttt    1560
   tatggttttt atttaggttt ggggatattt ttattcgttt tttagaatcg ggtcgtgggg    1620
   gatagaaggg gtttgcgtgc gggtagggag agtattttgg tttttttttg ttttcggggt    1680
   ttataaagtg tgttgggatt tgcggggttg ttttgtttaa gtttgggttt ggcgttcgcg    1740
   tttttgagtt tgtgagtgcg tgcgtttttt tgcgtttttt tgattgtcgg tgttggggtt    1800
   ttgcgttttg cgttcgcg                                                  1818
<210> 15
<400> 15
   000
<210> 16
<400> 16
   000
<210> 17
<400> 17
   000
<210> 18
<400> 18
   000
<210> 19
<400> 19
   000
<210> 20
<400> 20
   000
<210> 21
<400> 21
   000
<210> 22
<400> 22
   000
<210> 23
<400> 23
   000
<210> 24
<400> 24
   000
<210> 25
<400> 25
   000
<210> 26
<400> 26
   000
<210> 27
   <211> 1920
   <212> DNA
   <213> Artificial sequence
<220>
   <223> chemically treated genomic DNA (Homo sapiens)
<400> 27
   ttaggttgtc gtagatatag tttttgtttt ttcgaaaaat acgttttagg cgtcgggatt       60
   ttagatattt gggaaataga gtgtacgtag ttgttgagag gtttcgcgtt tggttttttt      120
   tattattgag gcgtagaggt gttgtggata gtttagattt atacggcgtt cgaggtgaaa      180
   tagaattttt agttttttta tgaggttatt ggtattttcg gttgttttta gagtttttcg      240
   atttagagtt gaatgtaaag taagcgttcg aaatgtagaa gtagtcgggg tcgtttacgg      300
   tatttgtttc gttcggggcg agagaagacg ttaggttgag gttttagcga ttttaggtat      360
   tagtttcgaa ggagggcggg gagatcgtaa aggggaagtg ttcggagggt taacggtttt      420
   cgcgtatttt gcgttttttt gaagcgcgtc gttttttcgc gtcggggatt gggatttgtt     480
   tttggggaat tcgtttagaa gacggcggcg gattggggtc gggtattttt tagggttgtt      540
   aggttttttt tagttttgta tttgtcgcgt cgttttattt cgttaggaag ttttagagat      600
   ttcggggatg gggtgggagc gtttttttat cgcgggttta aaaagaagga aggacgtttt     660
   taggggtcgt agaaggagga ttagttttaa gttataattt ttttcggatt taaggtaggt      720
   cggttggggt ttcgcgttta tacggttttt ggcgggggtt cgcgcgtttc gggagtttcg      780
   cggttcgggg aggaaagagg agataagaga taggcgagga ttacggggtt gatttagtcg      840
   gggtagggat tatcgtggaa aaattttggc gaggtggggg gacgcggaaa gagagcggtt      900
   cgcgttttgt attttgcgtc gggtatttcg cgttagtgtt tcgtttttag tgtttcgcgt      960
   ttcgcgtttc gcgttttgtt tttatttcgg gttagttgta tcgcgttcgc gtcgtaggaa     1020
   tcgtggagtt ggaaagtggg ggcgtcgcgg ttggggggtt gttttagttg cgtttcggtt    1080
   agcgatcggc gggtcgggtt taaatttagt taggttgggt aggcggtggt cgcgcgattg     1140
   gggatcgggc gtttcgtttt tttcgttttt tttttttttt tttttttttt ttttagtttt     1200
   ttggtttttt ttagttttta tcggatttgt tcgttcgttg tttttttttt tttttcgttt     1260
   tttatattat tttttatttt ttcgttttgt tttcgttttt tttttttttt ttgttttttg    1320
   tttttttttt tttttttttt ttttttaggg gcggagtttt tttttttttt tttagataat     1380
   gttgtggttg cgtttttttt ttcgttagtt cgtttaggtt ttcgtcgtta gcgatttttt     1440
   cgggttgggg gtggggaggt ggggggggag tgtagggttg gggaggatga gttggttttt     1500
   tttatttttt tgttgttgtt ttttttaaga gggatggaga tttggtttaa gtttttcggt     1560
   ttattcggag ttgtgatagt tatttttagg gaatagttac gttgttttat taagtttatt     1620
   tttagcggtt tggatttttt aggtagaggt tgtgggattt tgtttttttt aatattttag     1680
   tttattttta aaagggtttg agtcggatag gggttaaata ggttttttcg atttggcggg     1740
   tcggttagac gtgatagtaa tgttaaggag gttaagtttt tttgtttatt ttttattttt     1800
   ttttttttta tttttggatt ttttggcgtt tttagtatat agaggttttt gagtagttcg     1860
   gttgtaggtt ttttatttat ttagagtttt tttttttacg tgtttatttt taattttgta     1920
<210> 28
   <211> 1920
   <212> DNA
   <213> Artificial sequence
<220>
   <223> chemically treated genomic DNA (Homo sapiens)
<400> 28
   tgtagggttg gggataggta cgtgaggggg agaattttga gtagataggg aatttgtagt      60
   cgggttgttt aagggttttt gtgtattggg ggcgttagga ggtttaggga tggaaaaggg     120
   ggaggtgaga aatggataaa gaaatttggt ttttttggta ttgttgttac gtttggtcgg     180
   ttcgttaagt cgaaggggtt tgtttagttt ttgttcggtt taaatttttt tgggaataag     240
   ttgggatgtt agaaaaaata aaattttata atttttgttt ggggaattta ggtcgttgga     300
   ggtgggtttg gtagggtagc gtgattgttt tttgggagtg gttgttatag tttcgggtga     360
   atcgaggagt ttgggttaag tttttatttt ttttggagag ggtagtagta aggaggtgag     420
   gggagttagt ttattttttt taattttgta tttttttttt atttttttat ttttagttcg      480
   gaagaatcgt tggcggcggg agtttggacg agttggcggg gaaggggacg tagttatagt     540
   attgtttggg agggagggga gaagtttcgt ttttagaggg gaggggagaa gaggaggggg     600
   taggagataa ggggaggaag aaaggcgaag gtaaggcgaa ggggtggaga gtgatatgaa     660
   gagcgagaga aaagagagga tagcggacga gtagattcgg taggggttga aaaaggttaa     720
   ggggttggag ggagggagag gaaggaggag gggagcgagg agggcggggc gttcggtttt     780
   tagtcgcgcg gttatcgttt gtttagtttg gttggatttg agttcggttc gtcgatcgtt     840
   ggtcgaggcg tagttgaagt agttttttag tcgcggcgtt tttatttttt aattttacgg      900
   tttttgcggc gcgggcgcga tgtagttggt tcggggtgaa ggtaaggcgc ggggcgcggg      960
   gcgcggggta ttgggagcga ggtattggcg cgggatgttc ggcgtaaggt gtagggcgcg    1020
   ggtcgttttt ttttcgcgtt tttttatttc gttaaagttt ttttacgatg gtttttattt    1080
   cggttgggtt agtttcgtaa ttttcgtttg ttttttgttt tttttttttt tttcgagtcg    1140
   cggggttttc ggggcgcgcg gattttcgtt aggggtcgtg taggcgcgga gttttagtcg     1200
   gtttgttttg ggttcgaaga aagttgtggt ttggagttag tttttttttt acgatttttg     1260
   ggggcgtttt tttttttttt tgagttcgcg atgggaaggc gtttttattt tattttcggg    1320
   gtttttgaga ttttttggcg aggtggggcg gcgcggtagg tgtagggttg gggagggttt      1380
   gatagttttg gagagtgttc gattttagtt cgtcgtcgtt ttttaggcgg attttttaga     1440
   ggtaggtttt agttttcggc gcggggaggc ggcgcgtttt agaggggcgt agggtgcgcg    1500
   ggggtcgttg gtttttcggg tatttttttt ttgcggtttt ttcgtttttt ttcggagttg     1560
   gtgtttgagg tcgttgggat tttagtttgg cgtttttttt cgtttcgagc gaggtaggtg    1620
   tcgtgggcgg tttcggttat ttttgtattt cgagcgttta ttttgtattt agttttaagt    1680
   cggagagttt tggggatagt cgagagtgtt agtggtttta tagggagatt gagggttttg    1740
   ttttatttcg ggcgtcgtgt gggtttgggt tgtttatagt atttttgcgt tttagtgata    1800
   ggagaagtta agcgcgaggt tttttaatag ttgcgtgtat tttatttttt aggtatttgg     1860
   aatttcggcg tttagaacgt gtttttcggg agagtaaagg ttgtgtttac ggtagtttgg    1920
<210> 29
<400> 29
   000
<210> 30
<400> 30
   000
<210> 31
<400> 31
   000
<210> 32
<400> 32
   000
<210> 33
<400> 33
   000
<210> 34
<400> 34
   000
<210> 35
<400> 35
   000
<210> 36
<400> 36
   000
<210> 37
   <211> 219909
   <212> DNA
   <213> Artificial sequence
<220>
   <223> chemically treated genomic DNA (Homo sapiens)
<400> 37
<210> 38
   <211> 219900
   <212> DNA
   <213> Artificial sequence
<220>
   <223> chemically treated genomic DNA (Homo sapiens)
<400> 38
<210> 39
   <211> 1405
   <212> DNA
   <213> Artificial sequence
<220>
   <223> chemically treated genomic DNA (Homo sapiens)
<400> 39
   cgttattcga gttgtaaagg gcggtttttt gtgtttgttt cgttgtatcg atatatcgag      60
   ttgcgtacgg tgtttagcgt agggagaata aatgattatt tgtttaacgc gtttatttat     120
   aggtgaggaa attaaggttt taatttaatc gacgtatttt gtttttttga ttattagaaa     180
   agtagtagga taggtgtttt gtttcgtttt atttcggttt attaagtcgg tatttcggtt     240
   tcgattttcg gttgtgttcg gcgtcgtcgc ggtgttcggc gtcgtcgttt cgttcggcgg     300
   ggtcgttcgg agcgttcgta ttttcgttcg tttttatttg gtcgggttcg tttcgttcgg     360
   attcgggatt gggaagtgcg gcgattttcg gaattagtta ttggcgttag cgcggggagt     420
   tgggggtgta gagttgcggg cgcggcgggt tacgtaggcg gtttttattt tcggtttggt      480
   ttggtttggt ttggtttgcg ttgtcgcgcg ggggcgtttt tttttaggtt cggcgttcgt     540
   tagtttcgtt tcgttaggtg tagcgtagcg taggggtggg cgggggtggg gttcggcgcg     600
   tacgtttacg gggcggggag ggggcgggtt aggggcggga ttatagtcgg ttgggtcggg      660
   gttttatgcg tattttcggg gaggggcggg gcgggggcgg ggtcggggcg gggttcggtc     720
   ggtgtatttt agacggcggg tcgttttttt ttttcgtttt tttattatcg gtttaggatt     780
   agcgttttgg gagcgcgcgt ttcgttgttt cgtcgttata tttttttggg agcggcggtt     840
   acggaggtat tatgaagaag ttttatttag gtgggtttcg cgttcggggt ggggaggggt     900
   cggtgtttcg ggattagcgt tgtttatttg agtgtttgcg gtcgggagtg gcgaggcgtt     960
   ttcggagttg agcgagtttt cgcggcgggt atattgtagg tcgagttttt tttaggatag    1020
   ggtcgttgtc gggtcgtttt cgatttgagt cgatcgtttt ttgcgttgtt tttagttttt    1080
   gttcgagtgt cggaggggtt gttttggggg acgttttttt tttttcgttt tttgtatttt    1140
   cgtaggaatc gttgattttt taggtcggtc gggtgttttg ggtttttgtg cgtttgtgtg    1200
   ggtgaatggg gtcggggtta ggtggagggg tgtttttggg tttagttttt agggttggtg     1260
   gtttaggtcg tagtattttt ttttcggatt tttttcggtt ttttttttat ttagtggggt    1320
   acgggacggt ttttagatgg gatcgtttag tagcgtttaa atttggcgat tcgggtttac    1380
   gttttgcgtt taggacgtcg ttcgt                                          1405
<210> 40
   <211> 1405
   <212> DNA
   <213> Artificial sequence
<220>
   <223> chemically treated genomic DNA (Homo sapiens)
<400> 40
   gcgggcggcg ttttgagcgt aaaacgtgaa ttcgagtcgt taagtttggg cgttgttgga      60
   cggttttatt tggaggtcgt ttcgtgtttt attaagtaga ggagaaatcg aagagaattc     120
   gaagaaagga tgttgcggtt tggattatta gttttagagg ttgaatttaa ggatattttt     180
   ttatttagtt tcgattttat ttatttatat agacgtatag ggatttaaag tattcggtcg     240
   atttggaaag ttagcgattt ttgcgagggt gtaaggggcg aggaagaggg agcgtttttt      300
   agggtagttt tttcgatatt cgagtaaggg ttggagatag cgtaggggac ggtcggttta     360
   ggtcgaaagc ggttcgatag cggttttgtt ttgggaggaa ttcgatttgt agtgtgttcg     420
   tcgcggggat tcgtttagtt tcgggggcgt ttcgttattt tcggtcgtag gtatttaggt     480
   gagtagcgtt ggtttcggga tatcgatttt tttttatttc gggcgcgaag tttatttgag     540
   taagattttt ttatggtgtt ttcgtggtcg tcgtttttag gaaagtgtgg cggcgaggta     600
   gcggggcgcg cgtttttagg gcgttaattt tgggtcggta gtaggaaggc gggaagaggg     660
   ggcggttcgt cgtttggagt gtatcgatcg ggtttcgttt cggtttcgtt ttcgtttcgt     720
   tttttttcgg agatgcgtat agaatttcgg tttagtcggt tgtggtttcg tttttgattc     780
   gttttttttt cgtttcgtga acgtgcgcgt cgagttttat tttcgtttat ttttgcgttg     840
   cgttgtattt ggcggagcgg ggttggcggg cgtcgggttt gggagggggc gttttcgcgc       900
   ggtagcgtag attagattag attaggttag gtcgggggtg ggggtcgttt gcgtggttcg     960
   tcgcgttcgt agttttgtat ttttagtttt tcgcgttggc gttaatggtt ggtttcggga    1020
   gtcgtcgtat tttttagttt cgagttcggg cggggcgggt tcggttaggt ggaagcgggc    1080
   ggaggtgcgg gcgtttcggg cggtttcgtc gggcgaggcg gcggcgtcgg gtatcgcggc    1140
   ggcgtcgggt atagtcgggg gtcggagtcg gggtgtcggt ttagtgggtc ggggtagggc    1200
   gggataggat atttgttttg ttattttttt ggtaattaaa agggtagagt gcgtcgattg    1260
   agttggagtt ttagtttttt tatttgtaaa tggcgcgttt ggatagatga ttatttgttt     1320
   tttttgcgtt gggtatcgtg cgtagttcgg tgtatcggtg tagcggggta gatataggga    1380
   gtcgtttttt ataattcggg taacg                                           1405
<210> 41
   <211> 1818
   <212> DNA
   <213> Artificial sequence
<220>
   <223> chemically treated genomic DNA (Homo sapiens)
<400> 41
   cgtcgttcgt ttttggtttt tttgatagtc gtgttttatt ttcgttttgt gttttttttt      60
   tcggatagtg ttttttttag ggtttattta ggagggtgta gcggtggttt tcggggcggt     120
   ggtcgtggtg ggggtgttag ttgtaggggt gttttcggtg ggtgggagtt ggtggttttt     180
   cgttggtgtt atgggattcg tatgttcgtt ttgcgttttt cggtttttga gtttataggt     240
   cgggattttg tttgttagtc gcgtgcgttg tcgtttaatt tttgtaggcg tagagcgcgc     300
   ggcggcggtg atagagaatt ttgtttggtt gtttaaatat agttttttgt agaaggattt    360
   tgcgttcggg gaaggggagg aatttttttt tttttgggcg ttcgtttttt tcgttatggt     420
   tcggttttta tattcgttta tatttggtcg tagcggggcg ttcgggggga ggggttgagg     480
   tcgcgttttt cgtcgttttt tgggcgcggg ttaggcgggg aggagggggg cgtttcggtc      540
   gtgtgtttag gattgttttt tagcggttat tcgggtttta gttttttagg tttggttttg     600
   ataggcgggc ggagtagtta gtgcgagata gggaggtcgg tgcgggtgcg ggaatttgat     660
   tcgttcggga ggcgggggcg gggcgggggc gtagcgcgcg gggaggggtc ggcgttcgtt     720
   tttttttttt atttatttag ttgagttagg gggtttaggg gttttttcgg cggttagttt     780
   tgtattgtag gagcgcgggc gcggcgtttt agttagcgcg tagggttcgg gtttcgtcgg     840
   gggcgttttt tcgtcgttgt ttttcgcgcg attcgttgtt tattagttat tatgtcggat     900
   ttcgcggtta acgcgtagtt ggatgggatt atttcggatt tcgaaggtgg gtgttgggtt     960
   ggttgttgcg gtcgcggacg tgttggagag gattttgcgg gtgggtttgg cgcgggacgg    1020
   gggtgcgttg aggggagacg ggagtgcgtt gaggggagac gggattttta atttaggcgt    1080
   tttttcgttg agagcgtcgc gcgttttcgg tttcgtgttc gcgtcgttta cgtgggggat    1140
   tttgttaggg gtattcgcgt agattttgcg cgtttttata ggattttgtg ttcgttttgc     1200
   gtattgtcgt ttgggttttt ttttttttat tgttgtttgt gtttgttaag cgatagcgat    1260
   tttttcgagg gttcgcgagg ttgtttcgga attttttagg acgtatagtt ttattttggg     1320
   aaatttatcg gttttttttt tttggttttt ttcggcggtt ttcgggtttc gtttggattc     1380
   ggtaacggga tagggaggtc gttttttatt ttcgattgag tggatagtcg cgttttgttc     1440
   gggtggatag tttttttttt ttttacgtta gtttcggggt cgttaagttg tgtagttcgt     1500
   gggtcgggag tatcgaacgg atatagttta ggtcgtggta gggtttagag tgggatgttt     1560
   tatggttttt atttaggttt ggggatattt ttattcgttt tttagaatcg ggtcgtgggg     1620
   gatagaaggg gtttgcgtgc gggtagggag agtattttgg tttttttttg ttttcggggt     1680
   ttataaagtg tgttgggatt tgcggggttg ttttgtttaa gtttgggttt ggcgttcgcg     1740
   tttttgagtt tgtgagtgcg tgcgtttttt tgcgtttttt tgattgtcgg tgttggggtt     1800
   ttgcgttttg cgttcgcg                                                   1818
<210> 42
   <211> 1818
   <212> DNA
   <213> Artificial sequence
<220>
   <223> chemically treated genomic DNA (Homo sapiens)
<400> 42
   cgcgaacgta aaacgtaaaa ttttaatatc gataattaaa aaaacgtaaa aaaacgtacg       60
   tatttataaa tttaaaaacg cgaacgttaa atttaaattt aaataaaata atttcgtaaa      120
   ttttaatata ttttataaat ttcgaaaata aaaaaaaatt aaaatatttt ttttattcgt      180
   acgtaaattt tttttatttt ttacgattcg attttaaaaa acgaataaaa atatttttaa      240
   atttaaataa aaattataaa atattttatt ttaaatttta ttacgattta aattatattc      300
   gttcgatatt ttcgatttac gaattatata atttaacgat ttcgaaatta acgtaaaaaa      360
   aaaaaaaatt atttattcga ataaaacgcg attatttatt taatcgaaaa taaaaaacga      420
   tttttttatt tcgttatcga atttaaacga aattcgaaaa tcgtcgaaaa aaattaaaaa      480
   aaaaaaatcg ataaattttt taaaataaaa ttatacgttt taaaaaattt cgaaataatt      540
   tcgcgaattt tcgaaaaaat cgttatcgtt taataaatat aaataataat aaaaaaaaaa      600
   aaatttaaac gataatacgt aaaacgaata taaaatttta taaaaacgcg taaaatttac      660
   gcgaatattt ttaataaaat tttttacgta aacgacgcga atacgaaatc gaaaacgcgc      720
   gacgttttta acgaaaaaac gtttaaatta aaaatttcgt ttttttttaa cgtattttcg       780
   ttttttttta acgtattttc gtttcgcgtt aaatttattc gtaaaatttt ttttaatacg      840
   ttcgcgatcg taataattaa tttaatattt attttcgaaa ttcgaaataa ttttatttaa      900
   ttacgcgtta atcgcgaaat tcgatataat aattaataaa taacgaatcg cgcgaaaaat      960
   aacgacgaaa aaacgttttc gacgaaattc gaattttacg cgttaattaa aacgtcgcgt     1020
   tcgcgttttt ataatataaa attaatcgtc gaaaaaattt ttaaattttt taatttaatt     1080
   aaataaataa aaaaaaaaaa cgaacgtcga ttttttttcg cgcgttacgt tttcgtttcg     1140
   ttttcgtttt tcgaacgaat taaattttcg tattcgtatc gattttttta tttcgtatta     1200
   attatttcgt tcgtttatta aaattaaatt taaaaaatta aaattcgaat aatcgttaaa     1260
   aaataatttt aaatatacga tcgaaacgtt tttttttttt tcgtttaatt cgcgtttaaa     1320
   aaacgacgaa aaacgcgatt ttaatttttt ttttcgaacg tttcgttacg attaaatata     1380
   aacgaatata aaaatcgaat tataacgaaa aaaacgaacg tttaaaaaaa aaaaaatttt     1440
   tttttttttt cgaacgtaaa atttttttat aaaaaattat atttaaataa ttaaataaaa     1500
   ttttttatta tcgtcgtcgc gcgttttacg tttataaaaa ttaaacgata acgtacgcga     1560
   ttaataaata aaatttcgat ttataaattt aaaaatcgaa aaacgtaaaa cgaatatacg     1620
   aattttataa tattaacgaa aaattattaa tttttattta tcgaaaatat ttttataatt     1680
   aatattttta ttacgattat cgtttcgaaa attatcgtta tattttttta aataaatttt     1740
   aaaaaaaata ttattcgaaa aaaaaaatat aaaacgaaaa taaaatacga ttattaaaaa     1800
   aattaaaaac gaacgacg                                                   1818
<210> 43
<400> 43
   000
<210> 44
<400> 44
   000
<210> 45
<400> 45
   000
<210> 46
<400> 46
   000
<210> 47
<400> 47
   000
<210> 48
<400> 48
   000
<210> 49
<400> 49
   000
<210> 50
<400> 50
   000
<210> 51
<400> 51
   000
<210> 52
<400> 52
   000
<210> 53
<400> 53
   000
<210> 54
<400> 54
   000
<210> 55
   <211> 1920
   <212> DNA
   <213> Artificial sequence
<220>
   <223> chemically treated genomic DNA (Homo sapiens)
<400> 55
   ttaggttgtt gtagatatag tttttgtttt tttgaaaaat atgttttagg tgttgggatt      60
   ttagatattt gggaaataga gtgtatgtag ttgttgagag gttttgtgtt tggttttttt     120
   tattattgag gtgtagaggt gttgtggata gtttagattt atatggtgtt tgaggtgaaa     180
   tagaattttt agttttttta tgaggttatt ggtatttttg gttgttttta gagttttttg   240
   atttagagtt gaatgtaaag taagtgtttg aaatgtagaa gtagttgggg ttgtttatgg     300
   tatttgtttt gtttggggtg agagaagatg ttaggttgag gttttagtga ttttaggtat     360
   tagttttgaa ggagggtggg gagattgtaa aggggaagtg tttggagggt taatggtttt     420
   tgtgtatttt gtgttttttt gaagtgtgtt gtttttttgt gttggggatt gggatttgtt     480
   tttggggaat ttgtttagaa gatggtggtg gattggggtt gggtattttt tagggttgtt     540
   aggttttttt tagttttgta tttgttgtgt tgttttattt tgttaggaag ttttagagat     600
   tttggggatg gggtgggagt gtttttttat tgtgggttta aaaagaagga aggatgtttt     660
   taggggttgt agaaggagga ttagttttaa gttataattt tttttggatt taaggtaggt     720
   tggttggggt tttgtgttta tatggttttt ggtgggggtt tgtgtgtttt gggagttttg     780
   tggtttgggg aggaaagagg agataagaga taggtgagga ttatggggtt gatttagttg     840
   gggtagggat tattgtggaa aaattttggt gaggtggggg gatgtggaaa gagagtggtt    900
   tgtgttttgt attttgtgtt gggtattttg tgttagtgtt ttgtttttag tgttttgtgt     960
   tttgtgtttt gtgttttgtt tttattttgg gttagttgta ttgtgtttgt gttgtaggaa    1020
   ttgtggagtt ggaaagtggg ggtgttgtgg ttggggggtt gttttagttg tgttttggtt   1080
   agtgattggt gggttgggtt taaatttagt taggttgggt aggtggtggt tgtgtgattg    1140
   gggattgggt gttttgtttt ttttgttttt tttttttttt tttttttttt ttttagtttt    1200
   ttggtttttt ttagttttta ttggatttgt ttgtttgttg tttttttttt ttttttgttt   1260
   tttatattat tttttatttt tttgttttgt ttttgttttt tttttttttt ttgttttttg   1320
   tttttttttt tttttttttt ttttttaggg gtggagtttt tttttttttt tttagataat     1380
   gttgtggttg tgtttttttt tttgttagtt tgtttaggtt tttgttgtta gtgatttttt   1440
   tgggttgggg gtggggaggt ggggggggag tgtagggttg gggaggatga gttggttttt   1500
   tttatttttt tgttgttgtt ttttttaaga gggatggaga tttggtttaa gttttttggt     1560
   ttatttggag ttgtgatagt tatttttagg gaatagttat gttgttttat taagtttatt    1620
   tttagtggtt tggatttttt aggtagaggt tgtgggattt tgtttttttt aatattttag   1680
   tttattttta aaagggtttg agttggatag gggttaaata ggtttttttg atttggtggg   1740
   ttggttagat gtgatagtaa tgttaaggag gttaagtttt tttgtttatt ttttattttt    1800
   ttttttttta tttttggatt ttttggtgtt tttagtatat agaggttttt gagtagtttg    1860
   gttgtaggtt ttttatttat ttagagtttt tttttttatg tgtttatttt taattttgta     1920
<210> 56
   <211> 1920
   <212> DNA
   <213> Artificial sequence
<220>
   <223> chemically treated genomic DNA (Homo sapiens)
<400> 56
   tgtagggttg gggataggta tgtgaggggg agaattttga gtagataggg aatttgtagt      60
   tgggttgttt aagggttttt gtgtattggg ggtgttagga ggtttaggga tggaaaaggg     120
   ggaggtgaga aatggataaa gaaatttggt ttttttggta ttgttgttat gtttggttgg     180
   tttgttaagt tgaaggggtt tgtttagttt ttgtttggtt taaatttttt tgggaataag     240
   ttgggatgtt agaaaaaata aaattttata atttttgttt ggggaattta ggttgttgga     300
   ggtgggtttg gtagggtagt gtgattgttt tttgggagtg gttgttatag ttttgggtga     360
   attgaggagt ttgggttaag tttttatttt ttttggagag ggtagtagta aggaggtgag     420
   gggagttagt ttattttttt taattttgta tttttttttt atttttttat ttttagtttg     480
   gaagaattgt tggtggtggg agtttggatg agttggtggg gaaggggatg tagttatagt     540
   attgtttggg agggagggga gaagttttgt ttttagaggg gaggggagaa gaggaggggg     600
   taggagataa ggggaggaag aaaggtgaag gtaaggtgaa ggggtggaga gtgatatgaa     660
   gagtgagaga aaagagagga tagtggatga gtagatttgg taggggttga aaaaggttaa     720
   ggggttggag ggagggagag gaaggaggag gggagtgagg agggtggggt gtttggtttt     780
   tagttgtgtg gttattgttt gtttagtttg gttggatttg agtttggttt gttgattgtt     840
   ggttgaggtg tagttgaagt agttttttag ttgtggtgtt tttatttttt aattttatgg     900
   tttttgtggt gtgggtgtga tgtagttggt ttggggtgaa ggtaaggtgt ggggtgtggg     960
   gtgtggggta ttgggagtga ggtattggtg tgggatgttt ggtgtaaggt gtagggtgtg    1020
   ggttgttttt tttttgtgtt tttttatttt gttaaagttt ttttatgatg gtttttattt    1080
   tggttgggtt agttttgtaa tttttgtttg ttttttgttt tttttttttt ttttgagttg    1140
   tggggttttt ggggtgtgtg gatttttgtt aggggttgtg taggtgtgga gttttagttg    1200
   gtttgttttg ggtttgaaga aagttgtggt ttggagttag tttttttttt atgatttttg   1260
   ggggtgtttt tttttttttt tgagtttgtg atgggaaggt gtttttattt tatttttggg    1320
   gtttttgaga ttttttggtg aggtggggtg gtgtggtagg tgtagggttg gggagggttt   1380
   gatagttttg gagagtgttt gattttagtt tgttgttgtt ttttaggtgg attttttaga   1440
   ggtaggtttt agtttttggt gtggggaggt ggtgtgtttt agaggggtgt agggtgtgtg    1500
   ggggttgttg gttttttggg tatttttttt ttgtggtttt tttgtttttt tttggagttg   1560
   gtgtttgagg ttgttgggat tttagtttgg tgtttttttt tgttttgagt gaggtaggtg    1620
   ttgtgggtgg ttttggttat ttttgtattt tgagtgttta ttttgtattt agttttaagt    1680
   tggagagttt tggggatagt tgagagtgtt agtggtttta tagggagatt gagggttttg    1740
   ttttattttg ggtgttgtgt gggtttgggt tgtttatagt atttttgtgt tttagtgata    1800
   ggagaagtta agtgtgaggt tttttaatag ttgtgtgtat tttatttttt aggtatttgg    1860
   aattttggtg tttagaatgt gttttttggg agagtaaagg ttgtgtttat ggtagtttgg    1920
<210> 57
<400> 57
   000
<210> 58
<400> 58
   000
<210> 59
<400> 59
   000
<210> 60
<400> 60
   000
<210> 61
<400> 61
   000
<210> 62
<400> 62
   000
<210> 63
<400> 63
   000
<210> 64
<400> 64
   000
<210> 65
   <211> 219909
   <212> DNA
   <213> Artificial sequence
<220>
   <223> chemically treated genomic DNA (Homo sapiens)
<400> 65
<210> 66
   <211> 219909
   <212> DNA
   <213> Artificial sequence
<220>
   <223> chemically treated genomic DNA (Homo sapiens)
<400> 66
<210> 67
   <211> 1405
   <212> DNA
   <213> Artificial sequence
<220>
   <223> chemically treated genomic DNA (Homo sapiens)
<400> 67
   tgttatttga gttgtaaagg gtggtttttt gtgtttgttt tgttgtattg atatattgag     60
   ttgtgtatgg tgtttagtgt agggagaata aatgattatt tgtttaatgt gtttatttat    120
   aggtgaggaa attaaggttt taatttaatt gatgtatttt gtttttttga ttattagaaa    180
   agtagtagga taggtgtttt gttttgtttt attttggttt attaagttgg tattttggtt    240
   ttgatttttg gttgtgtttg gtgttgttgt ggtgtttggt gttgttgttt tgtttggtgg    300
   ggttgtttgg agtgtttgta tttttgtttg tttttatttg gttgggtttg ttttgtttgg    360
   atttgggatt gggaagtgtg gtgatttttg gaattagtta ttggtgttag tgtggggagt    420
   tgggggtgta gagttgtggg tgtggtgggt tatgtaggtg gtttttattt ttggtttggt    480
   ttggtttggt ttggtttgtg ttgttgtgtg ggggtgtttt tttttaggtt tggtgtttgt    540
   tagttttgtt ttgttaggtg tagtgtagtg taggggtggg tgggggtggg gtttggtgtg    600
   tatgtttatg gggtggggag ggggtgggtt aggggtggga ttatagttgg ttgggttggg    660
   gttttatgtg tatttttggg gaggggtggg gtgggggtgg ggttggggtg gggtttggtt    720
   ggtgtatttt agatggtggg ttgttttttt tttttgtttt tttattattg gtttaggatt    780
   agtgttttgg gagtgtgtgt tttgttgttt tgttgttata tttttttggg agtggtggtt    840
   atggaggtat tatgaagaag ttttatttag gtgggttttg tgtttggggt ggggaggggt    900
   tggtgttttg ggattagtgt tgtttatttg agtgtttgtg gttgggagtg gtgaggtgtt    960
   tttggagttg agtgagtttt tgtggtgggt atattgtagg ttgagttttt tttaggatag   1020
   ggttgttgtt gggttgtttt tgatttgagt tgattgtttt ttgtgttgtt tttagttttt   1080
   gtttgagtgt tggaggggtt gttttggggg atgttttttt ttttttgttt tttgtatttt   1140
   tgtaggaatt gttgattttt taggttggtt gggtgttttg ggtttttgtg tgtttgtgtg   1200
   ggtgaatggg gttggggtta ggtggagggg tgtttttggg tttagttttt agggttggtg   1260
   gtttaggttg tagtattttt tttttggatt ttttttggtt ttttttttat ttagtggggt   1320
   atgggatggt ttttagatgg gattgtttag tagtgtttaa atttggtgat ttgggtttat   1380
   gttttgtgtt taggatgttg tttgt                                         1405
<210> 68
   <211> 1405
   <212> DNA
   <213> Artificial sequence
<220>
   <223> chemically treated genomic DNA (Homo sapiens)
<400> 68
   gtgggtggtg ttttgagtgt aaaatgtgaa tttgagttgt taagtttggg tgttgttgga      60
   tggttttatt tggaggttgt tttgtgtttt attaagtaga ggagaaattg aagagaattt     120
   gaagaaagga tgttgtggtt tggattatta gttttagagg ttgaatttaa ggatattttt     180
   ttatttagtt ttgattttat ttatttatat agatgtatag ggatttaaag tatttggttg     240
   atttggaaag ttagtgattt ttgtgagggt gtaaggggtg aggaagaggg agtgtttttt     300
   agggtagttt ttttgatatt tgagtaaggg ttggagatag tgtaggggat ggttggttta     360
   ggttgaaagt ggtttgatag tggttttgtt ttgggaggaa tttgatttgt agtgtgtttg     420
   ttgtggggat ttgtttagtt ttgggggtgt tttgttattt ttggttgtag gtatttaggt     480
   gagtagtgtt ggttttggga tattgatttt tttttatttt gggtgtgaag tttatttgag     540
   taagattttt ttatggtgtt tttgtggttg ttgtttttag gaaagtgtgg tggtgaggta     600
   gtggggtgtg tgtttttagg gtgttaattt tgggttggta gtaggaaggt gggaagaggg     660
   ggtggtttgt tgtttggagt gtattgattg ggttttgttt tggttttgtt tttgttttgt     720
   ttttttttgg agatgtgtat agaattttgg tttagttggt tgtggttttg tttttgattt     780
   gttttttttt tgttttgtga atgtgtgtgt tgagttttat ttttgtttat ttttgtgttg     840
   tgttgtattt ggtggagtgg ggttggtggg tgttgggttt gggagggggt gtttttgtgt     900
   ggtagtgtag attagattag attaggttag gttgggggtg ggggttgttt gtgtggtttg     960
   ttgtgtttgt agttttgtat ttttagtttt ttgtgttggt gttaatggtt ggttttggga    1020
   gttgttgtat tttttagttt tgagtttggg tggggtgggt ttggttaggt ggaagtgggt    1080
   ggaggtgtgg gtgttttggg tggttttgtt gggtgaggtg gtggtgttgg gtattgtggt    1140
   ggtgttgggt atagttgggg gttggagttg gggtgttggt ttagtgggtt ggggtagggt    1200
   gggataggat atttgttttg ttattttttt ggtaattaaa agggtagagt gtgttgattg    1260
   agttggagtt ttagtttttt tatttgtaaa tgggtgtgtt ggatagatga ttatttgttt    1320
   tttttgtgtt gggtattgtg tgtagtttgg tgtattggtg tagtggggta gatataggga    1380
   gttgtttttt ataatttggg taatg                                          1405
<210> 69
   <211> 1818
   <212> DNA
   <213> Artificial sequence
<220>
   <223> chemically treated genomic DNA (Homo sapiens)
<400> 69
   tgttgtttgt ttttggtttt tttgatagtt gtgttttatt tttgttttgt gttttttttt      60
   ttggatagtg ttttttttag ggtttattta ggagggtgta gtggtggttt ttggggtggt    120
   ggttgtggtg ggggtgttag ttgtaggggt gtttttggtg ggtgggagtt ggtggttttt    180
   tgttggtgtt atgggatttg tatgtttgtt ttgtgttttt tggtttttga gtttataggt    240
   tgggattttg tttgttagtt gtgtgtgttg ttgtttaatt tttgtaggtg tagagtgtgt    300
   ggtggtggtg atagagaatt ttgtttggtt gtttaaatat agttttttgt agaaggattt    360
   tgtgtttggg gaaggggagg aatttttttt tttttgggtg tttgtttttt ttgttatggt    420
   ttggttttta tatttgttta tatttggttg tagtggggtg tttgggggga ggggttgagg    480
   ttgtgttttt tgttgttttt tgggtgtggg ttaggtgggg aggagggggg tgttttggtt     540
   gtgtgtttag gattgttttt tagtggttat ttgggtttta gttttttagg tttggttttg    600
   ataggtgggt ggagtagtta gtgtgagata gggaggttgg tgtgggtgtg ggaatttgat    660
   ttgtttggga ggtgggggtg gggtgggggt gtagtgtgtg gggaggggtt ggtgtttgtt    720
   tttttttttt atttatttag ttgagttagg gggtttaggg gtttttttgg tggttagttt    780
   tgtattgtag gagtgtgggt gtggtgtttt agttagtgtg tagggtttgg gttttgttgg    840
   gggtgttttt ttgttgttgt tttttgtgtg atttgttgtt tattagttat tatgttggat    900
   tttgtggtta atgtgtagtt ggatgggatt attttggatt ttgaaggtgg gtgttgggtt    960
   ggttgttgtg gttgtggatg tgttggagag gattttgtgg gtgggtttgg tgtgggatgg   1020
   gggtgtgttg aggggagatg ggagtgtgtt gaggggagat gggattttta atttaggtgt   1080
   ttttttgttg agagtgttgt gtgtttttgg ttttgtgttt gtgttgttta tgtgggggat   1140
   tttgttaggg gtatttgtgt agattttgtg tgtttttata ggattttgtg tttgttttgt    1200
   gtattgttgt ttgggttttt ttttttttat tgttgtttgt gtttgttaag tgatagtgat   1260
   ttttttgagg gtttgtgagg ttgttttgga attttttagg atgtatagtt ttattttggg  1320
   aaatttattg gttttttttt tttggttttt tttggtggtt tttgggtttt gtttggattt   1380
   ggtaatggga tagggaggtt gttttttatt tttgattgag tggatagttg tgttttgttt   1440
   gggtggatag tttttttttt ttttatgtta gttttggggt tgttaagttg tgtagtttgt   1500
   gggttgggag tattgaatgg atatagttta ggttgtggta gggtttagag tgggatgttt   1560
   tatggttttt atttaggttt ggggatattt ttatttgttt tttagaattg ggttgtgggg   1620
   gatagaaggg gtttgtgtgt gggtagggag agtattttgg tttttttttg tttttggggt   1680
   ttataaagtg tgttgggatt tgtggggttg ttttgtttaa gtttgggttt ggtgtttgtg   1740
   tttttgagtt tgtgagtgtg tgtgtttttt tgtgtttttt tgattgttgg tgttggggtt   1800
   ttgtgttttg tgtttgtg                                                  1818
<210> 70
   <211> 1818
   <212> DNA
   <213> Artificial sequence
<220>
   <223> chemically treated genomic DNA (Homo sapiens)
<400> 70
   tgtgaatgta aaatgtaaaa ttttaatatt gataattaaa aaaatgtaaa aaaatgtatg       60
   tatttataaa tttaaaaatg tgaatgttaa atttaaattt aaataaaata attttgtaaa      120
   ttttaatata ttttataaat tttgaaaata aaaaaaaatt aaaatatttt ttttatttgt      180
   atgtaaattt tttttatttt ttatgatttg attttaaaaa atgaataaaa atatttttaa      240
   atttaaataa aaattataaa atattttatt ttaaatttta ttatgattta aattatattt      300
   gtttgatatt tttgatttat gaattatata atttaatgat tttgaaatta atgtaaaaaa      360
   aaaaaaaatt atttatttga ataaaatgtg attatttatt taattgaaaa taaaaaatga      420
   tttttttatt ttgttattga atttaaatga aatttgaaaa ttgttgaaaa aaattaaaaa      480
   aaaaaaattg ataaattttt taaaataaaa ttatatgttt taaaaaattt tgaaataatt      540
   ttgtgaattt ttgaaaaaat tgttattgtt taataaatat aaataataat aaaaaaaaaa      600
   aaatttaaat gataatatgt aaaatgaata taaaatttta taaaaatgtg taaaatttat      660
   gtgaatattt ttaataaaat tttttatgta aatgatgtga atatgaaatt gaaaatgtgt      720
   gatgttttta atgaaaaaat gtttaaatta aaaattttgt ttttttttaa tgtatttttg      780
   ttttttttta atgtattttt gttttgtgtt aaatttattt gtaaaatttt ttttaatatg      840
   tttgtgattg taataattaa tttaatattt atttttgaaa tttgaaataa ttttatttaa      900
   ttatgtgtta attgtgaaat ttgatataat aattaataaa taatgaattg tgtgaaaaat      960
   aatgatgaaa aaatgttttt gatgaaattt gaattttatg tgttaattaa aatgttgtgt     1020
   ttgtgttttt ataatataaa attaattgtt gaaaaaattt ttaaattttt taatttaatt     1080
   aaataaataa aaaaaaaaaa tgaatgttga tttttttttg tgtgttatgt ttttgttttg     1140
   tttttgtttt ttgaatgaat taaatttttg tatttgtatt gattttttta ttttgtatta     1200
   attattttgt ttgtttatta aaattaaatt taaaaaatta aaatttgaat aattgttaaa     1260
   aaataatttt aaatatatga ttgaaatgtt tttttttttt ttgtttaatt tgtgtttaaa     1320
   aaatgatgaa aaatgtgatt ttaatttttt tttttgaatg ttttgttatg attaaatata     1380
   aatgaatata aaaattgaat tataatgaaa aaaatgaatg tttaaaaaaa aaaaaatttt     1440
   tttttttttt tgaatgtaaa atttttttat aaaaaattat atttaaataa ttaaataaaa     1500
   ttttttatta ttgttgttgt gtgttttatg tttataaaaa ttaaatgata atgtatgtga     1560
   ttaataaata aaattttgat ttataaattt aaaaattgaa aaatgtaaaa tgaatatatg     1620
   aattttataa tattaatgaa aaattattaa tttttattta ttgaaaatat ttttataatt     1680
   aatattttta ttatgattat tgttttgaaa attattgtta tattttttta aataaatttt     1740
   aaaaaaaata ttatttgaaa aaaaaaatat aaaatgaaaa taaaatatga ttattaaaaa     1800
   aattaaaaat gaatgatg                                                   1818
<210> 71
<400> 71
   000
<210> 72
<400> 72
   000
<210> 73
<400> 73
   000
<210> 74
<400> 74
   000
<210> 75
<400> 75
   000
<210> 76
<400> 76
   000
<210> 77
<400> 77
   000
<210> 78
<400> 78
   000
<210> 79
<400> 79
   000
<210> 80
<400> 80
   000
<210> 81
<400> 81
   000
<210> 82
<400> 82
   000
<210> 83
   <211> 22
   <212> DNA
   <213> Artificial sequence
<220>
   <223> chemically treated genomic DNA
<400> 83
   gaagtagtcg gggtcgttta cg 22
<210> 84
   <211> 20
   <212> DNA
   <213> Artificial sequence
<220>
   <223> chemically treated genomic DNA
<400> 84
   gcaaaatacg cgaaaaccgt 20
<210> 85
   <211> 23
   <212> DNA
   <213> Artificial sequence
<220>
   <223> chemically treated genomic DNA
<400> 85
   acgtcttctc tcgccccgaa cga 23
<210> 86
<400> 86
   000
<210> 87
<400> 87
   000
<210> 88
<400> 88
   000
<210> 89
<400> 89
   000
<210> 90
<400> 90
   000
<210> 91
<400> 91
   000
<210> 92
<400> 92
   000
<210> 93
<400> 93
   000
<210> 94
<400> 94
   000
<210> 95
<400> 95
   000
<210> 96
<400> 96
   000
<210> 97
<400> 97
   000
<210> 98
<400> 98
   000
<210> 99
<400> 99
   000
<210> 100
<400> 100
   000
<210> 101
<400> 101
   000
<210> 102
<400> 102
   000
<210> 103
<400> 103
   000
<210> 104
<400> 104
   000
<210> 105
<400> 105
   000
<210> 106
<400> 106
   000
<210> 107
   <211> 26
   <212> DNA
   <213> Artificial sequence
<220>
   <223> chemically treated genomic DNA
<400> 107
   gtagtagtta gtttagtatt tatttt 26
<210> 108
   <211> 24
   <212> DNA
   <213> Artificial sequence
<220>
   <223> chemically treated DNA
<400> 108
   gatttagagt tgaatgtaaa gtaa 24
<210> 109
   <211> 18
   <212> DNA
   <213> Artificial sequence
<220>
   <223> chemically treated genomic DNA
<400> 109
   ttttttggag ttgaaagg 18
<210> 110
   <211> 20
   <212> DNA
   <213> Artificial sequence
<220>
   <223> chemically treated genomic DNA
<400> 110
   ttaggaaatg aaattggaga 20
<210> 111
   <211> 20
   <212> DNA
   <213> Artificial sequence
<220>
   <223> chemically treated genomic DNA
<400> 111
   aaacccaaac ctaaattaaa 20
<210> 112
   <211> 17
   <212> DNA
   <213> Artificial sequence
<220>
   <223> chemically treated genomic DNA
<400> 112
   cccaccaacc atcatat 17
<210> 113
   <211> 18
   <212> DNA
   <213> Artificial sequence
<220>
   <223> chemically treated genomic DNA
<400> 113
   ctaaaacctc aacctaac 18
<210> 114
   <211> 23
   <212> DNA
   <213> Artificial sequence
<220>
   <223> chemically treated genomic DNA
<400> 114
   aaattactac catctccaac tac 23
<210> 115
   <211> 23
   <212> DNA
   <213> Artificial sequence
<220>
   <223> chemically treated genomic DNA
<400> 115
   tataaaaaat tacttcccac atc 23
<210> 116
   <211> 17
   <212> DNA
   <213> Artificial sequence
<220>
   <223> chemically treated genomic DNA
<400> 116
   ggaagtgtgt ggtaaag 17

## Description

### FIELD OF THE INVENTION

The present invention relates to genomic DNA sequences that exhibit altered expression patterns in disease states relative to normal. Particular embodiments provide methods and kits useful for detecting cell proliferative disorders. The methods for the detection and diagnosis of cell proliferative disorders as provided in the present invention, are used for the diagnosis of colorectal cancer.

### SEQUENCE LISTING

A Sequence Listing in paperform and comprising SEQ ID NOS:7, 12-14, 27, 28, 37 to 42, 55, 56, 65-70, 83-85 and 107-116is included with this application and is incorporated by reference herein in its entirety.

### BACKGROUND

### Incidence and diagnosis of cancer.

Cancer is the second leading cause of death of the United States. Mortality rates could be significantly improved if current screening methods would be improved in terms of patient compliance, sensitivity and ease of screening. Current recommended methods for diagnosis of cancer are often expensive and are not suitable for application as population wide screening tests.

In the United States the annual incidence of colorectal cancer is approximately 150,000, with 56,600 individuals dying from colorectal cancer each year. The lifetime risk of colorectal cancer in the general population is about 5 to 6 percent. Despite intensive efforts in recent years in screening and early detection of colon cancer, until today most cases are diagnosed in an advanced stage with regional or distant metastasis. While the therapeutic options include surgery and adjuvant or palliative chemotherapy, most patients die from progression of their cancer within a few months. Identifying the molecular changes that underlie the development of colon cancer may help to develop new monitoring, screening, diagnostic and therapeutic options that could improve the overall poor prognosis of these patients.

The current guidelines for colorectal screening according to the American Cancer Society utilizes one of five different options for screening in average risk individuals 50 years of age or older. These options include 1) fecal occult blood test (FOBT) annually, 2) flexible sigmoidoscopy every five years, 3) annual FPBT plus flexible sigmoidoscopy every five years, 4) double contrast barium enema (DCBE) every five years or 5) colonoscopy every ten years. Even though these testing procedures are well accepted by the medical community, the implementation of widespread screening for colorectal cancer has not been realized. Patient compliance is a major factor for limited use due to the discomfort or inconvenience associated with the procedures. FOBT testing, although a non-invasive procedure, requires dietary and other restrictions 3-5 days prior to testing. Sensitivity levels for this test are also very low for colorectal adenocarcinoma with wide variability depending on the trial. Sensitivity measurements for detection of adenomas is even less since most adenomas do not bleed. In contrast, sensitivity for more invasive procedures such as sigmoidoscopy and colonoscopy are quite high because of direct visualization of the lumen of the colon. No randomized trials have evaluated the efficacy of these techniques, however, using data from case-control studies and data from the National Polyp Study (U.S.) it has been shown that removal of adenomatous polyps results in a 76-90% reduction in CRC incidence. Sigmoidoscopy has the limitation of only visualizing the left side of the colon leaving lesions in the right colon undetected. Both scoping procedures are expensive, require cathartic preparation and have increased risk of morbidity and mortality. Improved tests with increased sensitivity, specificity, ease of use and decreased costs are clearly needed before general widespread screening for colorectal cancer becomes routine.

Early colorectal cancer detection is generally based on the fecal occult blood test (FOBT) performed annually on asymptomatic individuals. Current recommendations adapted by several healthcare organizations, including the American Cancer Society, call for fecal occult blood testing beginning at age 50, repeated annually until such time as the patient would no longer benefit from screening. A positive FOBT leads to colonoscopic examination of the bowel; an expensive and invasive procedure, with a serious complication rate of one per 5,000 examinations. Only 12% of patients with heme positive stool are diagnosed with cancer or large polyps at the time of colonoscopy. A number of studies show that FOBT screening does not improve cancer-related mortality or overall survival. Compliance with occult blood testing has been poor; less than 20 percent of the population is offered or completes FOBT as recommended. If FOBT is properly done, the patient collects a fecal sample from three consecutive bowel movements. Samples are obtained while the patient adheres to dietary guidelines and avoids medications known to induce occult gastrointestinal bleeding. In reality, physicians frequently fail to instruct patients properly, patients frequently fail to adhere to protocol, and some patients find the task of collecting fecal samples difficult or unpleasant, hence compliance with annual occult blood testing is poor. If testing sensitivity and specificity can be improved over current methods, the frequency of testing could be reduced, collection of consecutive samples would be eliminated, dietary and medication schedule modifications would be eliminated, and patient compliance would be enhanced. Compounding the problem of compliance, the sensitivity and specificity of FOBT to detect colon cancer is poor. Poor test specificity leads to unnecessary colonoscopy, adding considerable expense to colon cancer screening.

Specificity of the FOBT has been calculated at best to be 96%, with a sensitivity of 43% (adenomas) and 50% (colorectal carcinoma). Sensitivity can be improved using an immunoassay FOBT such as that produced under the tradename 'InSure™', with an improved sensitivity of 77 % (adenomas) and 88.9% (colorectal carcinoma.

*Molecular disease markers.* Molecular disease markers offer several advantages over other types of markers, one advantage being that even samples of very small sizes and/or samples whose tissue architecture has not been maintained can be analyzed quite efficiently. Within the last decade a number of genes have been shown to be differentially expressed between normal and colon carcinomas. However, no single or combination of marker has been shown to be sufficient for the diagnosis of colon carcinomas. High-dimensional mRNA based approaches have recently been shown to be able to provide a better means to distinguish between different tumor types and benign and malignant lesions. However its application as a routine diagnostic tool in a clinical environment is impeded by the extreme instability of mRNA, the rapidly occurring expression changes following certain triggers (*e.g*., sample collection), and, most importantly, the large amount of mRNA needed for analysis (Lipshutz, R. J. et al., Nature Genetics 21:20-24, 1999; Bowtell, D. D. L. Nature genetics suppl. 21:25-32, 1999), which often cannot be obtained from a routine biopsy.

The use of biological markers to further improve sensitivity and specificity of FOBT has been suggested, examples of such tests include the PreGen-Plus™ stool analysis assay available from EXACT Sciences which has a sensitivity of 20% (adenoma) and 52% (colorectal carcinoma) and a specificity of 95% in both cases. This test assays for the presence of 23 DNA mutations associated with the development of colon neoplasms. The use of DNA methylation as colon cancer markers is known. For example Sabbioni et al. (Molecular Diagnosis 7:201-207, 2003) detected hypermethylation of a panel of genes consisiting TPEF, HIC1, DAPK and MGMT in peripheral blood in 98% of colon carcinoma patients. However, this does provide a suitable basis for a commercially marketable test, as the specificity of such a test must also be sufficiently high.

The current model of colorectal pathogenesis favours a stepwise progression of adenomas, which includes the development of dysplasia and finally signs of invasive cancer. The molecular changes underlying this adenoma-carcinoma sequence include genetic and epigenetic alterations of tumor suppressor genes (APC, p53, DCC), the activation of oncogenes (K-ras) and the inactivation of DNA mismatch repair genes. Recently, further molecular changes and genetic defects have been revealed. Thus, activation of the Wnt signalling pathway not only includes mutations of the APC gene, but may also result from β-catenin mutations. Furthermore, alterations in the TGF-β signalling pathway together with its signal transducers SMAD4 and SMAD2 have been linked to the development of colon cancer.

Despite recent progress in the understanding of the pathogenesis of adenomas and carcinomas of the colon and their genetic and molecular changes, the genetic and epigenetic changes underlying the development of metastasis are less well understood. It is, however, generally well accepted that the process of invasion and proteolysis of the extracellular matrix, as well as infiltration of the vascular basement membrane involve adhesive proteins, such as members of the family of integrin receptors, the cadherins, the immunoglobulin superfamily, the laminin binding protein and the CD44 receptor. Apart from adhesion, the process of metastasis formation also includes the induction and regulation of angiogenesis (VEGF, bFGF), the induction of cell proliferation (EGF, HGF, IGF) and the activation of proteolytic enzymes (MMPs, TIMPs, uPAR), as well as the inhibition of apoptosis (Bcl-2, Bcl-X). More recently other groups have compared the genetic and molecular changes in metastatic lesions to the changes found in primary colorectal cancers. Thus, Kleeff et al. reported the loss of DOC-2, a candidate tumor suppressor gene, both in primary and metastatic colorectal cancer. Furthermore, Zauber et al. reported that in their series of 42 colorectal cancers Ki-ras mutations in the primary cancers were identical in all of the 42 paired primary and synchronous metastatic lesions. Similarly loss of heterozygosity at the APC locus was identical for 39 paired carcinomas and synchronous metastasis. The authors concluded that for Ki-ras and APC genes the genetic changes in metastasis are identical to the primary colorectal cancer. However, other groups have found genetic and molecular changes in metastatic colon cancers, that are not present in the primary cancers. Thus, the development of LOH of chromosome 3p in colorectal metastasis has been reported.

*CpG island methylation.* Apart from mutations aberrant methylation of CpG islands has been shown to lead to the transcriptional silencing of certain genes that have been previously linked to the pathogenesis of various cancers. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

Recently several groups have also analysed the methylation of various genes in colorectal cancer and reported the transcriptional silencing by promoter methylation for p16INK4, p14ARF, p15INK4b, MGMT, hMLH1, GSTP1, DAPK, CDH1, TIMP-3 and APC among others. Thus apart from mutational inactivation of certain genes, the hypermethylation of these genes also contributes significantly to the pathogenesis of this disease.

In recent years several genes that are methylated in colon cancer have been identified by MS-APPCR. This group of genes, among others, includes TPEF/HPP1 which is frequently methylated in colon cancers and which was independently identified by two different groups using the MS-APPCR method (see, e.g., Young J, Biden KG, Simms LA, Huggard P, Karamatic R, Eyre HJ, Sutherland GR, Herath N, Barker M, Anderson GJ, Fitzpatrick DR, Ramm GA, Jass JR, Leggett BA. HPP1: a transmembrane protein-encoding gene commonly methylated in colorectal polyps and cancers. Proc Natl Acad Sci USA 98:265-270, 2001).

*Multifactorial approach.* Cancer diagnostics has traditionally relied upon the detection of single molecular markers (*e.g*., gene mutations, elevated PSA levels). Unfortunately, cancer is a disease state in which single markers have typically failed to detect or differentiate many forms of the disease. Thus, assays that recognize only a single marker have been shown to be of limited predictive value. A fundamental aspect of this invention is that methylation-based cancer diagnostics and the screening, diagnosis, and therapeutic monitoring of such diseases will provide significant improvements over the state-of-the-art that uses single marker analyses by the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.

Key to the successful implementation of a panel approach to methylation based diagnostic tests is the design and development of optimized panels of markers that can characterize and distinguish disease states. The present disclosure describes a plurality of particularly efficient and unique panels of genes, the methylation analysis of one or a combination of the members of the panel enabling the detection of colon cell proliferative disorders with a particularly high sensitivity, specificity and/or predictive value.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, *i.e.,* individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, *i.e.,* individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. n example of a test that has high specificity is a gene-based test that can detect a p53 mutation. Specificity is important when the cost or risk associated with further diagnostic procedures or further medical intervention are very high.

*Pronounced need in the art.* It is generally accepted that there is a pronounced need in the art for improved screening and early detection of cancers. As an example, if colon cancer screening specificity can be increased, the problem of false positive test results leading to unnecessary colonoscopic examination would be reduced leading to cost savings and improved safety.

In view of the incidence of cancers in general and more particularly the disadvantages associated with current colorectal cell proliferative disorder screening methods there is a substantial need in the art for improved methods for the early detection of cancer, in particular colon cancer, to be used in addition to or as a substitute for currently available tests.

*Background of the genes of the present invention.* The human Septin 9 gene (also known as MLL septin-like fusion protein, MLL septin-like fusion protein MSF-A, Slpa, Eseptin, Msf, septin-like protein Ovarian/Breast septin (Ov/Br septin) and Septin D1) is located on chromosome 17q25 within contig AC068594.15.1.168501 and is a member of the Septin gene family. SEQ ID NO: 12 provides the sequence of said gene, comprising regions of both the Septin 9 and Q9HC74 transcripts and promoter regions. SEQ ID NO: 13 and SEQ ID NO: 14 provide particularly preferred CpG rich regions of said gene.

It has been postulated that members of the Septin gene family are associated with multiple cellular functions ranging from vesicle transport to cytokinesis. Disruption of the action of *Septin* 9 results in incomplete cell division, see Surka, M.C., Tsang, C.W., and Trimble, W.S. Mol Biol Cell, 13: 3532-45 (2002). *Septin* 9 and other proteins have been shown to be fusion partners of the proto-oncogene *MLL* suggesting a role in tumorogenesis, see Osaka, M, Rowley, J.D. and Zeleznik-Le, N.J. PNAS, 96:6428-6433 (1999). Burrows et al. reported an in depth study of expression of the multiple isoforms of the *Septin* 9 gene in ovarian cancer and showed tissue specific expression of various transcripts, see Burrows, J. F., Chanduloy, et al. S.E.H. Journal of Pathology, 201:581-588 (2003).

A recent study (post-priority date published prior art) of over 7000 normal and tumor tissues indicates that there is consistent over-expression of *Septin* 9 isoforms in a number of tumor tissues, see Scott, M., Hyland, P.L., et al. Oncogene, 24: 4688-4700 (2005). The authors speculate that the gene is likely a type II cancer gene where changes in RNA transcript processing control regulation of different protein products, and the levels of these altered protein isoforms may provide answers to the gene's role in malignancy.

Thierry Lecomte ET AL: "Detection of free-circulating tumor-associated DNA in plasma of colorectal cancer patients and its association with prognosis", International Journal of Cancer, vol. 100, no. 5, 2002, pages 542-548, discloses the detection of p16 methylation in blood samples.

HESSON L B ET AL: "CpG island promoter hypermethylation of a novel Ras-effector gene RASSF2A is an early event in colon carcinogenesis and correlates inversely with K-ras mutations" ONCOGENE, vol. 24, (2005), pages 3987-3994, discloses that hypermethylation of RASSF2 in a tumour sample correlates with colorectal cancer.

### BRIEF DESCRIPTION OF THE DRAWINGS

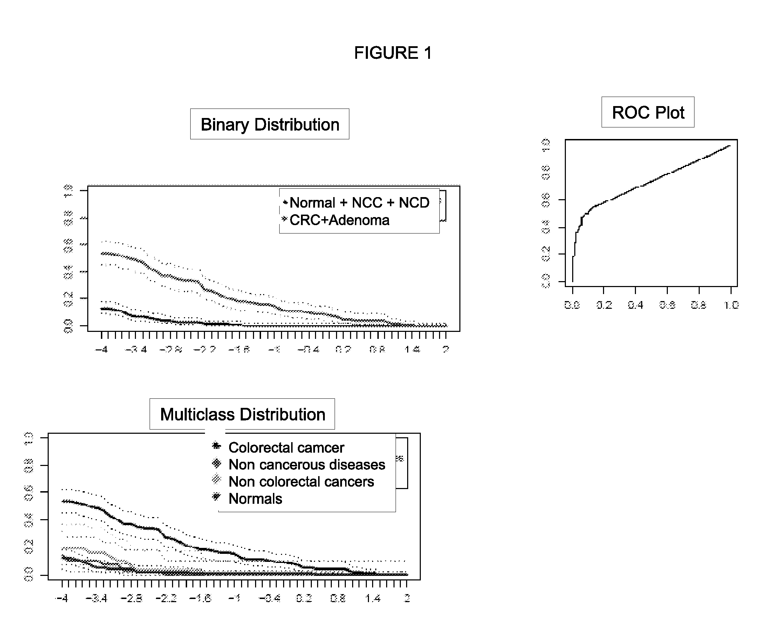

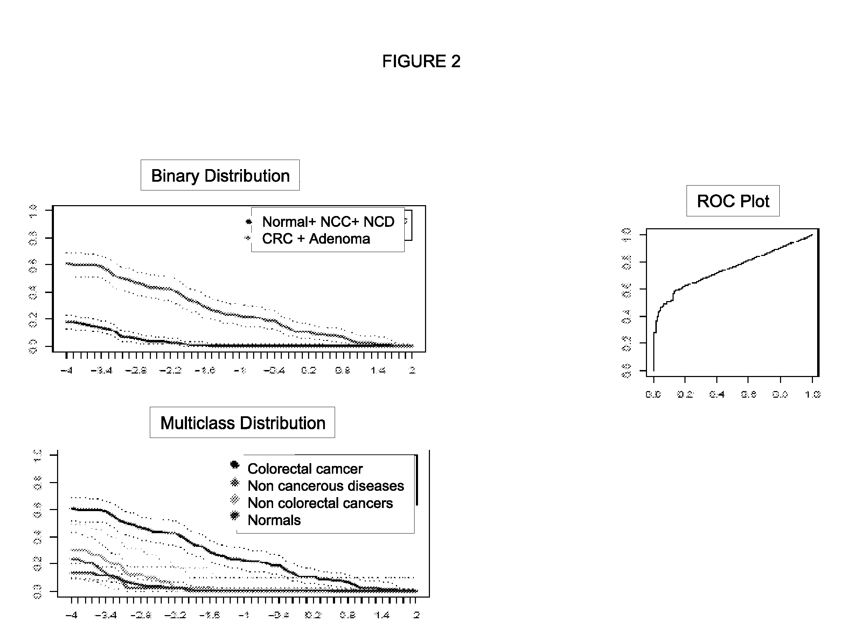

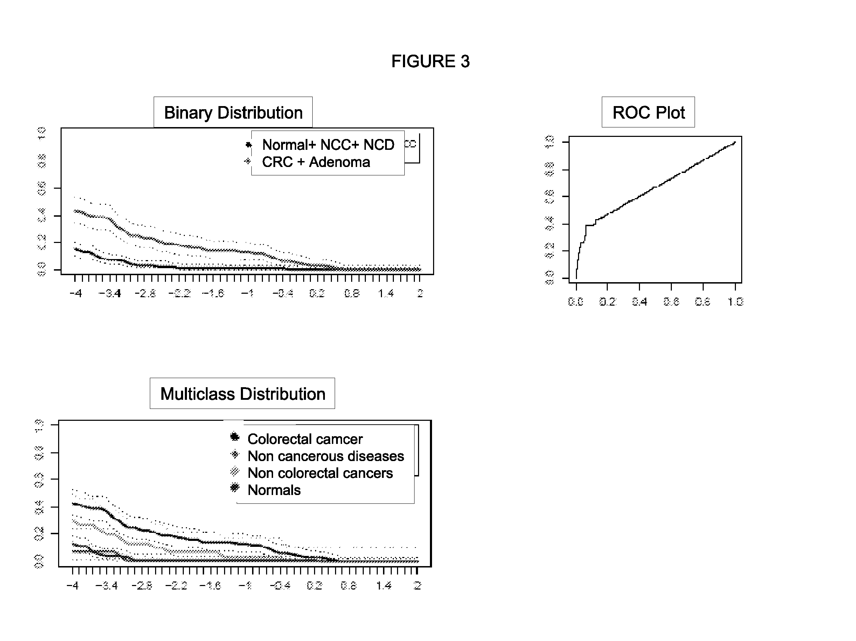

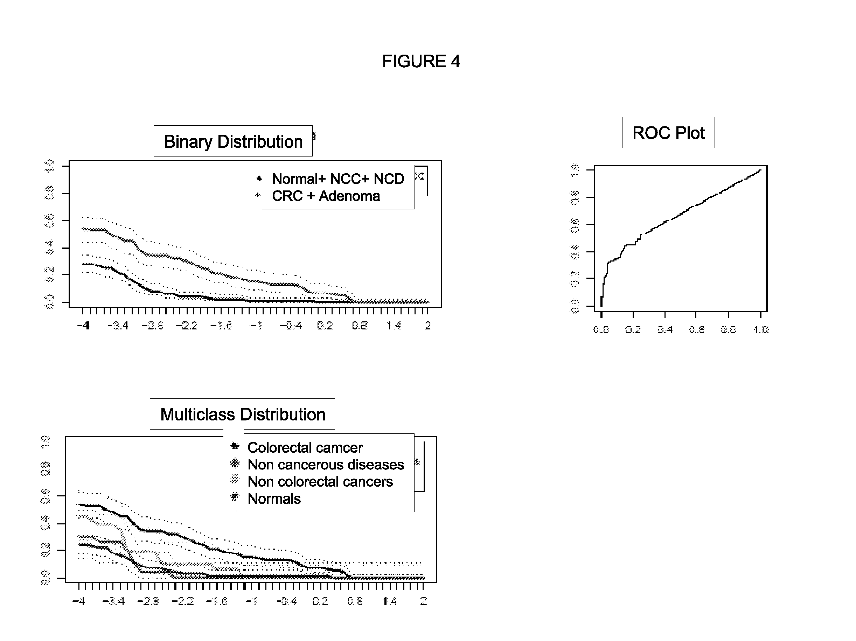

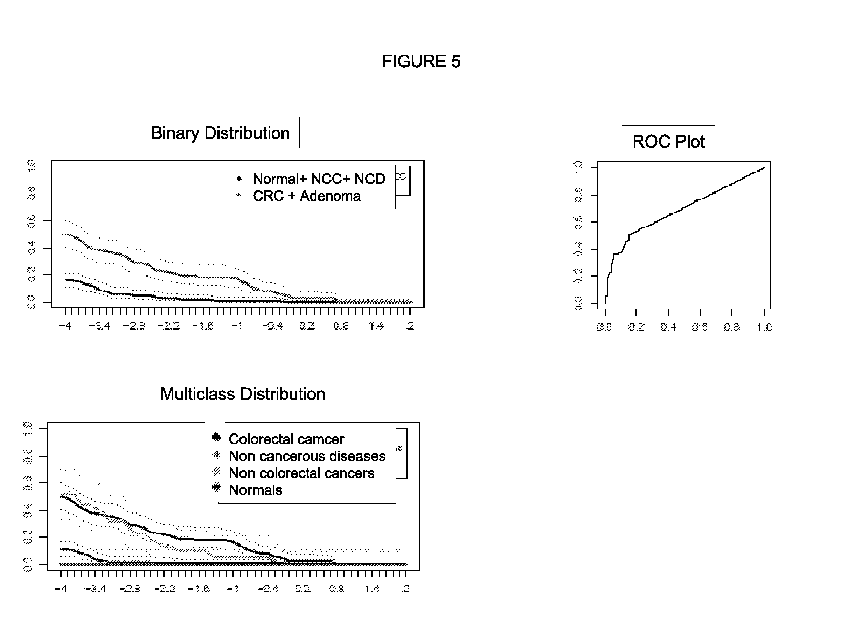

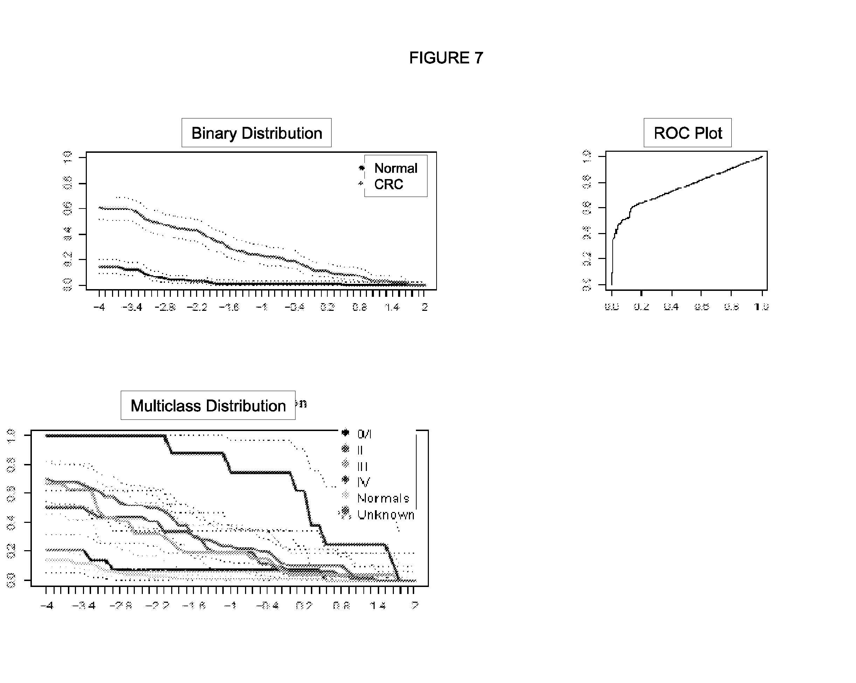

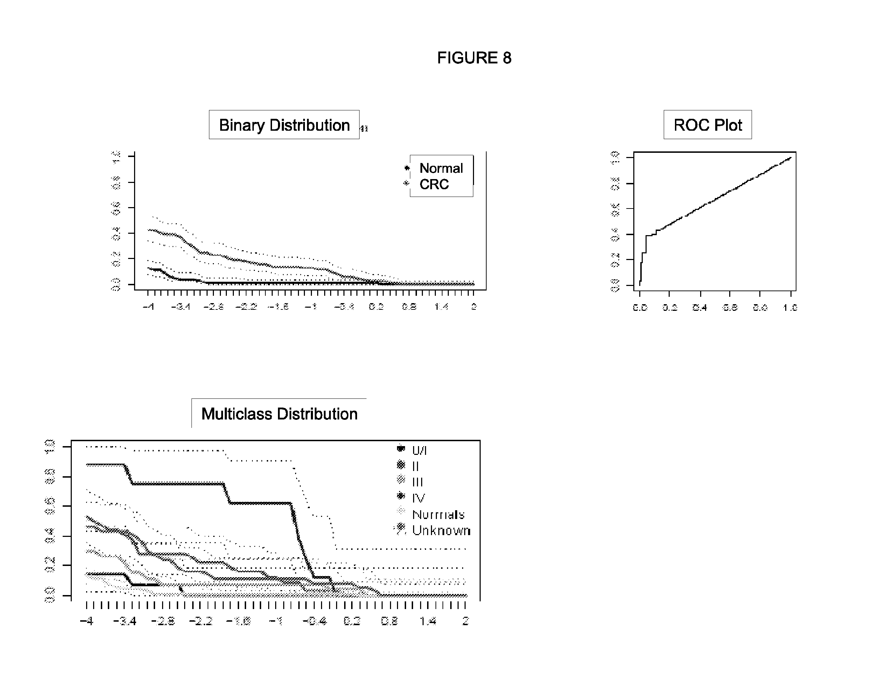

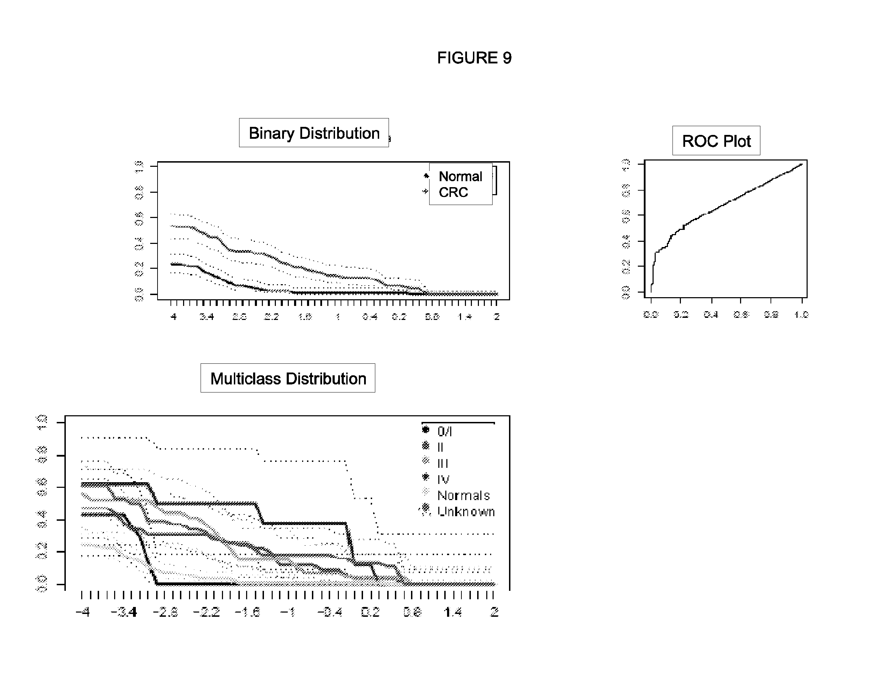

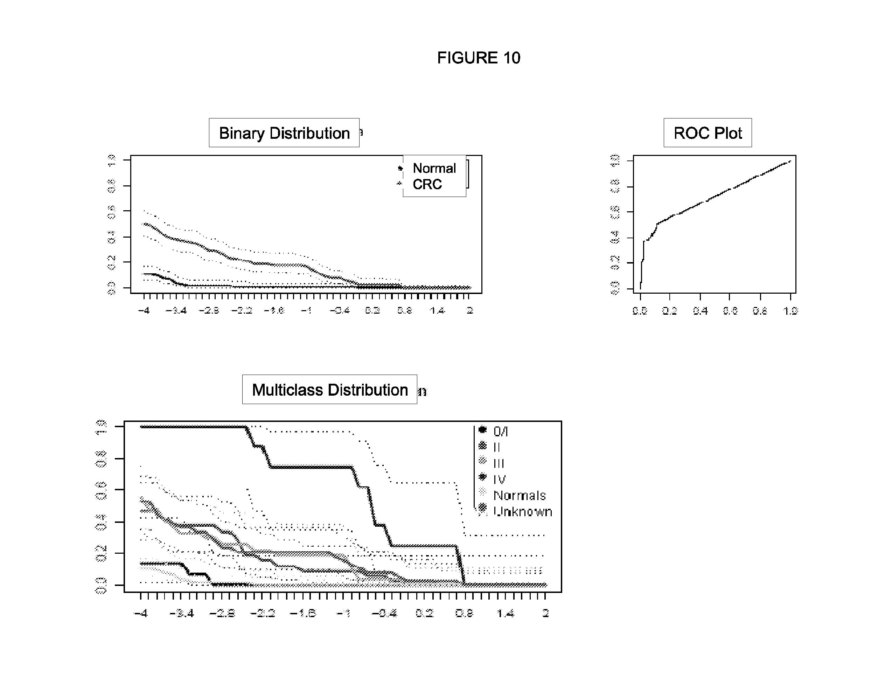

Figures 1 to 10 provide an overview of the log mean methylation measured by means of the HM assay according to Example 2. Each figures consists of three plots, the upper and lower left hand side plots provide the binary and multiclass analysis respectively, sensitivity is shown on the Y-axis, DNA methylation measured in (log 10 ng/mL) is shown on the X-axis.
   In each figure the right hand plot provides an ROC wherein sensitivity is shown on the Y-axis and 1-specificity is shown on the X-axis.
Figure 1 provides an overview of the performance of the RASSF2 HM assay according to Example 2, in all samples.
Figure 2 provides an overview of the performance of the Septin 9 HM assay according to Example 2, in all samples.
Figure 3 provides an overview of the performance of the SND1 HM assay according to Example 2, in all samples.
Figure 4 provides an overview of the performance of the PCDHGC3 HM assay according to Example 2, in all samples.
Figure 5 provides an overview of the performance of the TFAP2E HM assay according to Example 2, in all samples.
Figure 6 provides an overview of the performance of the RASSF2 HM assay according to Example 2, in all colorectal carcinoma and normal colorectal tissue samples.
Figure 7 provides an overview of the performance of the Septin 9 HM assay according to Example 2, in all colorectal carcinoma and normal colorectal tissue samples.
Figure 8 provides an overview of the performance of the SND1 HM assay according to Example 2, in all colorectal carcinoma and normal colorectal tissue samples.
Figure 9 provides an overview of the performance of the PCDHGC3 HM assay according to Example 2, in all colorectal carcinoma and normal colorectal tissue samples.
Figure 10 provides an overview of the performance of the TFAP2E HM assay according to Example 2, in all colorectal carcinoma and normal colorectal tissue samples.
Figure 11 provides an overview of the predictive power of the logistic regression model of combinations of markers. Se is sensitivitiy, sp is specificity, AUC is area under the curve.
Figures 12 to 21 provide an overview of the log majority mean methylation measured by means of the HM assay according to Example 2. Each figures consists of three plots, the upper and lower left hand side plots provide the binary and multiclass analysis respectively, sensitivity is shown on the Y-axis, DNA methylation measured in (log 10 ng/mL) is shown on the X-axis.
   In each figure the right hand plot provides an ROC wherein sensitivity is shown on the Y-axis and 1-specificity is shown on the X-axis.

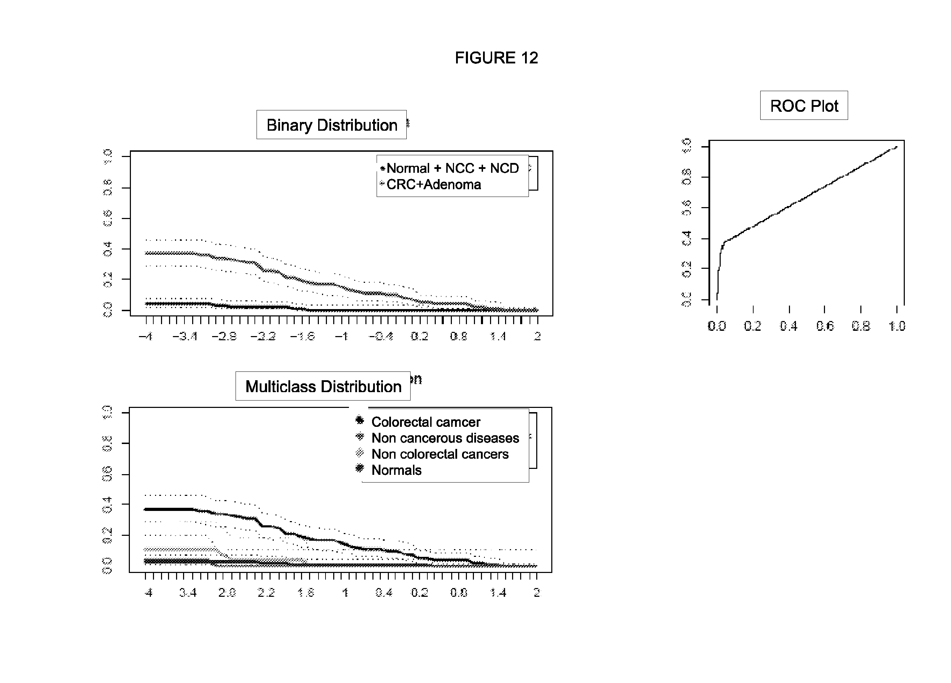

Figure 12 provides an overview of the performance of the RASSF2 HM assay according to Example 2, in all samples.

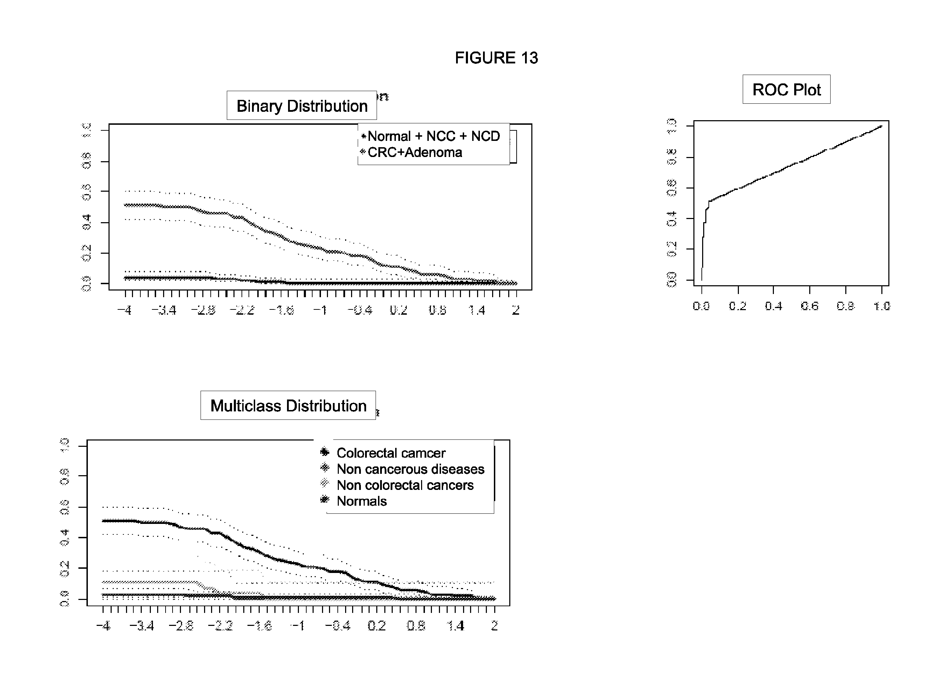

Figure 13 provides an overview of the performance of the Septin 9 HM assay according to Example 2, in all samples.

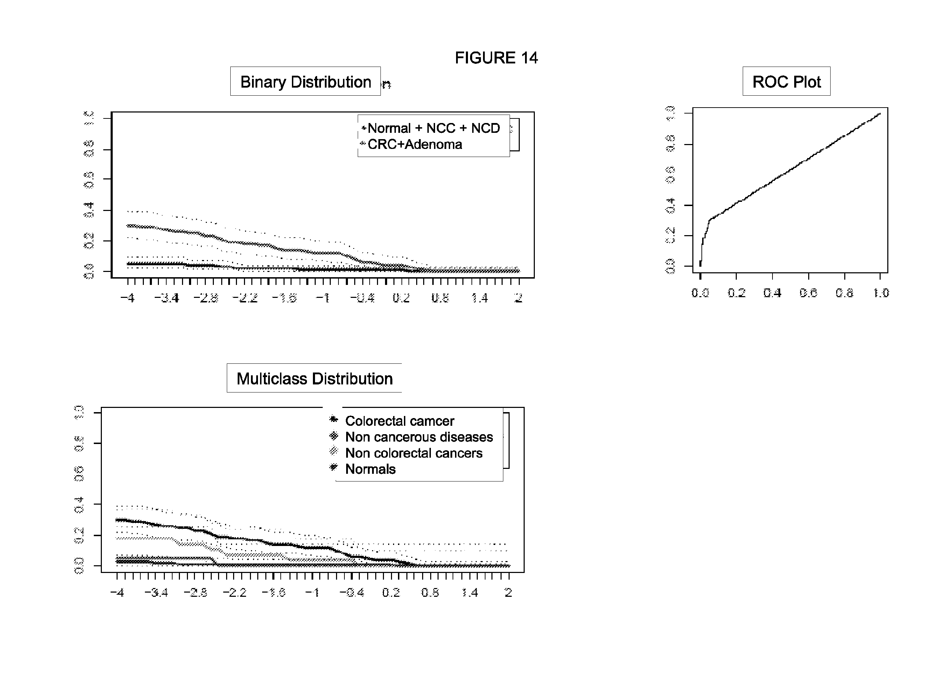

Figure 14 provides an overview of the performance of the SND1 HM assay according to Example 2, in all samples.

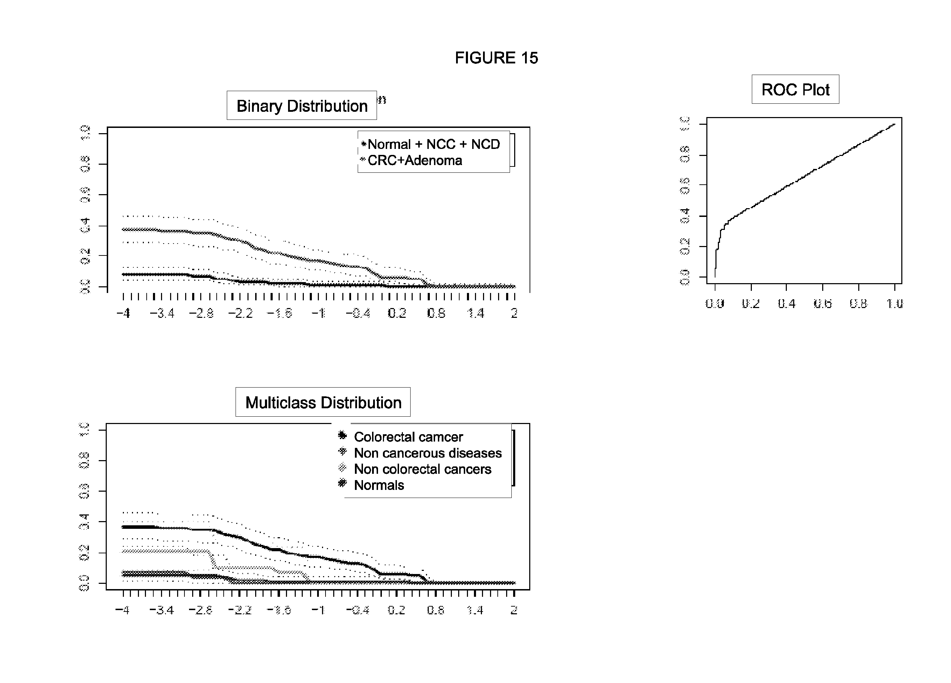

Figure 15 provides an overview of the performance of the PCDHGC3 HM assay according to Example 2, in all samples.

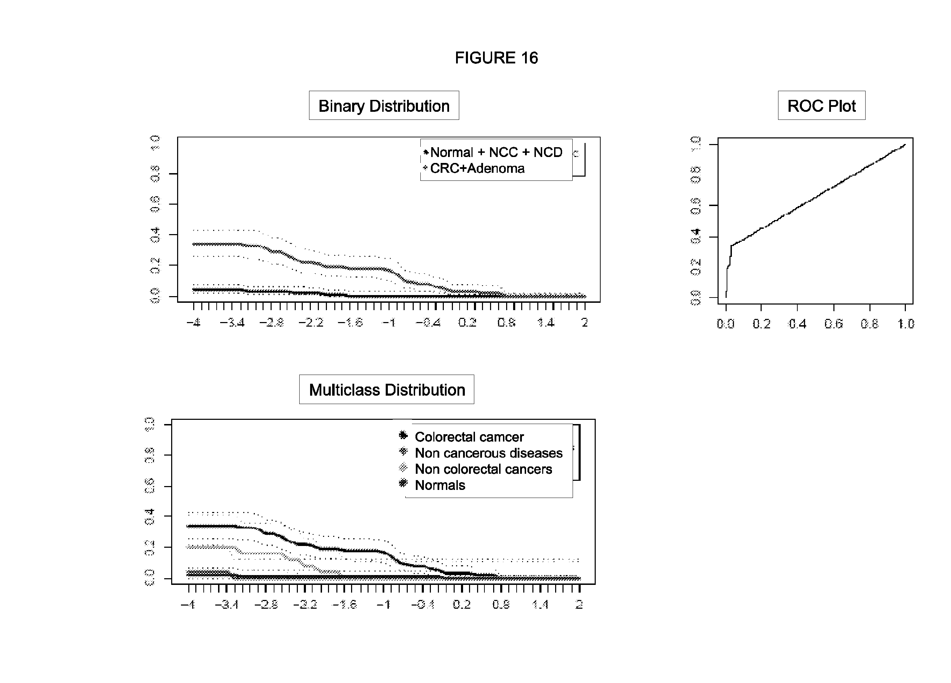

Figure 16 provides an overview of the performance of the TFAP2E HM assay according to Example 2, in all samples.

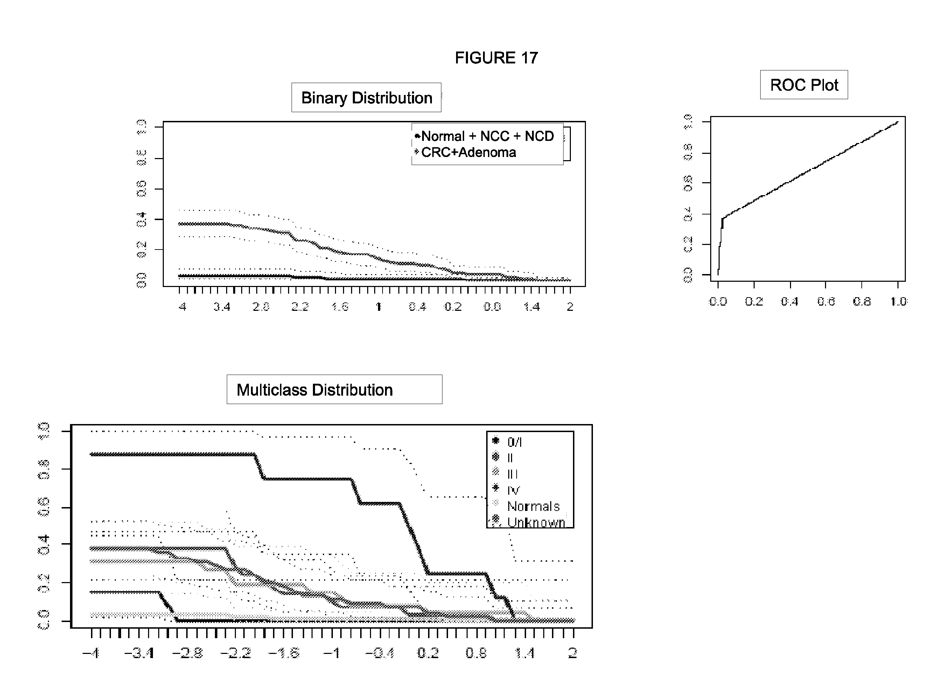

Figure 17 provides an overview of the performance of the RASSF2 HM assay according to Example 2, in all colorectal carcinoma and normal colorectal tissue samples.

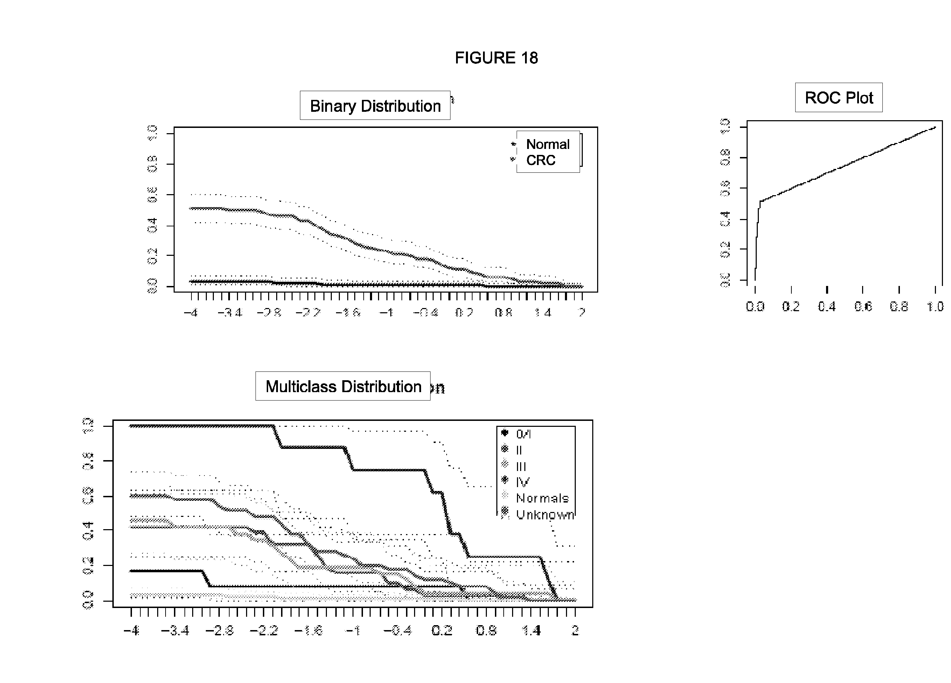

Figure 18 provides an overview of the performance of the Septin 9 HM assay according to Example 2, in all colorectal carcinoma and normal colorectal tissue samples.

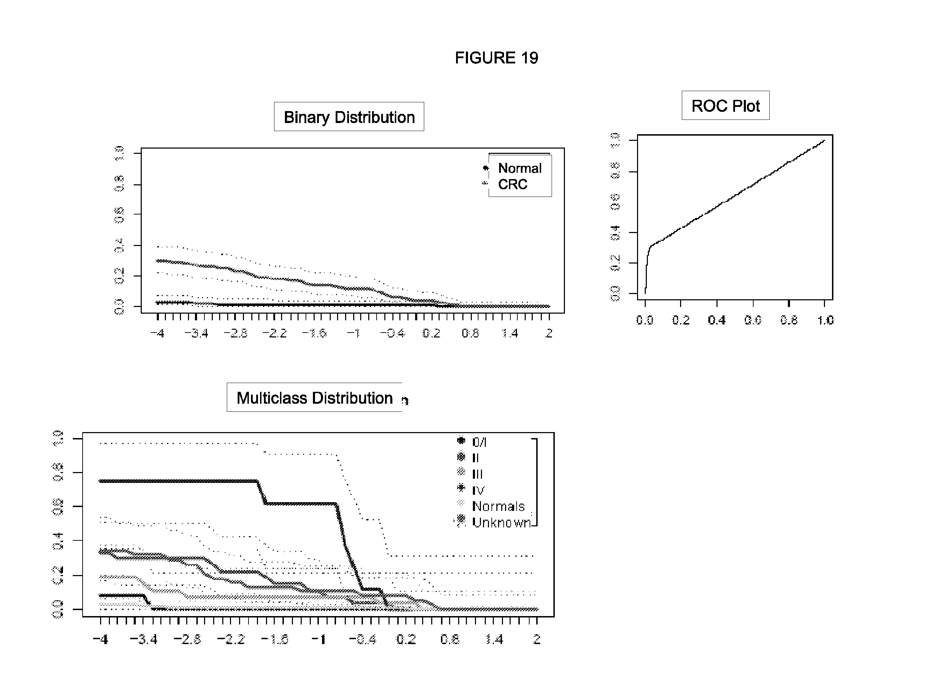

Figure 19 provides an overview of the performance of the SND1 HM assay according to Example 2, in all colorectal carcinoma and normal colorectal tissue samples.

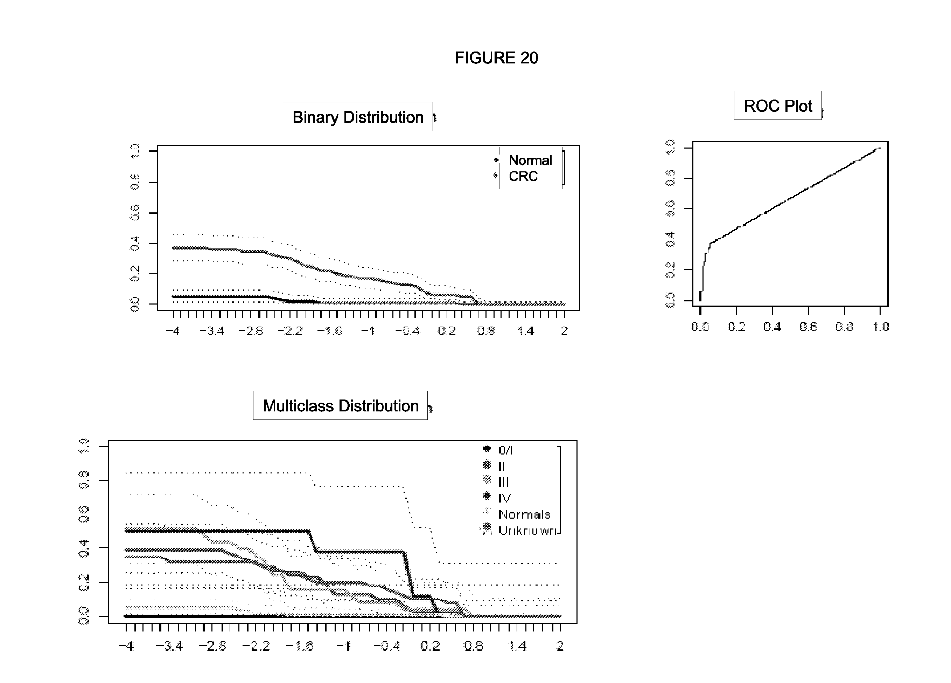

Figure 20 provides an overview of the performance of the PCDHGC3 HM assay according to Example 2, in all colorectal carcinoma and normal colorectal tissue samples.

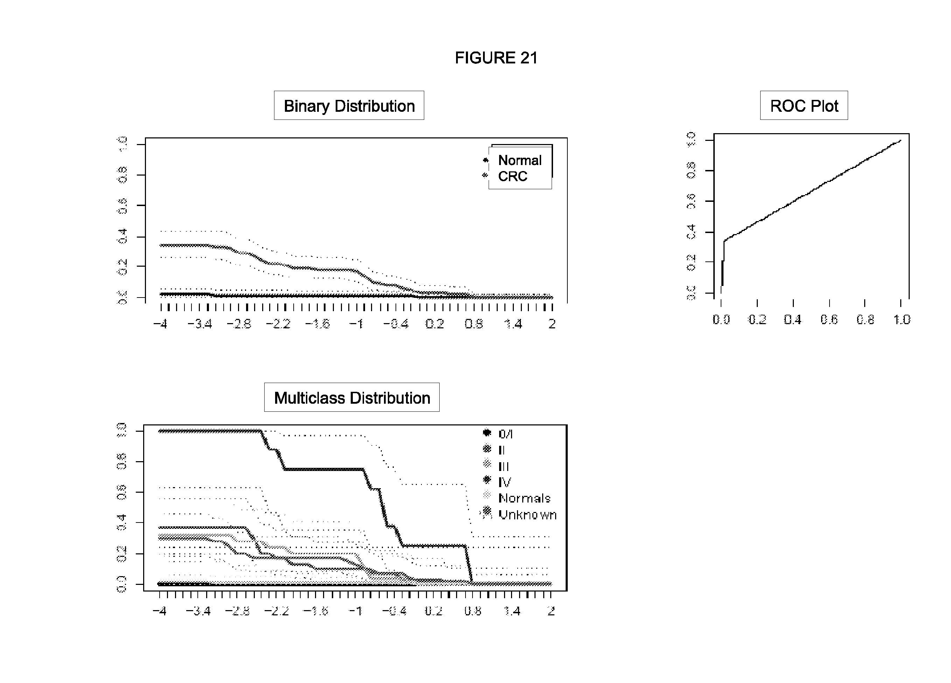

Figure 21 provides an overview of the performance of the TFAP2E HM assay according to Example 2, in all colorectal carcinoma and normal colorectal tissue samples.

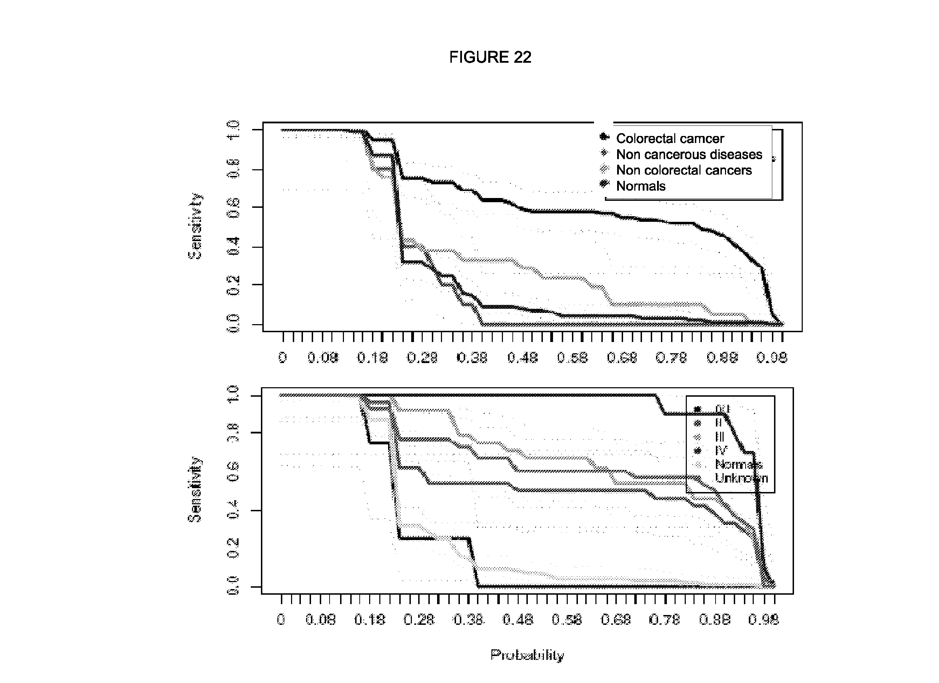

Figures 22 to 26 provide an overview of the log mean methylation measured by means of combinations HM assays (gene panels) according to Example 2. Each figures consists of two plots, The upper plot shows all samples (Normals, Non Colorectal Disease, Non-Coloretal Cancers and all CRC stages), the lower plot shows only Normaland CRC samples.
sensitivity is shown on the Y-axis, DNA methylation measured in (log 10 ng/mL) is shown on the X-axis.
Figure 22 provides an overview of the performance of the Septin 9+TFAP2E+RASSF2+PCDHGC3+SND1 assays.

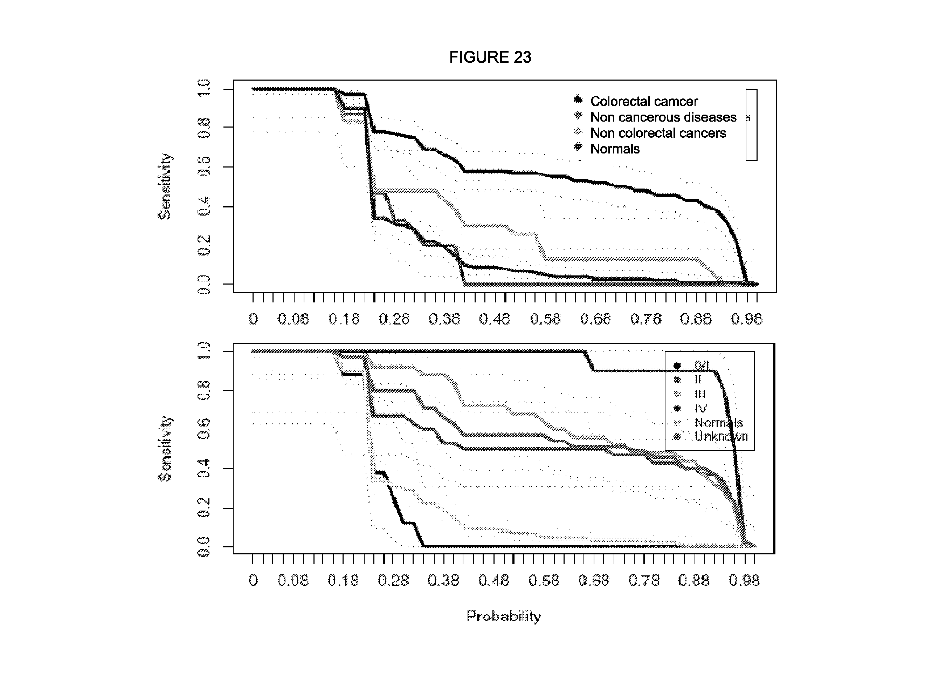

Figure 23 provides an overview of the performance of the Septin 9+TFAP2E+RASSF2+PCDHGC3 assays.

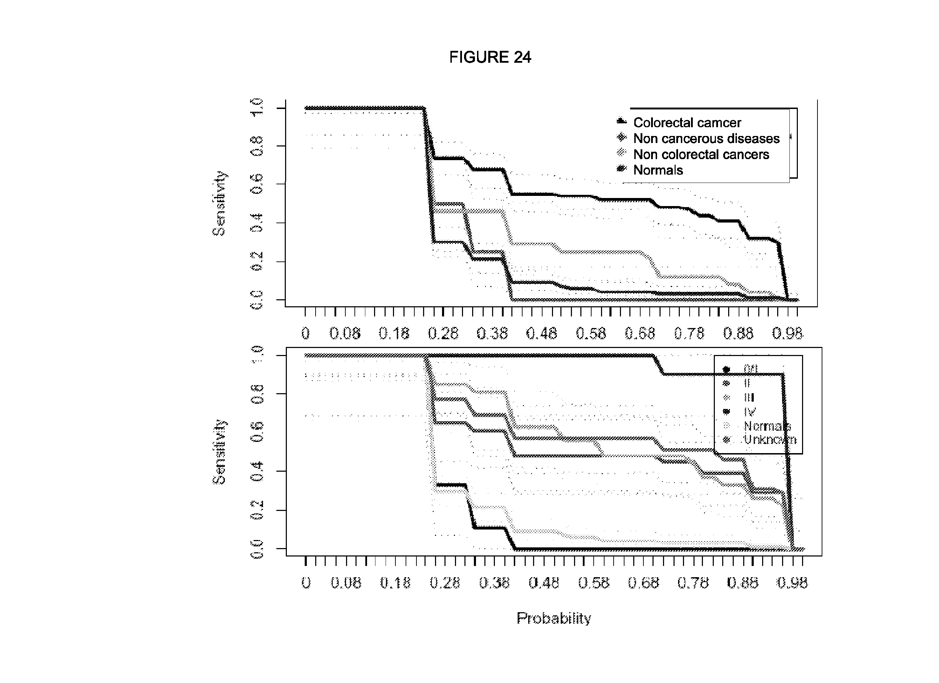

Figure 24 provides an overview of the performance of the Septin 9+TFAPE+RASSF2 assays.

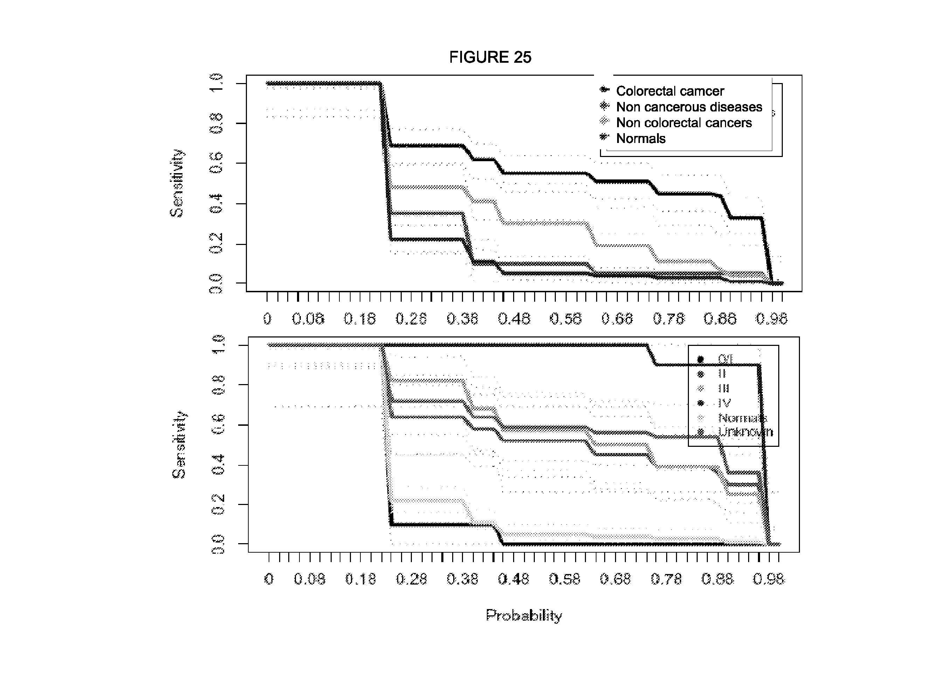

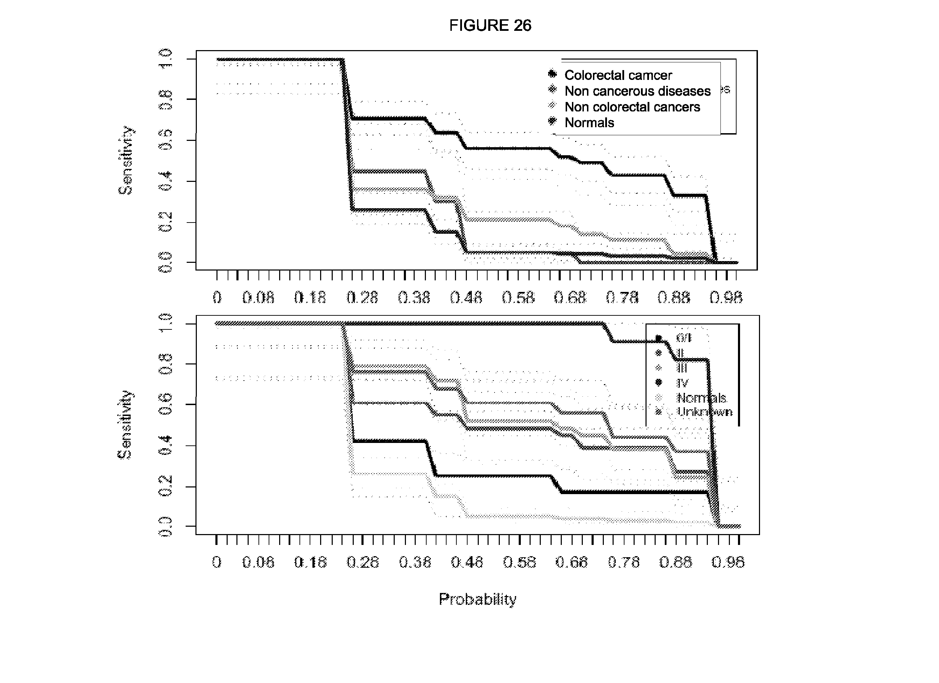

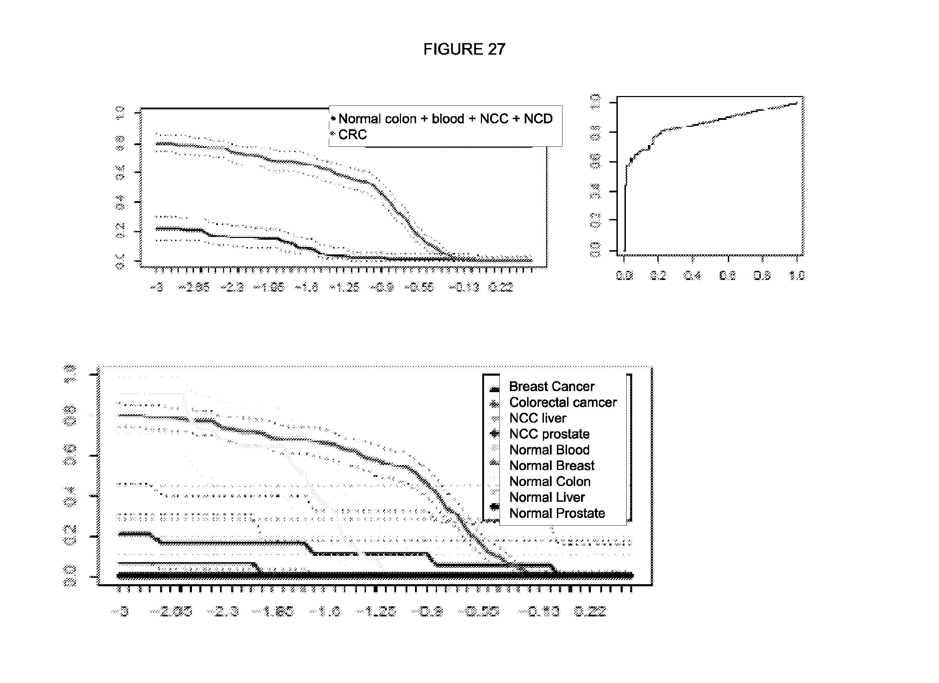

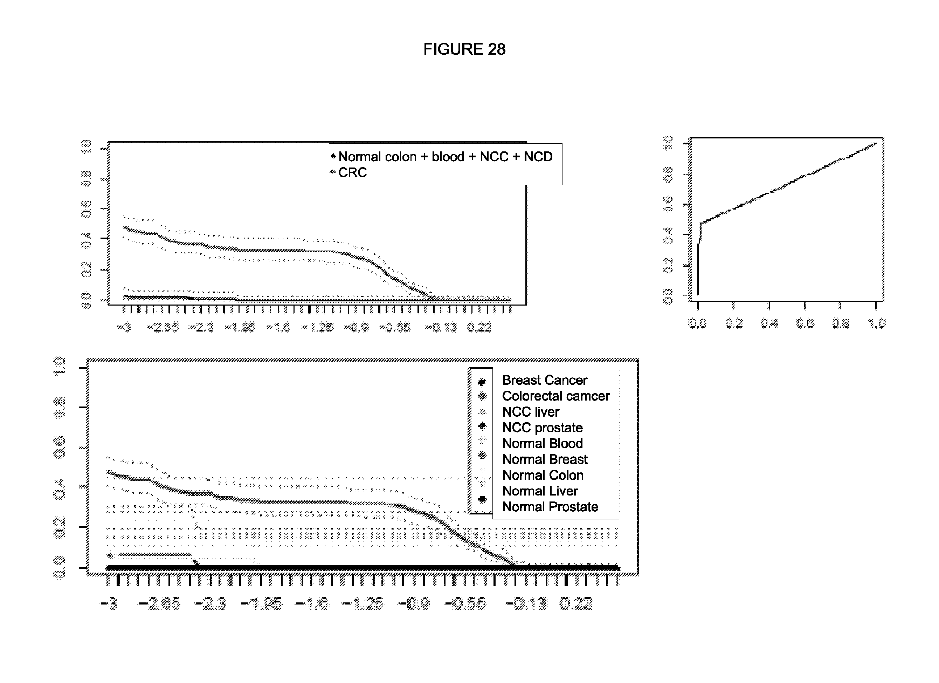

Figure 25 provides an overview of the performance of the Septin 9+TFAP2E assays. Figure 26 provides an overview of the performance of the Septin 9+RASSF2 assays. Figures 27 and 28 provide an overview of the methylation measured by means of the MSP assay according to Example 1. Each figure consists of three plots, the upper and lower left hand side plots provide the binary and multiclass analysis respectively, sensitivity is shown on the Y-axis, DNA methylation is shown on the X-axis.

In each figure the right hand plot provides an ROC wherein sensitivity is shown on the Y-axis and 1-specificity is shownon the X-axis. Figure 27 provides an overview of the MRPS21 assay according to Example 1. Figure 28 provides an overview of the DOCK10 assay according to Example 1.

### SUMMARY OF THE INVENTION

The present invention provides a method for detecting colorectal cell proliferative disorders, as defined in the claims. Disclosed is determining the expression levels of the gene RASSF2 in a biological sample isolated from said subject wherein underexpression and/or CpG methylation is indicative of the presence or class of said disorder

Aspects of the invention provide a method for detecting colorectal cell proliferative disorders in a subject comprising determining the methylation level of the gene Septin 9 and RASSF2.

Various aspects of the present invention provide an efficient and unique genetic marker, whereby expression analysis of said marker enables the detection of cellular proliferative disorders with a particularly high sensitivity, specificity and/or predictive value. In the context of colorectal carcinoma the inventive testing methods have particular utility for the screening of at-risk populations. The inventive methods have advantages over prior art methods (including the industry standard FOBT), because of improved sensitivity, specificity and likely patient compliance.

The kit of the present invention is most preferably utilised for detecting colorectal carcinoma.

The nucleotide sequence of said target region comprises at least one CpG dinucleotide sequence of the gene Septin 9 and RASSF2.

Preferably, the sensitivity of said detection is from about 75% to about 96%, or from about 80% to about 90%, or from about 80% to about 85%. Preferably, the specificity is from about 75% to about 96%, or from about 80% to about 90%, or from about 80% to about 85%. The method is novel as no methods cancer by analysis of body fluids, with a sensitivity and specificity high enough for use in a commercially available and regulatory body approved assay. For example, current methods used to detect and diagnose colorectal carcinoma include colonoscopy, sigmoidoscopy, and fecal occult blood colon cancer. In comparison to these methods, the disclosed invention is much less invasive than colonoscopy, and as, if not more sensitive than sigmoidoscopy and FOBT. The development of a body fluid assay represents a clear technical advantage over current methods known in the art in that it is anticipated that at least for colorectal carcinoma screening patient compliance for a single body fluid based test will be higher than the triplicate analysis of stool currently recommended for FOBT.

The present invention is particularly suited for the detection of colorectal cancerous cellular proliferative disorders. The methods of the present invention are particularly effective in the detection of colorectal which is enabled by means of analysis of the *methylation status* of RASSF2, and its promoter or regulatory elements.. It is further preferred that the detection of colorectal cell proliferative disorders is enabled by means of analysis of the *methylation status* of the gene Septin 9 in combination with RASSF2, and its promoter or regulatory elements

Disclosed is a method for the analysis of biological samples for features associated with the development of cellular proliferative disorders, the method characterised in that at least one nucleic acid, or a fragment thereofaccording to SEQ ID NO: 7 is contacted with a reagent or series of reagents capable of distinguishing between methylated and non methylated CpG dinucleotides within the genomic sequence, or sequences of interest.

It is prefered that at least one nucleic acid, or a fragment thereof, of SEQ ID NO: 12 TO SEQ ID NO: 14 and at least one nucleic acid, or a fragment thereof according to SEQ ID NO: 7is contacted with a reagent or series of reagents capable of distinguishing between methylated and non methylated CpG dinucleotides. Disclosed is a method for ascertaining epigenetic parameters of genomic DNA associated with the development of neoplastic cellular proliferative disorders (e.g. cancers). The method has utility for the improved diagnosis, treatment and monitoring of said diseases. Preferably, the source of the test sample is selected from the group consisting of cells or cell lines, histological slides, biopsies, paraffin-embedded tissue, body fluids, ejaculate, stool, urine, blood, and combinations thereof. More preferably, the source is selected from the group consisting of stool, blood plasma, blood serum, whole blood, isolated blood cells, cells isolated from the blood obtained from the subject.

Specifically, disclosed is a method for detecting neoplastic cellular proliferative disorders (preferably colorectal carcinoma) including at early stages, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within at least one target sequence of the subject nucleic acid, wherein the target sequence comprises, or hybridizes under stringent conditions to, a sequence comprising at least 16 contiguous nucleotides of a sequence according to SEQ ID NO: 7, said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. It is further preferred that at least two target sequences are analyzed wherein a first target sequence comprises, or hybridizes under stringent conditions to, a sequence comprising at least 16 contiguous nucleotides of a sequence selected from the group consisting SEQ ID NO: 12 to SEQ ID NO: 14 and a second target region comprises, or hybridizes under stringent conditions to, a sequence comprising at least 16 contiguous nucleotides of a sequence according to SEQ ID NO: 7.

Preferably, distinguishing between methylated and non methylated CpG dinucleotide sequences within the target sequence comprises methylation state dependent conversion or non-conversion of at least one such CpG dinucleotide sequence to the corresponding converted or non-converted dinucleotide sequence within a sequence selected from the group consisting of SEQ ID NO: 27 to SEQ ID NO: 28 and SEQ ID NO: 55 to SEQ ID NO: 56 , and contiguous regions thereof corresponding to the target sequence.

It is further preferred that distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within SEQ ID NO: 37toSEQ ID NO: 42 and SEQ ID NO: 65 to SEQ ID NO: 70 and at least one such CpG dinucleotide sequence to the corresponding converted or non-converted dinucleotide sequence selected from the group consisting of SEQ ID NO: 27 to SEQ ID NO: 28 and SEQ ID NO: 55 to SEQ ID NO:56 and contiguous regions thereof corresponding to the target sequence.

Additionally disclosed is a method for the detection of neoplastic cellular proliferative disorders, most preferably colorectal carcinoma, comprising: obtaining a biological sample having subject genomic DNA; extracting the genomic DNA; treating the genomic DNA, or a fragment thereof, with one or more reagents to convert 5- position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 27 to SEQ ID NO: 28 and SEQ ID NO: 55 toSEQ ID NO: 56, and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one , but more preferably a plurality of CpG dinucleotides of a sequence according to SEQ ID NO: 7. It is further preferred that said method additionally comprises contacting the treated genomic DNA, or the treated fragment thereof, with an amplification enzyme and least two primers comprising, in each case a contiguous sequence at least 9 nucleotides in length that is complementary to, or hybridizes under moderately stringent or stringent conditions to a sequence selected from the group consisting SEQ ID NO: 37 to SEQ ID NO: 42 and SEQ ID NO: 65 to SEQ ID NO: 70, and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one , but more preferably a plurality of CpG dinucleotides of a sequenceselected from the group consisting of SEQ ID NO: 12 to SEQ ID NO: 14.

Preferably, determining comprises use of at least one method selected from the group consisting of: I) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 27 to SEQ ID NO: 28 and SEQ ID NO: 55 to SEQ ID NO: 56 ( and complements thereof; ii) hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 27 to SEQ ID NO: 28 and SEQ ID NO: 55 to SEQ ID NO: 56 , and complements thereof; iii) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 27 to SEQ ID NO: 28 and SEQ ID NO: 55 to SEQ ID NO: 56 and complements thereof, and extending at least one such hybridized nucleic acid molecule by at least one nucleotide base; and iv) sequencing of the amplificate.

Further disclosed is a method for the analysis (i.e. detection) of cell proliferative disorders, comprising: obtaining a biological sample having subject genomic DNA; extracting the genomic DNA; contacting the genomic DNA, or a fragment thereof according to SEQ ID NO: 7 or a sequence that hybridizes under stringent conditions thereto with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of at least one genomic sequence according to SEQ ID NO: 7or an average, or a value reflecting an average methylation state of a plurality of CpG dinucleotide sequences thereof. Preferably, the digested or undigested genomic DNA is amplified prior to said determining.

It is further preferred that said method additionally comprises determining, based on a presence or absence of, or on property of at least one such fragment, the methylation state of at least one CpG dinucleotide sequence of at least one genomic sequence selected from the group consisting of SEQ ID NO: 12 to SEQ ID NO: 14, or an average, or a value reflecting an average methylation state of a plurality of CpG dinucleotide sequences thereof.

Additionally disclosed are genomic and chemically modified nucleic acid sequences, as well as oligonucleotides and/or PNA-oligomers for analysis of cytosine methylation patterns within the sequence according to SEQ ID NO: 7.

### DETAILED DESCRIPTION OF THE INVENTION

### Definitions:

The term "Observed/Expected Ratio" ("0/E Ratio") refers to the frequency ofCpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 KB, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analysed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers)* covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridisation" is to be understood as a bond of an oligonucleotide to a complementary sequence along the lines of the Watson-Crick base pairings in the sample DNA, forming a duplex structure.

"Stringent hybridisation conditions," as defined herein, involve hybridising at 68°C in 5x SSC/5x Denhardt's solution/1.0% SDS, and washing in 0.2x SSC/0.1% SDS at room temperature, or involve the art-recognized equivalent thereof (e.g., conditions in which a hybridisation is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "Methylation-specific restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5. "Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."

In reference to composite array sequences, the phrase "contiguous nucleotides" refers to a contiguous sequence region of any individual contiguous sequence of the composite array, but does not include a region of the composite array sequence that includes a "node," as defined herein above.

The term "gene" shall be taken to include all transcript variants thereof (e.g. the term "Septin 9" shall include for example its truncated transcript Q9HC74) and all promoter and regulatory elements thereof. Furthermore as a plurality of SNPs are known within said gene the term shall be taken to include all sequence variants thereof.

### Overview:

The present invention provides a method for detecting colorectal carcinoma, as defined in the claims.

Preferably a plurality of genes are analyzed. Preferably 2 genes are analyzed. Particularly preferred is the following combinations of genes:
-Septin 9+RASSF2

The markers are particularly efficient in detecting or distinguishing between colorectal cell proliferative disorders, thereby providing improved means for the early detection and thus improved treatment of said disorders. The disclosure presents a panel of genes comprising the gene RASSF2 with novel utility for the detection of cancers, in particular colorectal cancer. Preferably said gene comprise at least the gene RASSF2, and/or its promotor or regulatory regions. In a preferred method said panel comprises the gene SEPTIN 9 and the gene RASSF2 .

A first further disclosure is based upon the analysis of CpG methylaton status the gene RASSF2. It is preferred that the sequences of said gene is as according to Table 1 and/or its promotor or regulatory regions. It is preferred that the sequences of said genes are as according to Table 7. *Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g.,* PCR amplification. The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited. The prior art, in terms of sensitivity, is defined by a method comprising enclosing the DNA to be analysed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyse individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g.,* Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridisation has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

The present invention provides for the use of the bisulfite technique, in combination with one or more methylation assays, for determination of the methylation status of CpG dinucleotide sequences, as defined in the claims.

Genomic CpG dinucleotides can be methylated or unmethylated (alternatively known as up- and down- methylated respectively). However the methods of the present invention are suitable for the analysis of biological samples of a heterogeneous nature e.g. a low concentration of tumor cells within a background of blood or stool. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position. Unless specifically stated the terms "hypermethylated" or "unmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) Determination of the methylation status of CpG dinucleotide sequences within SEQ ID NO: 7 has utility both in the diagnosis and characterization of cellular proliferative disorders.

*Methylation Assay Procedures.* Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommeret al., Proc. Natl. Acad. Sei. USA 89:1827-1831, 1992). Additionally, restriction enzyme digestion of PCR products amplified from bisulfite-converted DNA is used, *e.g.,* the method described by Sadri & Hornsby (Nucl. Acids Res. 24:5058-5059, 1996), or COBRA (Combined Bisulfite Restrietion Analysis) (Xiong & Laird, Nucleic Acids Res. 25:2532-2534,1997). *COBRA.* COBRATM analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (*e.g*., as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (*e.g.,* precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999), Ms-SNuPE™ (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

The "HeavyMethyl™" assay, technique is a quantitative method for assessing methylation differences based on methylation specific amplification of bisulfite treated DNA. Methylation specific *blocking* probes (also referred to herein as *blockers)* covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers. The HeavyMethyl™ assay may also be used in combination with methylation specific amplification primers.

Typical reagents (*e.g*., as might be found in a typical MethyLig 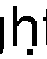 t□-based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1 % methylated alleles of a given CpG island locus, and can be performed on DNA extracted from paraffin-embedded samples. Typical reagents (*e.g*., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MethyLig* 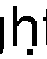 *t*™*.* The MethyLig 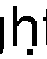 t™ assay is a high-throughput quantitative methylation assay that utilizes fluorescence-based real-time PCR (TaqMa 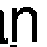 □) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process.

The MethyLig 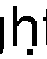 t™ assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLig 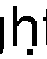 tT"" process can by used with any suitable probes e.g. "TaqMan®" , Lightcycler® etc.... For example, double-stranded genomic DNA is treated with sodium bisulfite and subjected to one of two sets of PCR reactions using TaqMan® probes; e.g., with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g*., as might be found in a typical MethyLig 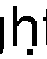 t□-based kit) for MethyLig 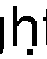 t™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The Q 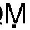 ™ (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl**™** and MSP techniques), or with oligonucleotides covering potential methylation sites.

The Q 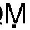 ™ process can by used with any suitable probes e.g. "TaqMan®" , Lightcycler® etc... in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (e.g., as might be found in a typical Q 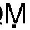 ™ -based kit) for Q 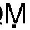 ™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

*Ms-SNuPE.* The Ms-SNuPE™ technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g.*, microdissected pathology sections), and it avoids utilization of restriction enzymes for determining the methylation status at CpG sites.

Typical reagents (*e.g*., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for specific gene; reaction buffer (for the Ms-SNuPE reaction); and labelled nucleotides.

Additionally, bisulfite conversion reage,ts may include: DNA denaturation buffer; sulfonation buffer; DNA recovery regents or kit (e.g., precipitation, Ultrafiltration, affinity column); desulfonation buffer; and DNA recovery components.

The genomic sequence according to SEQ ID NO:7 and non-naturally occurrinq treated variants thereof according to SEQ ID NO:27 to SEQ ID NO:28 and SEQ ID NO:55 to SEQ ID NO:56 were determined to have novel utility for the early detection of cellular proliferative disorders, in particular colorectal cell proliferative disorders.

Disclosed is a method which comprises the following steps: i) contacting genomic DNA (preferably isolated from body fluids) obtained from the subject with at least one reagent, or series of reagents that distinguishes between methylated and non-methylated CpG dinucleotides within the gene RASSF2 (including its promoter and regulatory regions); and ii) detecting, or detecting and distinguishing between or among colon cell proliferative disorders. Preferably the accuracy of said method is increased by additionally determining the methylation status of the gene SEPTIN 9 by means of said method steps. Particularly preferred is the combination of Septin 9 and RASSF2.

Preferably, the sensitivity is from about 75% to about 96%, or from about 802% to about 90%, or from about 80% to about 85%. Preferably, the specificity is from about 75% to about 96%, or from about 80% to about 90%, or from about 80% to about 85%.

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising cellular matter are suitable for us e in the present method, preferred are cell lines, histological slides, biopsies, paraffin-embedded tissue, body fluids, stool, colanie effluent, urine, blood plasma, blood serum, whole blood, isolated blood cells, cells isolated from the blood and combinations thereof. Body fluids are the preferred source of the DNA; particularly preferred are blood plasma, blood serum, whole blood, isolated blood cells and cells isolated from the blood. The genomic DNA sample is then treated with at least one reagent, or series of reagents that distinguishes between methylated and non-methylated CpG dinucleotides within at least one target region of the genomic DNA, wherein the target region comprises, or hybridizes under stringent conditions to a sequence of at least 16 contiguous nucleotides of the sequenc according to SEQ ID NO: 7respectively, wherein said contiguous nucleotides comprise at least one CpG dinucleotide sequence. It is further preferred that said reagent, or series of reagents distinguishes between methylated and non-methylated CpG dinucleotides within at least two target regions of the genomic DNA, wherein a first the target region comprises, or hybridizes under stringent conditions to a sequence of at least 16 contiguous nucleotides of at least one sequence selected from the group consisting of SEQ ID NO: 12 to SEQ ID NO: 14 and a second target region that comprises, or hybridizes under stringent conditions to a sequence of at least 16 contiguous nucleotides of the sequence according to SEQ ID NO: 7. wherein said contiguous nucleotides comprise at least one CpG dinucleotide sequence.

It is particularly preferred that said reagent converts cytosine bases which are unmethylated at the 5'-position to uracil, thymine, or another base which is dissimilar to cytosine in terms of hybridization behaviour. However in an alternative embodiment said reagent may be a methylation sensitive restriction enzyme.

Wherein the genomic DNA sample is treated in such a manner that cytosine bases which are unmethylated at the 5'-position are converted to uracil, thymine, or another base which is dissimilar to cytosine in terms of hybridization behavior It is preferred that this treatment is carried out with bisulfite (hydrogen sulfite, disulfite) and subsequent alkaline hydrolysis. Such a treatment results in the conversion of SEQ ID NO: 7 and SEQ ID NO: 12 to 14, , SEQ ID NO: 55, SEQ ID NO: 56 and SEQ ID NO: 65 to SEQ ID NO: 70 wherein said CpG dinucleotides are unmethylated and to SEQ ID NO: 28 andSEQ ID NO: 37 to SEQ ID NO: 42wherein said CpG dinucleotides are methylated.

The treated DNA is then analyzed in order to determine the methylation state of the target gene sequence (the gene RASSF2, prior to the treatment). It is particularly preferred that the target region comprises, or hybridizes under stringent conditions to at least 16 contiguous nucleotides of thegene RASSF2,. It is preferred that the sequence of said gene according to SEQ ID NO: 7 is analyzed

The method of analysis may be selected from those known in the art, including those listed herein. Particularly preferred are MethylightTM, MSP and the use of blocking oligonucleotides (HeavyMethyl™) as described herein. It is further preferred that any oligonucleotides used in such analysis (including primers, blocking hybridize under stringent or highly stringent conditions to an at least 16-base-pair long segment of the base sequences of one or more of SEQ ID NO: 27 to SEQ ID NO: 28 and SEQ ID NO: 55 to SEQ iD NO: 56 and sequences complementary thereto.

Aberrant methylation, more specifically hypermethylation of the gene or genomic sequence of RASSF2 (including its promoter and/or regulatory regions) is associated with the presence of neoplastic cellular proliferative disorders, and is particularly prevalent in colorectal carcinomas. Accordingly wherein a biological sample presents within any degree of methylation, said sample should be determined as cancerous.

Analysis of the geneor genomic sequence of RASSF2 enables for the first time detecting, or detecting and distinguishing between or among colon cell proliferative disorders afforded with a sensitivity of greater than or equal to 80% and a specificity of greater than or equal to 80%.

Sensitivity is calculated as (detected neoplasia/all neoplasia) e.g. (detected colon neoplasia/all colon neoplasia); and specificity is calculated as (non-detected negatives/total negatives)

Preferably, the sensitivity is from about 75% to about 96%, or from about 80% to about 90%, or from about 80% to about 85%. Preferably, the specificity is from about 75% to about 96%, or from about 80% to about 90%, or from about 80% to about 85%.

Colon neoplasia is herein defined as all colon malignancies and adenomas greater than 1 cm., or subsets thereof. Negatives can be defined as healthy individuals.

Disclosed is the use of the gene RASSF2 (or promoter and/or regulatory regions thereof) as a marker for the detection of cellular proliferative disorders (in particular colon disorders).

Said method may be enabled by means of any analysis of the expression of an RNA transcribed therefrom or polypeptide or protein translated from said RNA, preferably by means of mRNA expression analysis or polypeptide expression analysis. Further disclosed diagnostic assays and methods, both quantitative and qualitative for detecting the expression of thegene RASSF2 in a subject and determining therefrom upon the presence or absence of cancer in said subject. Even further disclosed are diagnostic assays and methods, both quantitative and qualitative for detecting the expression of the gene SEPTIN 9 in combination with the gene RASSF2 in a subject and determining therefrom upon the presence or absence of cancer in said subject.

Aberrant expression of mRNA transcribed from the gene or genomic sequence of RASSF2 is associated with the presence of cancer in a subject. According to the present invention, presence of methylation is associated with the presence of cancer, and vice versa absence of methylation is associated with the absence of cancer. To detect the presence of mRNA encoding a gene, a sample is obtained from a patient. The sample may be any suitable sample comprising cellular matter of the tumour. Suitable sample types include cell lines, histological slides, biopsies, paraffin-embedded tissue, body fluids, stool, colonic effluent, urine, blood plasma, blood serum, whole blood, isolated blood cells, cells isolated from the blood and all possible combinations thereof. It is preferred that said sample types are stool or body fluids selected from the group consisting colonic effluent, urine, blood plasma, blood serum, whole blood, isolated blood cells, cells isolated from the blood.

The sample may be treated to extract the RNA contained therein. The resulting nucleic acid from the sample is then analysed. Many techniques are known in the state of the art for determining absolute and relative levels of gene expression, commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligonucleotide dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987).

Further preferred is the "Real-time" variant of RT- PCR, wherein the PCR product is detected by means of hybridisation probes (e.g. TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridisation to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, mass labels or fluorescent labels). The size of the target RNA, not the probe, will determine the size of the detected band, so methods such as random-primed labelling, which generates probes of variable lengths, are suitable for probe synthesis. The specific activity of the probe will determine the level of sensitivity, so it is preferred that probes with high specific activities, are used..

In an RNase protection assay, the RNA target and an RNA probe of a defined length are hybridised in solution. Following hybridisation, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length single-stranded RNA probes and the reproducibility and reliability of RNA:RNA duplex digestion with RNases (Ausubel et al. 2003),particularly preferres are probes with high specificity.

Particularly preferred is the use of microarrays. The microarray process can be divided into two main parts. First is the immobilization of known gene sequences onto glass slides or other solid support followed by hybridization of the fluorescently labelled cDNA (comprising the sequences to be interrogated) to the known genes immobilized on the glass slide (or other solid phase). After hybridization, arrays are scanned using a fluorescent microarray scanner. Analyzing the relative fluorescent intensity of different genes provides a measure of the differences in gene expression. DNA arrays can be generated by immobilizing presynthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of the genes of interest (in this case the gene or genomic sequence of RASSF2 and Septin 9) and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Preferably said synthesized nucleic acids are locked nucleic acids In expression profiling microarray experiments, the RNA template used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labelling of the cDNA can be accomplished by either direct labelling or indirect labelling methods. During direct labelling, fluorescently modified nucleotides (e.g., Cy®3- or Cy®5-dCTP) are incorporated directly into the cDNA during the reverse transcription. Alternatively, indirect labelling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy^{®}3. The resulting labelled cDNA is purified to remove unincorporated nucleotides, free dye and residual RNA. Following purification, the labelled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridisation using a fluorescent microarray scanner. The fluorescent intensity of each spot indicates the level of expression of the analysed gene; bright spots correspond to strongly expressed genes, while dim spots indicate weak expression..

Once the images are obtained, the raw data must be analysed. First, the background fluorescence must be subtracted from the fluorescence of each spot. The data is then normalized to a control sequence, such as exogenously added nucleic acids (preferably RNA or DNA), or a housekeeping gene panel to account for any non-specific hybridisation, array imperfections or variability in the array set-up, cDNA labelling, hybridisation or washing. Data normalization allows the results of multiple arrays to be compared.

Another aspect of the invention relates to a kit for use in diagnosis of cancer in a subject according to the methods of the present invention, as defined in claim 10.

Disclosed is that the means for measuring the level of transcription comprise oligonucleotides or polynucleotides able to hybridize under stringent or moderately stringent conditions to the transcription products of the RASSF2 It is further preferred that said kit further comprises means for measuring the level of transcription of the gene Septin 9 comprising oligonucleotides or polynucleotides able to hybridize under stringent or moderately stringent conditions to the transcription products of said gene. Preferably, said oligonucleotides or polynucleotides comprise at least 9, 18 or 25 bases of a sequence complementary to or hybridizing to said transcription product. Disclosed is that the level of transcription is determined by techniques selected from the group of Northern Blot analysis, reverse transcriptase PCR, real-time PCR, RNAse protection, and microarray. Alternatively, the kit further comprises means for obtaining a biological sample of the patient. Preferred is a kit, which further comprises a container which is most preferably suitable for containing the means for measuring the level of transcription and the biological sample of the patient, and most preferably further comprises instructions for use and interpretation of the kit results. In a preferred disclosure the kit comprises (a) a plurality of oligonucleotides or polynucleotides able to hybridize under stringent or moderately stringent conditions to the transcription products of thegene RASSF2 (b) a container, preferably suitable for containing the oligonucleotides or polynucleotides and a biological sample of the patient comprising the transcription products wherein the oligonucleotides or polynucleotides can hybridize under stringent or moderately stringent conditions to the transcription products, (c) means to detect the hybridization of (b); and optionally, (d) instructions for use and interpretation of the kit results. It is further preferred that said oligonucleotides or polynucleotides of (a) comprise in each case at least 9, 18 or 25 bases of a sequence complementary to or hybridizing to the transcription products of the RASSF2.

It is further preferred that said kit comprises (e) a plurality of oligonucleotides or polynucleotides able to hybridize under stringent or moderately stringent conditions to the transcription products of the gene Septin 9. Said oligonucleotides or polynucleotides of (e) preferably comprise in each case at least 9, 18 or 25 bases of a sequence complementary to or hybridizing to a Septin 9 transcription products. The kit may also contain other components such as hybridization buffer (where the oligonucleotides are to be used as a probe) packaged in a separate container. Alternatively, where the oligonucleotides are to be used to amplify a target region, the kit may contain, packaged in separate containers, a polymerase and a reaction buffer optimized for primer extension mediated by the polymerase, such as PCR. Preferably said polymerase is a reverse transcriptase. It is further preferred that said kit further contains an Rnase reagent.

Further disclosed are methods for the detection of the presence of the polypeptide encoded by said gene sequences in a sample obtained from a patient.

Aberrant levels of polypeptide expression of the polypeptide encoded by the gene or genomic sequence of RASSF2 is associated with the presence of cancer. The accuracy of the diagnosis of colorectal cancer is increased when polypeptides of the gene Septin 9 are analyzed in combination with said aforementioned polypeptide.

Under expression of said polypeptides is associated with the presence of cancer. Any method known in the art for detecting polypeptides can be used. Such methods include, but are not limited to mass-spectrometry, immunodiffusion, immunoelectrophoresis, immunochemical methods, binder-ligand assays, immunohistochemical techniques, agglutination and complement assays (e.g., see Basic and Clinical Immunology, Sites and Terreds., Appleton & Lange, Norwalk, Conn. pp 217-262,1991). Preferred are binder-ligand immunoassay methods including reacting antibodies with an epitope or epitopes and competitively displacing a labelled polypeptide or derivative thereof.

The disclosure comprises the use of antibodies specific to the polypeptide encoded by thr gene RASSF2.

Further parts of the disclosure comprise the use of a first species of antibodies specific to the polypeptide encoded by the gene SEPTIN 9 and a second species of antibodies specific to the polypeptide encoded by the gene RASSF2. Such antibodies are useful for cancer diagnosis. In certain embodiments production of monoclonal or polyclonal antibodies can be induced by the use of an epitope encoded by a polypeptide encoded by the gene RASSF2Such antibodies may in turn be used to detect expressed polypeptides as markers for cancer diagnosis. The levels of such polypeptides present may be quantified by conventional methods. Antibody-polypeptide binding may be detected and quantified by a variety of means known in the art, such as labelling with fluorescent or radioactive ligands. Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescens, chemiluminescens, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

Alternatively, proteins may be detected by means of western blot analysis. Said analysis is standard in the art, briefly proteins are separated by means of electrophoresis e.g. SOS-PAGE. The separated proteins are then transferred to a suitable membrane (or paper) e.g. nitrocellulose, retaining the spacial separation achieved by electrophoresis. The membrane is then incubated with a blocking agent to bind remaining sticky places on the membrane, commonly used agents include generic protein (e.g. milk protein). An antibody specific to the protein of interest is then added, said antibody being detectably labelled for example by dyes or enzymatic means (e.g. alkaline phosphatase or horseradish peroxidase). The location of the antibody on the membrane is then detected. Alternatively, the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case. One approach for preparing antibodies to a polypeptide is the selection and preparation of an amino acid sequence of all or part of the polypeptide, chemically synthesizing the amino acid sequence and injecting it into an appropriate animal, usually a rabbit or a mouse (Milstein and Kahler Nature 256:495-497, 1975; Gulfre and Milstein, Methods in Enzymology: Immunochemical Techniques 73:1-46, Langone and Banatis eds., Academic Press, 1981). Methods for preparation of the polypeptides or epitopes thereof include, but are not limited to chemical synthesis, recombinant DNA techniques or isolation from biological samples. In the final step of the method the diagnosis of the patient is determined, whereby underexpression (of at thegenes RASSF2.

and Septin 9 is indicative of the presence of cancer. The term under-expression shall be taken to mean expression at a detected level less than a pre-determined cut off which may be selected from the group consisting of the mean, median or an optimized threshold value. Disclosed is further a kit for use in diagnosis of cancer in a subject comprising: a means for detecting a polypeptide of thegene RASSF2. The means for detecting the polypeptide comprise preferably antibodies, antibody derivatives, or antibody fragments. The polypeptide ismost preferably detected by means of Western Blotting utilizing a labelled antibody. The kit may further comprise means for obtaining a biological sample of the patient. Preferred is a kit, which further comprises a container suitable for containing the means for detecting the polypeptide in the biological sample of the patient, and most preferably further comprises instructions for use and interpretation of the kit results. The kit may comprise (a) a means for detecting a polypeptide of thegene RASSF2; (b) a container suitable for containing the said means and the biological sample of the patient comprising the polypeptides wherein the means can form complexes with the polypeptide; (c) a means to detect the complexes of (b); and optionally (d) instructions for use and interpretation of the kit results. The kit may also contain other components such as buffers or solutions suitable for blocking, washing or coating, packaged in a separate container. Disclosed is a novel application of the analysis of methylation levels and/or patterns within said sequences that enables a precise detection, characterization and/or treatment of colorectal cell proliferative disorders. Early detection of cancer is directly linked with disease prognosis, and the disclosed method thereby enables the physician and patient to make better and more informed treatment decisions.

### FURTHERIMPROVEMENTS

Disclosed are novel uses for the genomic sequence SEQ ID NO: 7 Modified variants of SEQ ID NO: 7as well as oligonucleotides and/or PNA-oligomers for analysis of cytosine methylation patterns within SEQ ID NO: 7 An objective of the disclosure is analysis of the methylation state of one or more CpG dinucleotides within at least one sequence selected from the group consisting of SEQ ID NO: 7, SEQ ID NO: 12 TO SEQ ID NO: 14 and sequences complementary thereto.

Disclosed are treated nucleic acids, derived from genomic SEQ ID NO: 7, SEQ ID NO: 12 to SEQ ID NO: 14, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. Preferred is a non-naturally occurring modified nucleic acid comprising a sequence of at least 16 contiguous nucleotide bases in length of a sequence selected from the group consisting of SEQ ID NO: 27 to SEQ ID NO: 28 and SEQ ID NO: 37 to SEQ ID NO: 42 and SEQ ID NO: 55 to SEQ ID NO: 56 and to SEQ ID NO: 65 to SEQ ID NO: 70. Preferably, said nucleic acid is at least 50, 100, 150, 200, 250 or 500 base pairs in length of a segment of the nucleic acid sequence disclosed in SEQ ID NO: 27 to SEQ ID NO: 28 and SEQ ID NO: 55 to SEQ ID NO: 56. Particularly preferred is a nucleic acid molecule that is not identical or complementary to all or a portion of the sequences SEQ ID NO: 27 to SEQ ID NO: 28 and SEQ ID NO: 37 to SEQ ID NO: 42 and SEQ ID NO: 55 to SEQ ID NO: 56 and to SEQ ID NO: 65 to SEQ ID NO: 70. but not SEQ ID NO: 7, SEQ ID NO: 12 to SEQ ID NO: 14 or other naturally occurring DNA It is preferred that said sequence comprises at least one CpG, TpA or CpA dinucleotide and sequences complementary thereto. The sequences of SEQ ID NO: 27 to SEQ ID NO: 28 and SEQ ID NO: 37 to SEQ ID NO: 42 and SEQ ID NO: 55 to SEQ ID NO: 56 and to SEQ ID NO: 65 to SEQ ID NO: 70provide non-naturally occurring modified versions of the nucleic acid according to SEQ ID NO: 7, SEQ ID NO: 12 to SEQ ID NO: 14 wherein the modification of each genomic sequence results in the synthesis of a nucleic acid having a sequence that is unique and distinct from said genomic sequence as follows. For each sense strand genomic DNA converted versions are disclosed. A first version wherein "C" is converted to "T," but "CpG" remains "CpG" (i.e., corresponds to case where, for the genomic sequence, all "C" residues of CpG dinucleotide sequences are methylated and are thus not converted); a second version discloses the complement of the disclosed genomic DNA sequence (i.e. antisense strand), wherein "C" is converted to "T," but "CpG" remains "CpG" (i.e., corresponds to case where, for all "C" residues of CpG dinucleotide sequences are methylated and are thus not converted). The 'upmethylated' converted sequences of SEQ ID NO: 7, SEQ ID NO: 12 to SEQ ID NO: 14 correspond to SEQ ID NO: 27 to SEQ ID NO: 28 and SEQ ID NO: 37 ro SEQ ID NO: 42. A third chemically converted version of each genomic sequences is provided, wherein "C" is converted to "T" for all "C" residues, including those of "CpG" dinucleotide sequences (i.e., corresponds to case where, for the genomic sequences, all "C" residues of CpG dinucleotide sequences are unmethylated); a final chemically converted version of each sequence, discloses the complement of the disclosed genomic DNA sequence (i.e. antisense strand), wherein "C" is converted to "T" for all "C" residues, including those of "CpG" dinucleotide sequences (i.e., corresponds to case where, for the complement (antisense strand) of each genomic sequence, all "C" residues of CpG dinucieotide sequences are unmethylated). The 'downmethylated' converted sequences of SEQ ID NO: 7, SEQ ID NO: 12 TO SEQ ID NO: correspond to SEQ ID NO: 55 to SEQ ID NO: 56 and SEQ ID NO: 65 to SEQ ID NO: 70. See Table 1 for further details. Significantly, heretofore, the nucleic acid sequences and molecules according SEQ ID NO: 27 to SEQ ID NO: 28 and SEQ ID NO: 55 to SEQ ID NO: 56 were not implicated in or connected with the detection of cellular proliferative disorders.

Further disclosed are oligonucleotides or oligomers suitable for use in the methods for detecting the cytosine methylation state within genomic or treated (chemically modified) DNA, according to SEQ ID NO: 27 to SEQ ID NO: 28, SEQ ID NO: 37 to SEQ ID NO: 42 and SEQ ID NO: 55 to SEQ ID NO: 56 and to SEQ ID NO: 65 to SEQ ID NO: 70 SEQ ID NO: 7, SEQ ID NO: 12 to SEQ ID NO: 14. Said oligonucleotide or oligomer nucleic acids provide novel diagnostic means. Said oligonucleotide or oligomer comprising a nucleic acid sequence having a length of at least nine (9) nucleotides which is identical to, hybridizes, under moderately stringent or stringent conditions (as defined herein above), to a treated nucleic acid sequence according to SEQ ID NO: 27 to SEQ ID NO: 28, SEQ ID NO: 37 to SEQ ID NO: 42, SEQ ID NO: 55, SEQ ID NO: 56, and SEQ ID NO: 65to SEQ ID NO: 70 and/or sequences complementary thereto, or to a genomic sequence according to SEQ ID NO: 12 TO SEQ ID NO: 14 and/or sequences complementary thereto.

Thus, disclosed are nucleic acid molecules (e.g.oligonucleotides and peptide nucleic acid (PNA) molecules (PNA-oligomers)) that hybridize under moderately stringent and/or stringent hybridization conditions to all or a portion of a sequence selected from the group consisting of SEQ ID NO: 27 to SEQ ID NO: 28, SEQ ID NO: 37 to SEQ ID NO: 42, SEQ ID NO: 55, SEQ ID NO: 56, SEQ ID NO: 65 to SEQ ID NO: 70, SEQ ID NO: 12 TO SEQ ID NO: 14 or to the complements thereof. Particularly preferred is a nucleic acid molecule that hybridizes under moderately stringent and/or stringent hybridization conditions to all or a portion of the sequences SEQ ID NO: 27 to SEQ ID NO: 28, SEQ ID NO: 37 to SEQ ID NO: 42, SEQ ID NO: 55, SEQ ID NO: 56, SEQ ID NO: 65 to SEQ ID NO: 70 but not SEQ ID NO: 12 to SEQ ID NO: 14 or other human genomic DNA.

The identical or hybridizing portion of the hybridizing nucleic acids is typically at least 9, 16, 20, 25, 30 or 35 nucleotides in length. However, longer molecules have utility.

Preferably, the hybridizing portion of the inventive hybridizing nucleic acids is at least 95%, or at least 98%, or 100% identical to the sequence, or to a portion thereof of a sequence selected from the group consisting of SEQ ID NO: 7 SEQ ID NO: 27 to SEQ ID NO: 28 and SEQ ID NO: 55 to SEQ ID NO: 56, or to the complements thereof.

Hybridizing nucleic acids of the type described herein can be used, for example, as a primer (e.g., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions. For target sequences that are related and substantially identical to the corresponding sequence of SEQ ID NO: 7, SEQ ID NO: 12 TO SEQ ID NO: 14 (such as allelic variants and SNPs), rather than identical, it is useful to first establish the lowest temperature at which only homologous hybridization occurs with a particular concentration of salt (e.g., SSC or SSPE). Then, assuming that 1% mismatching results in a 1°C decrease in the Tm, the temperature of the final wash in the hybridization reaction is reduced accordingly (for example, if sequences having >95% identity with the probe are sought, the final wash temperature is decreased by5°C). In practice, the change in Tm can be between 0.5°C and 1.5°C per 1% mismatch.

Examples of oligonucleotides of length X (in nucleotides), as indicated by polynucleotide positions include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:
n to (n + (X-1));
   where n=1, 2, 3, ... (Y-(X-1));
   where Y equals the length (nucleotides or base pairs) of SEQ ID NO: 1 (2519);
   where X equals the common length (in nucleotides) of each oligonucleotide in the set (e.g., X=20 for a set of consecutively overlapping 20-mers); and
   where the number (Z) of consecutively overlapping oligomers of length X for a given SEQ ID NO of length Y is equal to Y-(2519-1).

Preferably, the set is limited to those oligomers that comprise at least one CpG, TpG or CpA dinucleotide.

Preferably, the set is limited to those oligomers that comprise at least one CpG, TpG or CpA dinucleotide.

Likewise, examples of 25-mer oligonucleotides include the following set of 2,256 oligomers (and the antisense set complementary thereto).

Preferably, the set is limited to those oligomers that comprise at least one CpG,

TpG or CpA dinucleotide. Disclosed are for *each* of SEQ ID NO: 7 SEQ ID NO: 27 TO SEQ ID NO: 28 and SEQ ID NO: 55 TO SEQ ID NO: 56 (sense and antisense), multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers constitute effective tools useful to ascertain genetic and epigenetic parameters of the genomic sequences selected from the group consisting of SEQ ID NO: 7, SEQ ID NO: 12 to SEQ ID NO: 14 . Preferred sets of such oligonucleotides or modified oligonucleotides of length X are those consecutively overlapping sets of oligomers corresponding to SEQ ID NO: 7, SEQ ID NO: 12 to SEQ ID NO: SEQ ID NO: 27 to SEQ ID NO: 28, SEQ ID NO: 37 to SEQ ID NO: 42, SEQ ID NO. 55; SEQ ID NO: 56 and SEQ ID NO: 65 to SEQ ID NO: 70 (and to the complements thereof). Preferably, said oligomers comprise at least one CpG, TpG or CpA dinucleotide. Particularly preferred oligonucleotides or oligomers are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Kral et al., BioTechniques 6:958-976, 1988) or intercalating agents (Zon, Pharm. Res. 5:539-549, 1988). To this end, the oligonucleotide may be conjugated to another molecule, e.g., a Chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers are typically used in 'sets,' which contain at least one oligomer for analysis of each of the CpG dinucleotides of a genomic sequence according to SEQ ID NO: 7and sequences complementary thereto or the corresponding CpG, TpG or CpA dinucleotide within a sequence of the treated nucleic acids according to SEQ ID NO: 27 toSEQ ID NO: 28 and SEQ ID NO: 55 to SEQ ID NO: 56 and sequences complementary thereto. However, it is anticipated that for economic or other factors it may be preferable to analyze a limited selection of the CpG dinucleotides within said sequences, and the content of the set of oligonucleotides is altered accordingly. Therefore, particularly disclosed is a set of at least two (2) (oligonucleotides and/or PNA-oligomers) useful for detecting the cytosine methylation state in treated genomic DNA (SEQ ID NO: 27, SEQ ID NO: 28, SEQ ID NO: 37 to SEQ ID NO: 42, SEQ ID NO: 55, SEQ ID NO: 56, SEQ ID NO: 65 to SEQ ID NO: 70or in genomic DNA (SEQ ID NO: 12 to SEQ ID NO: 14and sequences complementary thereto). These probes enable diagnosis, classification and/or therapy of genetic and epigenetic parameters of colorectal cell proliferative disorders. The set of oligomers may also be used for detecting single nucleotide polymorphisms (SNPs) in treated genomic DNA (SEQ ID NO: 27, SEQ ID NO: 28, SEQ ID NO: 37 to SEQ ID NO: 42, SEQ ID NO: 55, SEQ ID NO: 56, SEQ ID NO: 65 to SEQ ID NO: 70 or in genomic DNA (SEQ ID NO: 7, SEQ ID NO: 12 to SEQ ID NO: 14 and sequences complementary thereto).

Preferably, at least one, and more preferably all members of a set of oligonucleotides is bound to a solid phase.

Disclosed is a set of at least two (2)oligonucleotides that are used as 'primer' oligonucleotides for amplifying DNA sequences of one of SEQ I D NO: 7, SEQ ID NO: 12 TO SEQ ID NO: 14, SEQ ID NO: 27, SEQ ID NO: 28, SEQ ID NO: 37 to SEQ ID NO: 42, SEQ ID NO: 55, SEQ ID NO: 56, SEQ ID NO: 65 to SEQ ID NO: 70 and sequences complementary thereto, or segments thereof.

It is anticipated that the oligonucleotides may constitute all or part of an "array" or "ONA chip" (i.e., an arrangement of different oligonucleotides and/or PNA-oligomers bound to a solid phase). Such an array of different oligonucleotide- and/or PNA- oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics *(*Nature Genetics Supplement, Volume 21, January 1999, and from the literature cited therein). Fluorescently labelled probes are often used for the scanning of immobilized ONA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is alto anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (i.e., each analyte) can be uniquely bound by a unique label and thus detected (each label is directly counted, resulting in a digital readout of each molecular species in the mixture).

It is particularly preferred that the oligomers are utilised for at least one of: detection of; diagnosis of; treatment of; monitoring of; and treatment and monitoring of colorectal cell proliferative disorders. This is enabled by use of said sets for the detection of one or more of the following classes of tissues: colorectal carcinoma, colon adenoma, inflammatory colon tissue, grade 2 dysplasia colon adenomas less than 1 cm, grade 3 dysplasia colon adenomas larger than 1 cm, normal colon tissue, non-colon healthy tissue and non-colon cancer tissue.

Particularly preferred are those sets of oligomers according to the Examples. Most preferably, the presence or absence of a cellular proliferative disorder, most preferably colorectal carcinoma is determined. This is achieved by analysis of the methylation status of at least one target sequence comprising at least one CpG position said sequence comprising, or hybridizing under stringent conditions to at least 16 contiguous nucleotides of a sequence according toSEQ ID NO: 7 and complements thereof Preferably, this is achieved by analysis of the methylation status of a target sequence comprising at least one CpG position said sequence comprising, or hybridizing under stringent conditions to at least 16 contiguous nucleotides of a sequence selected from the group consisting SEQ ID NO: 12 TO SEQ ID NO: 14 and at least one further target sequence comprising at least one CpG position said sequence comprising, or hybridizing under stringent conditions to at least 16 contiguous nucleotides of a sequence according to SEQ ID NO: 7) and complements thereof. Further disclosed is a method for ascertaining genetic and/or epigenetic parameters of the genomic sequence according to SEQ ID NO: 7 within a subject by analyzing cytosine methylation and single nucleotide polymorphisms. Said method comprising contacting a nucleic acid comprising SEQ ID NO: 7in a biological sample obtained from said subject with at least one reagent or a series of reagents, wherein said reagent or series of reagents, distinguishes between methylated and non-methylated CpG dinucleotides within the target nucleic acid.

Said method may comprise the following steps: In the *first step,* a sample of the tissue to be analysed is obtained. The source may be any suitable source, such as cell lines, histological slides, biopsies, paraffin-embedded tissue, body fluids, stool, colonic effluent, urine, blood plasma, blood serum, whole blood, isolated blood cells, cells isolated from the blood and all possible combinations thereof. It is preferred that said sources of DNA are stool or body fluids selected from the group consisting colonic effluent, urine, blood plasma, blood serum, whole blood, isolated blood cells, cells isolated from the blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g.ultrafiltration , silica surfaces or membranes, magnetic particles, polystyrol particles, polystyrol surfaces, positively charged surfaces, and positively charged membranse, charged membranes, charged surfaces, charged switch membranes, charged switched surfaces.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pre-treatment' or 'treatment' herein.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. WO 2005/038051). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. Preferably, the denaturing solvents are used in concentrations between 1%and 35% (v/v). It is also preferred that the bisulfite reaction is carried out in the presence of scavengers such as but not limited to chromane derivatives, e.g., 6- hydroxy-2, 5,7,8, -tetramethylchromane 2-carboxylic acid or trihydroxybenzoe acid and derivates thereof, e.g. Gallic acid (see: WO 2005/038051). The bisulfite conversion is preferably carried out at a reaction temperature between 30°C and 70°C, whereby the temperature is increased to over 85°C for short periods of times during the reaction (see: WO 2005/038051). The bisulfite treated DNA is preferably purified priori to the quantification. This may be conducted by any means known in the art, such as but not limited to Ultrafiltration, preferably carried out by means of Microcon"(TM) columns (manufactured by Millipore"(TM)). The purification is carried out according to a modified manufacturer's protocol (see: WO 2005/038051). In the *third step* of the method, fragments of the treated DNA are amplified, using sets of primer oligonucleotides and an amplification enzyme. The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridize under stringent or highly stringent conditions to an at least 16-base-pair lang segment of the base sequences of one of SEQ ID NO: 27 to SEQ ID NO: 28 and SEQ ID NO: 55 to SEQ ID NO: 56 and sequences complementary thereto. It is preferred that the set of primer oligonucleotides further comprises at least two oligonucleotides whose sequences are each reverse complementary, identical, or hybridize under stringent or highly stringent conditions to an at least 16-base-pair long segment of the base sequences of one of SEQ ID NO: 37 to SEQ iD NO: 42 and SEQ ID NO: 65 to SEQ ID NO: 70 and sequences complementary thereto.

Alternatively, the methylation status of pre-selected CpG positions within at least one nucleic acid sequence according to SEQ ID NO: 7 and preferably additionallyof SEQ ID NO: 12 TO SEQ iD NO: 14 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NO: 27 to SEQ ID NO: 28 and SEQ ID NO: 55 to SEQ ID NO: 56 and sequences complementary thereto, wherein the base sequence of said oligomers comprises at least one CpG dinucleotide. Preferably, the method comprises the use of blockeroligonucleotides (the HeavyMethyl™ assay). The use of such blocker oligonucleotides has been described by Yu et al., BioTechniques 23:714-720, 1997. Blocking probe oligonucleotides are hybridised to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-0-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NO: 27 TO SEQ ID NO: 28 and SEQ ID NO: 55 and SEQ ID NO: 56 and sequences complementary thereto, wherein the base sequence of said oligonucleotides comprises at least one CpG, TpG or CpA dinucleotide. Also preferred is a blocking oligonucleotides having a length of at least 9 nucleotides which hybridizes to a treated nucleic acid sequence according to one of SEQ ID NO: 37 TO SEQ ID NO: 42 and SEQ ID NO: 65 TO SEQ ID NO: 70 and sequences complementary thereto, wherein the base sequence of said oligonucleotides comprises at least one CpG, TpG or CpA dinucleotide

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labelled amplificates have a single positive or negative net charge, allowing for better delectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

in embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of based-based methods such as, but not limited to, array technology and probe based technologies as well as by means of techniques such as sequencing and template directed extension.

In one embodiment of the method, the amplificates synthesized in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide. The hybridizing portion of the hybridizing nucleic acids is typically at least 9, 15, 20, 25, 30 or 35 nucleotides in length. However, longer molecules have utility.

Preferably, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence according to SEQ ID NO: 7 to the equivalent positions within SEQ ID NO: 27 to SEQ ID NO: 28 and SEQ ID NO: 55 toSEQ ID NO: 56 Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

The genomic methylation status of the CpG positions may be ascertained by means of oligonucleotide probes (as detailed above) that are hybridised to the bisulfite treated DNA concurrently with the PCR amplification primers (wherein said primers may either be methylation specific or standard).

A particularly preferred embodiment of this method is the use of fluorescence- based Real Time Quantitative PCR (Heid et al., Genome Res. 6:986-994, 1996; *also see* United States Patent No. 6,331,393) employing a dual-labelled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridize to a CpG-rich sequence located between the torward and reverse amplification primers. The TaqMan™ probe further comprises a fluorescent "reporter moiety" and a "quencher moiety" covalently bound to linker moieties *(e.g.,* phosphoramidites) attached to the nucleotides of the TaqManTM oligonucleotide. For analysis of methylation within nucleic acids subsequent to bisulfite treatment, it is required that the probe be methylation specific, as described in United States Patent No. 6,331,393, also known as the MethylightTM™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the *fourth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sei USA 74:5463-5467, 1977).

### Best mode:

In the most preferred disclosure the genomic nucleic acids are isolated and treated according to the first three steps of the method outlined above, namely:
a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides,* and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO: 27 and SEQ ID NO: 28 and SEQ ID NO: 55 TO SEQ ID NO: 56 and sequences complementary thereto, wherein the base sequence of said oligomers comprise at least one CpG dinucleotide. It is particularly preferred that the subsequent amplification of d) is additionally carried out by means of methylation specific primers each comprising a sequence having a length of at least 9 nucleotides which hybridizes to a treated nucleic acid sequence according to one of SEQ ID NO: 37 TO SEQ ID NO: 42 AND SEQ ID NO: 65 to SEQ ID NO: 70 (and more preferably one of the subgroup thereof consisting of SEQ ID NOS: 27, 28, 55, 56) and sequences complementary thereto, wherein the base sequence of said oligomers comprise at least one CpG dinucleotide.

Step e) of the method, namely the detection of the specific amplificates indicative of the methylation status of one or more CpG positions of a sequence according to SEQ ID NO: 7 and preferably also SEQ ID NO: 12 to SEQ ID NO: 14 is carried out by means of *real-time* detection methods as described above.

Disclosed is a method for the analysis of the methylation status of genomic DNA (SEQ ID NO: 7, SEQ ID NO: 12 to SEQ ID NO: 14 and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH.

In the *first step* of such additional methods, the genomic DNA sample is isolated from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, *e.g.,* by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic or potentially neoplastic matter are suitable for us e in the present method, preferred are cell lines, histological slides, biopsies, paraffin- embedded tissue, body fluids, stool, colanie effluent, urine, blood plasma, blood serum, whole blood, isolated blood cells, cells isolated from the blood and combinations thereof. Body fluids are the preferred source of the DNA; particularly preferred is blood plasma, blood serum, whole blood, isolated blood cells and cells isolated from the blood. Once the nucleic acids have been extracted, the genomic double-stranded DNA is used in the analysis. The DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting of Msel, Bfal, Csp61, Tru11, Tvu11, Tru91, Tvu91, Mael and Xspl. Particularly preferred is the use of two or three such enzymes. Particularly preferred is the use of a combination of Msel, Bfal and Csp61. The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length. In the *third step,* the DNA (or fragments thereof) is then digested with one or more methylation sensitive restriction enzymes. The digestion is carried out such that hydrolysis of the DNA at the restriction site is informative of the methylation status of a specific CpG dinucleotide of the gene RASSF2lt is particularly preferred that the digestion is carried out such that hydrolysis of the DNA at the restriction site is informative of the methylation status of at least one CpG dinucleotide of the gene SEPTIN 9 and furthermore of at least one CpG dinucleotide of thegene RASSF2

Preferably, the methylation-specific restriction enzyme is selected from the group consisting of *Bsi Et, Hga I HinPI*, *Hpy991, Ava I, Bce Al, Bsa Hf, Bis*/*, BstUI, Bsh*/*2361, Acel*/*, BstFNI, McrBC, Glal, MvnI, Hpa*/*1* (*Hap*/*1*), *Hhal, Acil, Smal, HinP11, HpyCH4*/*V, Eagl* and mixtures of two or more of the above enzymes. Preferred is a mixture containing the restriction enzymes BstUI, HpaII, HpyCH41V and HinP11.

In the *fourth step,* which is optional but preferred the restriction fragments are amplified. This is preferably carried out using a polymerase chain reaction, and said amplificates may carry suitable detectable labeis as discussed above, namely fluorophore labeis, radionuclides and mass labels.

Particularly preferred is amplification by means of an amplification enzyme, at least two primers comprising, in each case a contiguous sequence at least16 nucleotides in length that is complementary to, or hybridizes under moderately stringent or stringent conditions to a sequence according to SEQ ID NO: 7 and complements thereof.

Further preferred is amplification by means of an amplification enzyme and at least two primers comprising, in each case a contiguous sequence at least 16 nucleotides in length that is complementary to, or hybridizes under moderately stringent or stringent conditions to a sequence according to SEQ ID NO: 7 and complements thereof and at least two primers comprising, in each case a contiguous sequence at least 16 nucleotides in length that is complementary to, or hybridizes under moderately stringent or stringent conditions to a sequence selected from the group consisting SEQ ID NO: 12 TO SEQ ID NO: 14, and complements thereof. Preferably said contiguous sequence is at least 16, 20 or 25 nucleotides in length. Alternatively, said primers may be complementary to any adaptors linked to the fragments. In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis. Preferably said detection is carried out by hybridization to at least one nucleic acid or peptide nucleic acid comprising in each case a contiguous sequence at least 16 nucleotides in length that is complementary to, or hybridizes under moderately stringent or stringent conditions to a according to SEQ ID NO: 7 and complements thereof. It is further preferred that said detection is carried out by hybridization to at least one nucleic acid or peptide nucleic acid comprising in each case a contiguous sequence at least 16 nucleotides in length that is complementary to, or hybridizes under moderately stringent or stringent conditions to a sequence according to SEQ ID NO: 7 and complements thereof and additionally at least one nucleic acid or peptide nucleic acid comprising in each case a contiguous sequence at least 16 nucleotides in length that is complementary to, or hybridizes under moderately stringent or stringent conditions to a sequence selected from the group consisting SEQ ID NO: 12 to SEQ ID NO: 14. Preferably said contiguous sequence is at least 16, 20 or 25 nucleotides in length. Subsequent to the determination of the methylation state or level of the genomic nucleic acids the presence or absence of cellular proliferative disorder (most preferably colorectal carcinoma) is deduced based upon the methylation state or level of at least one CpG dinucleotide sequence of at least one sequence according to SEQ ID NO: 7 or an average, or a value reflecting an average methylation state of a plurality of CpG dinucleotide sequences of sequence according to SEQ ID NO: 7 wherein methylation is associated with the presence of colorectal carcinoma. It is further preferred that the determination of the methylation state or level of the genomic nucleic acids the presence or absence of cellular proliferative disorder (most preferably colorectal carcinoma) is deduced based upon the methylation state or level of at least one CpG dinucleotide sequence of the gene Septin 9 and furthermore of at least one CpG dinucleotide sequence of at least one sequence selected from the group consisting SEQ ID NO: 12 TO SEQ ID NO: 14, or an average, or a value reflecting an average methylation state of a plurality of CpG dinucleotide sequences of a sequence according to SEQ ID NO: 7wherein methylation of any of the analyzed CpG positions is associated with the presence of colorectal carcinoma.

Wherein said methylation is determined by quantitative means the cut-off point for determining said the presence of methylation is preferably zero (i.e. wherein a sample displays any degree of methylation it is determined as having a methylated status at the analyzed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected from the group consisting of 0%-5%, 5%-10%, 10%-15%, 15%-20%, 20%-30% and 30%-50%. Particularly preferred are the cut-offs 10%, 15%, 25%, and 30%.

Alternatively disclosed are genes comprising the Septin 9 or its truncated transcript Q9HC74 and the gene RASSF2subsequent to the determination of the methylation state of the genomic nucleic acids the presence or absence of cellular proliferative disorders, in particular colorectal cell proliferative disorder is deduced based upon the methylation state of at least one CpG dinucleotide sequence of SEQ ID NO: 12 to SEQ ID NO: 14 and at least one CpG dinucleotide sequence of SEQ ID NO: 7 or an average, or a value reflecting an average methylation state of a plurality of CpG dinucleotide sequences thereof wherein hypermethylation is associated with cancers, in particular colorectal cancer.

### Diagnostic and Prognostic Assays for cellular proliferative disorders

The present disclosure enables diagnosis of events which are disadvantageous to patients or individuals in which important genetic and/or epigenetic parameters within the gene RASSF2 may be used as a marker. Said parameters obtained by means of the present invention may be compared to another set of genetic and/or epigenetic parameters, the differences serving as the basis for a diagnosis and/or prognosis of events which are disadvantageous to patients or individuals.

More specifically the present disclosure enables the screening of at-risk populations for the early detection of cancers, most preferably colorectal carcinomas. Neoplastic cellular proliferative disorders, most particularly carcinomas, present decreased methylation (i.e. decreased expression) within the gene RASSF2as opposed to benign disorders and normal tissues which do not.

Specifically, disclosed are diagnostic cancer assays based on measurement of differential expression (preferably methylation) of one or more CpG dinucleotide sequences of a sequence according to SEQ ID NO: 7 that comprise such a CpG dinucleotide sequence. Typically, such assays involve obtaining a sample from a subject, performing an assay to measure the expression of the gene RASSF2, preferably by determining the methylation status of a the sequence according to SEQ ID NO: 7derived from the sample, relative to a control sample, or a known standard and making a diagnosis based thereon. Preferably said gene is RASSF2.

Particularly preferred is the following combination of genes:
-Septin 9+RASSF2Oligomers may be used to assess the CpG dinucleotide methylation status, such as those based on SEQ ID NO: 27 to SEQ ID NO: 28 and SEQ ID NO: 55 to SEQ ID NO 56, or arrays thereof, as well as in kits based thereon and useful for the diagnosis of cellular proliferative disorders, most preferably colorectal carcinoma.

### Kits

Moreover, an additional aspect of the present disclosure relates to a kit comprising: a means for determining methylation of the gene RASSF2.

The means for determining methylation comprise preferably a bisulfite-containing reagent; one or a plurality of oligonucleotides consisting whose sequences in each case are identical, are complementary, or hybridize under stringent or highly stringent conditions to an at least 9 or more preferably 18 base long segment of a sequence selected from SEQ ID NO: 27 to SEQ ID NO: 28 and SEQ ID NO: 55 to SEQ ID NO: 56 and optionally instructions for carrying out and evaluating the described method of methylation analysis. The base sequence of said oligonucleotides comprises at least one CpG, CpA or TpG dinucleotide kit may further comprise one or a plurality of oligonucleotides consisting whose sequences.

in each case are identical, are complementary, or hybridize under stringent or highly stringent conditions to an at least 9 or more preferably 18 base long segment of a sequence selected from SEQ ID NO: 27 to SEQ ID NO: 28 and SEQ ID NO: 55 to SEQ ID NO: 56 (

Said kit may further comprise standard reagents for performing a CpG position-specific methylation analysis, wherein said analysis comprises one or more of the following techniques: MS-SNuPE, MSP, Methylight™, HeavyMethyl, COBRA, and nucleic acid sequencing. However, a kit can also contain only part of the aforementioned components. The kit may comprise additional bisulfite conversion reagents selected from the group consisting: DNA denaturation buffer; sulfonation buffer; DNA recovery reagents or kits (e.g., precipitation, Ultrafiltration, affinity column); desulfonation buffer; and DNA recovery components. Alternatively, the kit may contain, packaged in separate containers, a polymerase and a reaction buffer optimized for primer extension mediated by the polymerase, such as PCR. In another aspect of the disclosure the kit further comprising means for obtaining a biological sample of the patient. Preferred is a kit, which further comprises a container suitable for containing the means for determining methylation of the gene RASSF2 in the biological sample of the patient, and most preferably further comprises instructions for use and interpretation of the kit results.

Preferably, the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridize under stringent or highly stringent conditions to an at least 9 or more preferably 18 base long segment of a sequence selected from SEQ ID NO: 27 to SEQ ID NO: 28 AND SEQ ID NO: 55 to SEQ ID NO: 56 and optionally (d) instructions for use and interpretation of the kit results. In an alternative preferred embodiment the kit comprises: (a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridizes to a pre-treated nucleic acid sequence according to one of SEQ ID NO: 27 to SEQ ID NO: 28 and SEQ ID NO: 55 to SEQ ID NO: 56 and sequences complementary thereto; and optionally (d) instructions for use and interpretation of the kit results.

Alternatively, the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridize under stringent or highly stringent conditions to an at least 9 or more preferably 18 base long segment of a sequence selected from SEQ ID NO: 27 to SEQ ID NO: 28 and SEQ ID NO: 55to SEQ ID NO: 56 (d) at least one oligonucleotides and/or PNA-oligomer having a length of at least 9 or 16 nucleotides which is identical to or hybridizes to a pre-treated nucleic acid sequence according to one of SEQ ID NO: 27 to SEQ ID NO: 28 AND SEQ ID NO: 55 to SEQ ID NO: 56 and sequences complementary thereto; and optionally (e) instructions for use and interpretation of the kit results.

Said kits may further comprise at least one set of primer oligonucleotides containing two oligonucleotides whose sequences in each case are identical, are complementary, or hybridize under stringent or highly stringent conditions to a 9 or more preferably 18 base long segment of a sequence selected from SEQ ID NO: 37 to SEQ ID NO: 42 and SEQ ID NO: 65 to SEQ ID NO: 70.

The kit may also contain other components such as buffers or solutions suitable for blocking, washing or coating, packaged in a separate container. Typical reagents *(e.g.,* as might be found in a typical COBRA™-based kit) for COBRATM analysis may include, but are not limited to: PCR primers for the gene RASSF2 restriction enzyme and appropriate buffer; gene-hybridization oligo; control hybridization oligo; kinase labeling kit for oligo probe; and labeled nucleotides. Typical reagents (e.g., as might be found in a typical Methylight TM -based kit) for Methylight™ analysis may include, but are not limited to: PCR primers for the bisulfite converted sequence of the RASSF2; bisulfite specific probes (e.g. TaqMan TM or Lightcycler ™I; optimized PCR buffers and deoxynucleotides; and Taq polymerase.

Typical reagents (e.g., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for the bisulfite converted sequence of the gene RASSF2; reaction buffer (for the Ms- SNuPE reaction); and labelled nucleotides.

Typical reagents (e.g., as might be found in a typical MSP-based kit) for MSP analysis may include, but are not limited to: methylated and unmethylated PCR primers for the bisulfite converted sequence of or genomic sequence of RASSF2, optimized PCR buffers and deoxynucleotides, and specific probes.

Moreover, an additional aspect of the present disclosure is an alternative kit comprising a means for determining methylation of the gene RASSF2wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence according to SEQ ID NO: 7 and optionally instructions for carrying out and evaluating the described method of methylation analysis. In one embodiment the base sequence of said oligonucleotides are identical, are complementary, or hybridize under stringent or highly stringent conditions to an at least18 base long segment of a sequence according to SEQ ID NO: 7 Preferably said kit further comprises one or a plurality of primer oligonucleotides (preferably one or a plurality of primer pairs) suitable for the amplification of a sequence comprising at least one CpG dinucleotide of a sequence selected from SEQ ID NO: 12 to SEQ ID NO: 14.

Further disclosed is that said kit may comprise one or a plurality of oligonucleotide probes for the analysis of the digest fragments, preferably said oligonucleotides are identical, are complementary, or hybridize under stringent or highly stringent conditions to an at least 16 base long segment of a sequence according to SEQ ID NO: 7 Further, said kit may further comprise one or a plurality of oligonucleotide probes for the analysis of the digest fragments, preferably said oligonucleotides are identical, are complementary, or hybridize under stringent or highly stringent conditions to an at least 16 base long segment of a sequence selected from SEQ ID NO: 12 TO SEQ ID NO: 14.

In a preferred disclosure the kit may comprise additional reagents selected from the group consisting: buffer (e.g. restriction enzyme, PCR, storage or washing buffers); DNA recovery reagents or kits (e.g., precipitation, Ultrafiltration, affinity column) and DNA recovery components.

Alternatively, the kit may contain, packaged in separate containers, a polymerase and a reaction buffer optimized for primer extension mediated by the polymerase, such as PCR. In another embodiment of the invention the kit further comprising means for obtaining a biological sample of the patient. In a preferred embodiment the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to an at least 9 base long segment of a sequence according to SEQ ID NO: 7 and optionally (c) instructions for use and interpretation of the kit results.

Said kit may further comprise (d) at least one set of oligonucleotides one or a plurality of nucleic acids or peptide nucleic acids which are identical, are complementary, or hybridize under stringent or highly stringent conditions to an at least 9 base long segment of a sequence selected from SEQ ID NO: 12 to SEQ ID NO: 14.

Alternatively, the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence according to SEQ ID NO: 7 and optionally (d) instructions for use and interpretation of the kit results. Disclosed is that said kit further comprises (e) at least one set of primer oligonucleotides suitable for the amplification of a sequence comprising at least one CpG dinucleotide of a sequence selected from SEQ ID NO: 12 to SEQ ID NO: 14.

Alternatively, the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence according to SEQ ID NO: 7); (d) at least one set of oligonucleotides one or a plurality of nucleic acids or peptide nucleic acids which are identical, are complementary, or hybridize under stringent or highly stringent conditions to an at least 9base long segment of a sequence according to SEQ ID NO: 7 and optionally (e) instructions for use and interpretation of the kit results. In one embodiment said kit further comprises (f) at least one set of primer oligonucleotides suitable for the amplification of a sequence comprising at least one CpG dinucleotide of a sequence selected from SEQ ID NO: 12 to SEQ ID NO: 14. The kit may also contain other components such as buffers or solutions suitable for blocking, washing or coating, packaged in a separate container.

The disclosure further relates to a kit for use in providing a diagnosis of the presence of a cell proliferative disorder in a subject by means of methylation-sensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for the gene RASSF2 and comprises a sequence of at least 15 base pairs in length but no more than 200 bp of a sequence according to SEQ ID NO: 7Preferably said sequence is at least 15 base pairs in length but no more than 80 bp of a sequence according to SEQ ID NO: 7 It is further preferred that said sequence is at least 20 base pairs in length but no more than 30 bp of a sequence according to SEQ ID NO: 7

Said kit may further comprise a sequence of at least 15 base pairs in length but no more than 200 bp of a sequence according to one of SEQ ID NO: 12 to SEQ ID NO: 14. Preferably said sequence is at least 15 base pairs in length but no more than 80 bp of a sequence according to one of SEQ ID NO: 12 to SEQ ID NO: 14. It is further preferred that said sequence is at least 20 base pairs in length but no more than 30 bp of a sequence according to one of SEQ ID NO: 12 to SEQ ID NO: 14.

Said test kit preferably further comprises a restriction enzyme component comprising one or a plurality of methylation-sensitive restriction enzymes. Further disclosed is that said test kit is further characterized in that it comprises at least one methylation-specific restriction enzyme, and wherein the oligonucleotides comprise a restriction site of said at least one methylation specific restriction enzymes.

The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step.

Disclosed is further a composition of matter useful for the detection of colon cell proliferative disorders. Said composition comprising at least one nucleic acid 18 base pairs in length of a segment of the nucleic acid sequence disclosed in SEQ ID NO: 27 to SEQ ID NO: 28 and SEQ ID NO: 55 to SEQ ID NO:56 , and one or more substances taken from the group comprising : 1-5 mM Magnesium Chloride, 100-500 11 M dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 27 to SEQ ID NO: 28 and SEQ ID NO: 55to SEQ ID NO: 56 (and more preferably according to SEQ ID NO: 7 and sequences complementary thereto. In one aspect of the disclosure said composition further comprises an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 37 TO SEQ ID NO: 42 and SEQ ID NO: 65 TO SEQ ID NO: 70 and sequences complementary thereto. It is preferred that said composition of matter comprises a buffer solution appropriate for the stabilization of said nucleic acid in an aqueous solution and enabling polymerase based reactions within said solution. Suitable buffers are known in the art and commercially available.

Preferably, said at least one oligomer is at least 50, 100, 150, 200, 250 or 500 base pairs in length of a segment of the nucleic acid sequence disclosed in SEQ ID NO: 27 to SEQ ID NO: 28 AND SEQ ID NO: 55 to SEQ ID NO: 56, or SEQ ID NO: 37 to SEQ ID NO: 42 AND SEQ ID NO: 65 to SEQ ID NO: 70.

While the present invention has been described with specificity in accordance with certain of its preferred embodiments, the following examples serve only to illustrate the invention and are not intended to limit the invention.

### EXAMPLES

### Example 1

In the following example a variety of assays suitable for the methylation analysis of the genes and genomic sequences according to tables 1 and 2 were designed, in order to validate the suitable of said markers for the detection of colorectal carcinoma. Furthermore the performance of gene panels (combinations of a plurality of markers) was assessed, of particular interest were panels comprising the gene Septin 9 that provided improved accuracy over the use of Septin 9 alone.

The assays were designed to be run on the LightCycler platform (Roche Diagnostics), but other such instruments commonly used in the art are also suitable. The assays were MSP assays. MSP amplificates were designed to be detected by means of Taqman style fluorescent labelled detection probes.

### Samples

In total 314 samples were analysed:
198 colorectal carcinoma of the following stages:
   - Stage 0: 4 samples
   - Stage 1: 19 samples
   - Stage 2: 84 samples
   - Stage 3: 57 samples
   - Stage 4: 20 samples
   - Stage unknown: 14 samples
22 normal or normal adjacent tissue
26 whole blood samples
40 other cancers (liver, breast & prostate)
28 other normal or normal adjacent tissues (liver, breast & prostate)

### DNA extraction and bisulfite treatment

The DNA was isolated from the all samples according to a modified protocol based on that disclosed in the Qiagen Genomic DNA Handbook (August 2001) (pg 28-31, 44-47). The eluate resulting from the purification was then converted according to the following bisulfite reaction.

The eluate was mixed with 354 µl of bisulfite solution (5.89 mol/1) and 146 111 of dioxane containing a radicl scavenger (6-hydroxy-2,5,7,8-tetramethylchromane 2-carboxylic acid, 98.6 mg in 2.5 ml of dioxane). The reaction mixture was denatured for 3 min at 99 o C and subsequently incubated at the following temperature program for a total of 7 h min 50°C; one thermospike (99.9°C) for 3 min; 1.5 h 50°C; one thermospike (99°C) for 3 min; 3 h 50 o C. The reaction mixture was subsequently purified by Ultrafiltration using a Millipore Microcon TM column. The purification was conducted essentially according to the manufacturer's instructions. For this purpose, the reaction mixture was mixed with 300 111 of water, loaded onto the Ultrafiltration membrane, centrifuged for 15 min and subsequently washed with 1 x TE buffer. The DNA remains on the membrane in this treatment. Then desulfonation is performed. For this purpose, 0.2 mol/1 NaOH was added and incubated for 10 min. A centrifugation (10 min) was then conducted, followed by a washing step with 1 x TE buffer. After this, the DNA was eluted. For this purpose, the membrane was mixed for 10 minutes with 75 111 of warm 1 x TE buffer (50° C). The membrane was turned over according to the manufacturer's instructions. Subsequently a repeated centrifugation was conducted, with which the DNA was removed from the membrane. 10 µl of the eluate was utilized for the Lightcycler Real Time PCR assay.

PCR assay component sequences and thermal cycling conditions are provided in Table 3.

### Control assay

### Control assay

The GSTP1-C3 assay design makes it suitable for quantitating DNAs from different sources, including fresh/frozen samples, remote samples such as plasma or serum, and DNA obtained from
archival specimen such as paraffin embedded material. The following oligonucleotides were used in the reaction to amplify the control amplificate:Control Primer1: GGAGTGGAGGAAATTGAGAT
Control Primer2: CCACACAACAAATACTCAAAAC
Control Probe: FAM-TGGGTGTTTGTAATTTTTGTTTTGTGTTAGGTT-TAMRA
Cycle program (40 cycles): 95°C,10 min
95 °C, 15 sec
58 °C, 1 min

### Data interpretation

### Calculation of DNA concentration.

The Cp (crossing point values) as calculated by the Lightcycler instrument software were used to determine DNA concentration. The DNA concentration was calculated by reference of the CP value of each well to a standard curve for both the methylation assays and the C3 assay.

### Percentage methylation

For each sample the detected percentage methylation was calculated as the measured concentration of DNA quantified using the methylation assays over the concentration of DNA in the sample as quantified by the C3 assay.

Detection of methylation was determined at multiple different threshold levels, see tables) as well as at all methylation levels (i.e. any samples wherein methylation was detected were deemed positive).

The sensitivity of each assay was determined from the colorectal carcinoma sample positive detection rate, wherein sensitivity was determined as the % samples wherein methylation was positively detected (i.e. true positives).

The specificity of each assay was determined from the whole blood sample negative detection rate (i.e. true negative detection rate) wherein false positives were discounted from the total number of analysed samples.

### Results

The AUC of each analyzed marker both alone or in some cases in combination with other markers is provided in Tables 4 to 6 (with the exception of MRPS21 & DOCK10). The term 'AUC' is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

Table 4 provides an overview of the measured AUC of each marker in the detection of colorectal carcinoma at all methylation levels, provides information on their complementarity to Septin 9, and also their methylation AUC in whole blood. It is necessary to determine the methylation of a marker in whole blood, as the preferred sample type for a screening assay would be blood, accordingly markers that are methylated in both blood and cancer are not preferred.

Table 5 provides an overview of the measured AUC of each marker in the detection of colorectal carcinoma at methylation cut off values of 10%, 20% and 30%.

Table 6 provides an overview of the measured AUC of panels of markers comprising the gene Septin 9 and 2 further markers in the detection of colorectal carcinoma at methylation cut off values of 0%, 10%, 20% and 30%. The panel data was compiled by determining the proportion or number of the analysed samples with methylation measured within a given threshold using at least one assay of the panel.

For MRPS21 & DOCK10 assay performance is provided in Figures 27 and 28. Sensitivity of the DOCK10 assay was 0.48 as measured at a specificity of 0.97 (cut off was -3). Sensitivity of the MRPS21 assay was 0.63 as measured at a specificity of 0.96 (cut off was -1.422).

### Example 2

In the following investigation, the performace of selected markers from example 1, according to Table 7 were selected for further analysis by means of the HM (Heavymethy) assay. Target regions of each gene were bisulfite converted and amplified by means of non-MSP primers, in the presence of a blocker oligonucleotides designed to suppress amplificates that had not been methlated prior to bisulfite treatment. Amplificates were then detected by means of Lightcycler (dual) probes.

Plasma samples from the following patient classes were analysed:
- Colorectal carcinoma (131 total)
   Stage 0 = 1
   Stage I = 13
   Stage II = 32
   Stage III = 27
   Stage IV = 8
   Unclassified = 50
- Healthy colorectal (colonoscopy verified) = 169
- Non- cancerous diseases (NCD) = 29
- Cancers of non-colorectal origin (NCC) = 31

In total 360 samples were analysed.

### DNA extraction and bisulfite treatment

The DNA was isolated from the all samples by means of the Magna Pure method (Roche) according to the manufacturer's instructions. The eluate resulting from the purification was then converted according to the following bisulfite reaction.

The eluate was mixed with 354 µl of bisulfite solution (5.89 mol/l) and 146 µl of dioxane containing a radical scavenger(6-hydroxy-2,5,7,8-tetramethylchromane 2-carboxylic acid, 98.6 mg in 2.5 ml of dioxane). The reaction mixture was denatured for 3 min at 99°C and subsequently incubated at the following temperature program for atotal of 7 h min 50°C; one thermospike (99.9°C) for 3 min; 1.5 h 50°C; one thermospike (99°C) for 3 min; 3 h 50°C. The reaction mixture was subsequently purified by ultrafiltration using a Millipore Microcon **™** column. The purification was conducted essentially according to the manufacturer's instructions. For this purpose, the reaction mixture was mixed with 300 µl of water, loaded onto the ultrafiltration membrane, centrifuged for 15 min and subsequently washed with 1 x TE buffer. The DNA remains on the membrane in this treatment. Then desulfonation is performed. For this purpose, 0.2 mol/l NaOH was added and incubated for 10 min. A centrifugation (10 min) was then conducted, followed by a washing step with 1 x TE buffer. After this, the DNA was eluted. For this purpose, the membrane was mixed for 10 minutes with 75 µl of warm 1 x TE buffer (50°C). The membrane was turned over according to the manufacturer's instructions. Subsequently a repeated centrifugation was conducted, with which the DNA was removed from the membrane. 10 µl of the eluate was utilized for the Lightcycler Real Time PCR assay.

### Reaction solutions and thermal cycling conditions

PCR assay component sequences are provided in Table 10. Each assay was performed twice (independently) in each sample.

Thermal cycling conditions were:

| PCDHGC3 | | | |
|---|---|---|---|
| activation: | 95°C | 10min | |
| 50 cycles: | 95°C | 10 sec | (20°C/s) |
| | 56°C | 30 sec | (20°C/s) |
| | 60°C | 3 sec | (20°C/s)detection |
| | 72°C | 10 sec | (20°C/s) |
| | | | |
| melting curve: | 95°C | 10sec | (20°C/s) |
| | 40°C | 10sec | (20°C/s) |
| | 95°C | 0sec | (0,1°C/s)Continuous |
| cooling: | 40°C 5 sec | | |
| | | | |
| All other assays: | | | |
| activation: | 95°C | 10min | |
| 55 cycles: | 95°C | 10sec | (20°C/s) |
| | 56°C | 30 sec | (20°C/s)detection |
| | 72°C | 10 sec | (20°C/s) |
| melting curve: | 95°C | 10 sec | (20°C/s) |
| | 40°C | 10 sec | (20°C/s) |
| | 95°C | 0sec | (0,1°C/s)Continuous |
| cooling: | 40°C | 5 sec | |

### Results:

In order to predict the presence of CRC tumor DNA in the measured plasma samples we use a logistic regression model. The logistic regression model is build as follows.

First the measurement data for each marker assay is encoded in a qualitative way by the following 3 levels:
- Level0 - both replicate PCR reactions showed no amplification
- Level1 - exactly one of the two PCR replicates showed an amplification curve
- Level2 - both of the two PCR replicates showed amplification curves

If any of the two PCR replicates could not be successfully measured the respective marker measurement was regarded as invalid.

The five different DNA methylation markers were used as independent factors with 3 levels in a logistic regression model. An additional intercept factor but no factor interactions were included in the model. The logistic regression model was trained and optimal weights for all factor levels were determined by using the maximum likelihood procedure.

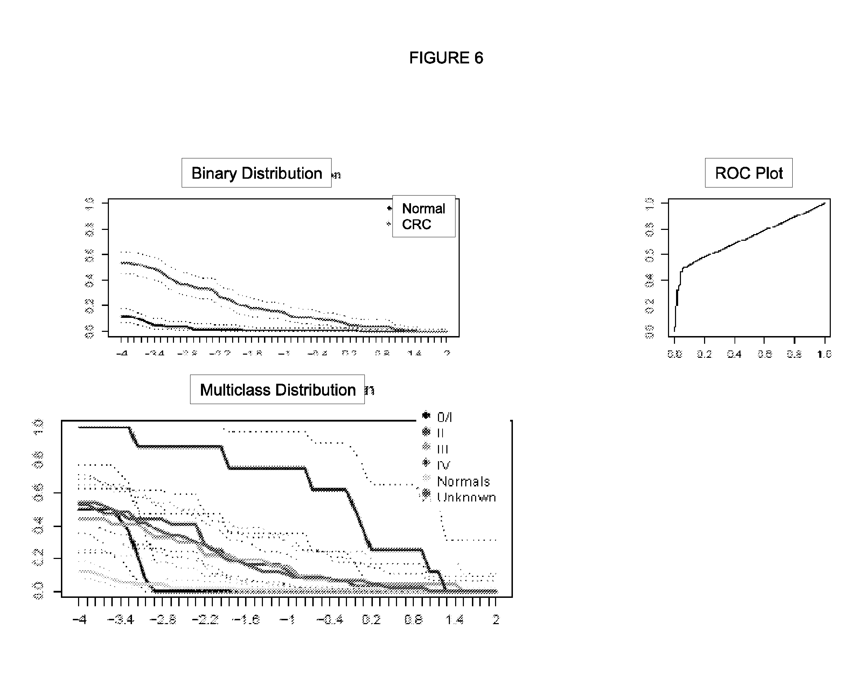

Figures 1 to 10 provide the plotsof the measured log mean methylation of the individual assays. Each figures consists of three plots, the upper and lower left hand side plots provide the binary and multiclass analysis respectively, the right hand plot provides an ROC wherein sensitivity is shown on the Y-axis and 1-specificity is shown on the X-axis. Table 8 and figures 1 to 5 provide an overview of marker performances in all sample groups. Table 9 and figures 6 to 10 provide an overview of marker performances in the colorectal carcinoma and normal colorectal groups.

Figures 12 to 21 provide the plotsof the measured log majority mean (analysed sample is only counted as positive if both replicates are positive, the mean of the two measurements is taken as the quantitative methylation measurement) methylation of the individual assays. Each figures consists of three plots, the upper and lower left hand side plots provide the binary and multiclass analysis respectively, the right hand plot provides an ROC wherein sensitivity is shown on the Y-axis and 1-specificity is shown on the X-axis. Table 12 and figures 12 to 16 provide an overview of marker performances in all sample groups. Table 13 and figures 18 to 21 provide an overview of marker performances in the colorectal carcinoma and normal colorectal groups.

Table 11 provides an overview of the AUC and snd sensitivities of the single assays at 95% specificity (all p-values were less than 0.00001). Wherein said classes are:
All: Normal + NCD +NCC vs. CRC stages I to IV
I-IV: Normal vs. CRC stages I to IV
I-III: Normal vs. CRC stages I to III

From the multiclass distribution of figure 6 (bottom left hand plot) and table 11 it can be determined that the gene RASSF2 is particularly effective at detecting Stage 1 and early colorectal carcinomas. Accordingly expression, most preferably CpG methylation, of said gene is in additioan to being a preferred diagnostic marker is particularly preferred for the screening of general populations (individuals not displaing any indicators or symptoms of colorectal carcinoma) for the early detection of colorectal carcinomas.

### Marker combinations (panels)

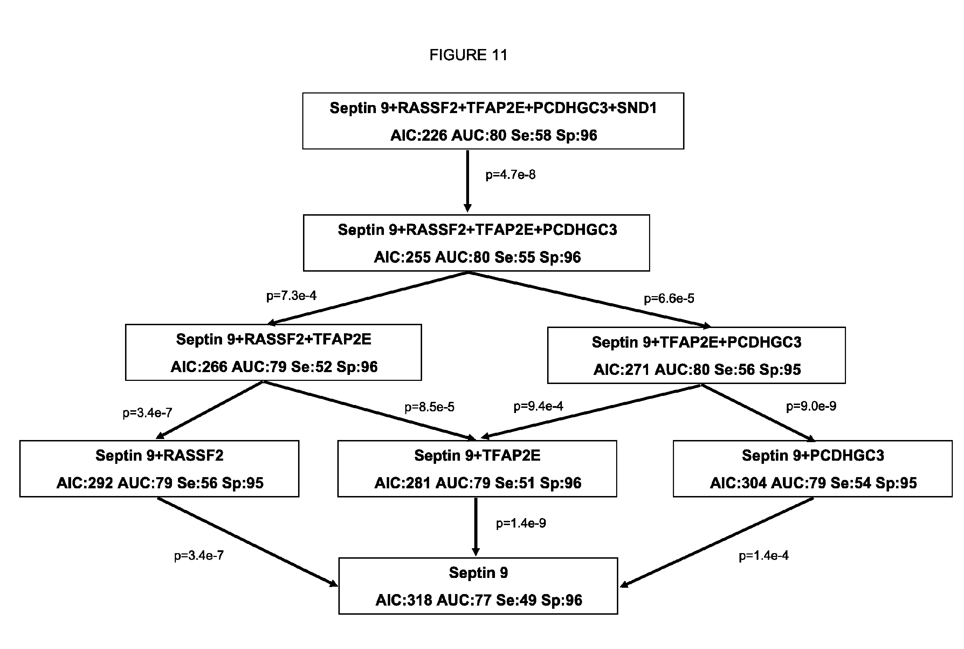

To identify the subset of DNA methylation markers that optimally predicts the presence of CRC we use the backward elimination procedure . In each elimination step the DNA methylation marker with the lowest factor levels was removed from the model. We compared the predictive power of the reduced model with the complete model by using the likelihood ratio test. To identify the subset of DNA methylation markers that optimally predicts the presence of CRC we use the backward elimination procedure . In each elimination step the DNA methylation marker with the lowest factor levels was removed from the model. We compared the predictive power of the reduced model with the complete model by using the likelihood ratio test. Fig. 11 shows that (in the Normal vs. CRC stages I to IV comparison) at each elimination step the predictive power of the logistic regression model was significantly reduced. We conclude that all listed DNA methylation marker models give superior prediction performance as compared to the single marker or the respective simpler marker panels.

We conclude that the following DNA marker models give superior prediction performance as compared to the single marker or the respective simpler marker panels.

Figures 22 to 26 provide an overview of the performance of the following marker combinations:

| Septin 9+TFAP2E+RASSF2+PCDHGC3+SND1 (Figure 22) | | |
|---|---|---|
| All | AUC | 80 |
| | Sens/Spec | 57/95 |
| All CRC | AUC | 80 |
| | Sens/Spec | 58/96 |
| CRC I-III | AUC | 76 |
| | Sens/Spec | 50/96 |
| | | |

| Septin 9+TFAP2E+RASSF2+PCDHGC3 (Figure 23) | | |
|---|---|---|
| All | AUC 80 | |
| | Sens/Spec | 53/95 |
| All CRC | AUC | 80 |
| | Sens/Spec | 55/96 |
| CRC I-III | AUC | 77 |
| | Sens/Spec | 48/96 |
| | | |

| Septin 9+TFAP2E+RASSF2 (Figure 24) | | |
|---|---|---|
| All | AUC 77 | |
| | Sens/Spec | 48/96 |
| All CRC | AUC | 79 |
| | Sens/Spec | 52/96 |
| CRC I-III | AUC | 75 |
| | Sens/Spec | 42/96 |
| | | |

| Septin 9+TFAP2E (Figure 25) | | |
|---|---|---|
| All | AUC 77 | |
| | Sens/Spec | 45/96 |
| All CRC | AUC | 79 |
| | Sens/Spec | 51/96 |
| CRC I-III | AUC | 75 |
| | Sens/Spec | 41/96 |
| | | |

| Septin 9+RASSF2 (Figure 26) | | |
|---|---|---|
| All | AUC 77 | |
| | Sens/Spec | 43/96 |
| All CRC | AUC | 79 |
| | Sens/Spec | 56/95 |
| CRC I-III | AUC | 74 |
| | Sens/Spec | 46/95 |

In each case the upper plot shows all samples (Normals, Non Colorectal Disease, Non Coloretal Cancers and all CRC stages), the lower plot shows only Normal and CRC samples.

**Table 1: Sequences used in the examples**

| Genomic SEQ ID NO: | Gene | Methylated bisulfite converted sequence I(sense) | Methylated bisulfite converted sequence l(antisense) | Unmethylated bisulfite converted sequence 'sense) | Unmethylated bisulfite converted sequence Ifantisense) |
|---|---|---|---|---|---|
| 7 | RASSF2 | 27 | 28 | 55 | 56 |
| 12 | Septin 9 | 37 | 38 | 65 | 66 |
| 13 | Septin 9 | 39 | 40 | 67 | 68 |
| 14 | Septin 9 | 41 | 42 | 69 | 70 |

**Table 2: Genes used in the examples**

| Genomic SEQ ID NO: | Gene | Abbreviation | Chromo somal location * | Contig location * |
|---|---|---|---|---|
| 7 | Ras association domain family 2 | RASSF2 | 20 | AL 133354.14.1.5 |
| | | | 4750982 to 4752901 (+) | 9726 |
| | | | | 44336 to 46255 |
| | | | | (+) |
| 12 | Septin-9 (MLL septin-like fusion protein) (MLL septin-like fusion protein | Septin 9 | 17 | AC068594.15.1.1 |
| | | | 7278908 | 68501 |
| | | | 2 to 730082 | 150580 to 151086 (+) to AC111170.11.1.1 |
| | | | 58 | |
| | | | I(+) ** | 58988 |
| | | | | 137268 to 138151 (+)** |
| 13 | Septin 9 CpG island | Septin 9 CpG island | 17 | |
| | | | 7278908 | AC068594.15.1.1 |
| | | | 2 to | 68501 |
| 14 | Septin 9 CpG | Septin 9 CpG island | | |

**Table 3: Assay components according to Example 1**

| **Genename** | **Forward primer SEQID NO:** | **Reverse primer SEQID NO:** | **Probe SEQ ID NO:** | **PCR conditions** | | | | | |
|---|---|---|---|---|---|---|---|---|---|
| | | | | Activation | | Cycling (50x | | | |
| | | | | degrees C | time | degrees C | time | degrees C | time |
| RASSF2 | 83 | 84 | 85 | 95 | 10 mir | 9E | 15sec | 60 | 60 sec |

**Table 4: AUC of colorectal carcinoma detection according to Example 1 with methylation above 0% (single genes).**

| Marker | AUC | Complementarity to 8eptin 9 | Whole Blood |
|---|---|---|---|
| RASSF2 | 0.86 | 0.04 | 0.00 |
| Septin 9 | 0.97 | | 0.04 |

**Table 5: AUC of colorectal carcinoma detection according to Example 1 with methylation within various thresholds (single genes)**

| Marker | CRC detection rate at various methylation thresholds | | |
|---|---|---|---|
| | 10% | 20% | 30% |
| Septin 9 | 0.92 | 0.85 | 0.74 |
| RASSF2 | 0.73 | 0.54 | 0.33 |

**Table 7: Particularly preferred genes and sequences**

| Genomic SEQ ID NO: | Gene | Methylated bisulfite converted sequence (sense) | Methylated bisulfite converted sequence (antisense) | Unmethylated bisulfite converted sequence (sense) | Unmethylated bisulfite converted sequence (antisense) |
|---|---|---|---|---|---|
| 7 | RASSF2 | 27 | 28 | 55 | 56 |

**Table 10: Primer sequences according to Example 2.**

| | Septin 9 | RASSF2 |
|---|---|---|
| Forward primer SEQ ID NO: | 107 | 112 |
| Reverse primer SEQ ID NO: | 108 | 113 |
| Blocker SEQ ID NO: | 109 | 114 |
| Probe SEQ ID NO: | 110 | 115 |
| Probe SEQ ID NO: | 111 | 116 |

| AUC (95% confidence interval) | 0.67(0.61,0.73) | 0.74(0.69,0.79) | 0.64(0.58,0.7) | 0.66(0.6,0.71) | 0.66(0.6,0.72) |
|---|---|---|---|---|---|
| Sens/Spec | 0.37/0.97 | 0.51/0.97 | 0.3/0.97 | 0.35/0.95 | 0.34/0.98 |
| Sens/Spec cut off | -4 | -4 | -4 | -2.599 | -4 |
| Wilcoxon P | 0 | 0 | 0 | 0 | 0 |
| CRC+Adenoma (pos) | 121 | 121 | 113 | 127 | 120 |
| Normal (neg) | 154 | 164 | 146 | 167 | 154 |

## Claims

1. A method for detecting colorectal carcinoma in a subject, comprising determining the expression level of RASSF2 in a biological sample selected from the group consisting of blood plasma, blood serum, whole blood, and blood cells, isolated from said subject, wherein said expression level is determined by detecting the presence or absence of CpG methylation within said gene, wherein the presence of methylation indicates the presence of colorectal carcinoma.

2. The method for detecting colorectal carcinoma in a subject according to claim 1, further comprising determining the expression level of the gene Septin 9, wherein said expression level is determined by detecting the presence or absence of CpG methylation within said gene, wherein the presence of methylation indicates the presence of colorectal carcinoma.

3. A method for detecting colorectal carcinoma in a subject, according to claim 1, comprising contacting genomic DNA isolated from a biological sample selected from the group consisting of blood plasma, blood serum, whole blood, and blood cells, obtained from said subject with at least one reagent, or series of reagents that distinguishes between methylated and non-methylated CpG dinucleotides within at least one target region of the genomic DNA, wherein the at least one target region is a sequence of at least 16 contiguous nucleotides of SEQ ID NO: 7, wherein said contiguous nucleotides comprise at least one CpG dinucleotide sequence, and whereby detecting colorectal carcinoma is, at least in part, afforded.

4. A method for detecting colorectal carcinoma in a subject, comprising contacting genomic DNA isolated from a biological sample obtained from said subject with at least one reagent, or series of reagents that distinguishes between methylated and non-methylated CpG dinucleotides within at least two target regions of the genomic DNA, wherein a first target region is a sequence of at least 16 contiguous nucleotides of at least one sequence selected from the group consisting of SEQ ID NO: 12 to SEQ ID NO: 14 respectively and wherein a second target region is a sequence of at least 16 contiguous nucleotides of SEQ ID NO: 7 respectively, wherein said contiguous nucleotides comprise at least one CpG dinucleotide sequence, and whereby detecting colorectal carcinoma is, at least in part, afforded.

5. A method for detecting colorectal carcinoma, according to claim 1, comprising:
a) extracting or otherwise isolating genomic DNA from a biological sample selected from the group consisting of blood plasma, blood serum, whole blood, and blood cells, obtained from a subject;
b) treating the genomic DNA of a), or a fragment thereof, with one or more reagents, preferably a reagent selected from the group comprising bisulfite, hydrogen sulfite, disulfite, and combinations thereof, to convert cytosine bases that are unmethylated in the 5- position thereof to uracil or to another base that is detectably dissimilar to cytosine in terms of hybridization properties;
c) contacting the treated genomic DNA, or the treated fragment thereof, with an amplification enzyme and at least one primer comprising, a contiguous sequence of at least 9 nucleotides that is complementary to, or hybridizes under moderately stringent or stringent conditions to a sequence selected from the group consisting of SEQ ID NO: 27, 28, 55, 56, and complements thereof, wherein the treated genomic DNA or the fragment thereof is either amplified to produce at least one amplificate, or is not amplified; and
d) determining, based on a presence or absence of, or on a property of said amplificate, the methylation state or level of at least one CpG dinucleotide of SEQ ID NO: 7, or an average, or a value reflecting an average methylation state or level of a plurality of CpG dinucleotides of SEQ ID NO: 7, whereby detecting colorectal carcinoma is, at least in part, afforded.

6. The method of claim 5, wherein contacting or amplifying in c) comprises use of at least one method selected from the group comprising: use of a heat-resistant DNA polymerase as the amplification enzyme; use of a polymerase lacking 5'-3' exonuclease activity; use of a polymerase chain reaction (PCR); generation of an amplificate nucleic acid molecule carrying a detectable label.

7. The method of claim 5, further comprising in step d) the use of at least one nucleic acid molecule or peptide nucleic acid molecule comprising in each case a contiguous sequence of at least 9 nucleotides in length that is complementary to, or hybridizes under moderately stringent or stringent conditions to a sequence selected from the group consisting of SEQ ID NO: 27, 28, 55, 56, and complements thereof, wherein said nucleic acid molecule or peptide nucleic acid molecule suppresses amplification of the nucleic acid to which it is hybridized, or wherein determining in d) comprises hybridization of at least one nucleic acid molecule or peptide nucleic acid molecule in each case comprising a contiguous sequence at least 9 nucleotides in length that is complementary to, or hybridizes under moderately stringent or stringent conditions to a sequence selected from the group consisting of SEQ ID NO: 27, 28, 55, 56, and complements thereof.

8. A method for detecting colorectal carcinoma, according to claim 1, comprising
a) extracting or otherwise isolating genomic DNA from a biological sample selected from the group consisting of blood plasma, blood serum, whole blood, and blood cells, obtained from a subject;
b) digesting the genomic DNA of a), or a fragment thereof, with one or more methylation sensitive restriction enzymes; contacting the DNA restriction enzyme digest of b), with an amplification enzyme and at least two primers suitable for the amplification of a sequence comprising at least one CpG dinucleotide of SEQ ID NO: 7;
c) determining, based on a presence or absence of an amplificate, wherein the presence or absence of an amplificate is preferably determined by means of hybridization to at least one nucleic acid or peptide nucleic acid which is identical, complementary, or hybridizes under stringent or highly stringent conditions to an at least 16 base long segment of SEQ ID NO: 7, the methylation state or level of at least one CpG dinucleotide of SEQ ID NO: 7, whereby detecting colorectal carcinoma is, at least in part, afforded.

9. Use of a treated nucleic acid derived from SEQ ID NO: 7, wherein the treatment is suitable to convert at least one unmethylated cytosine base of the genomic DNA sequence to uracil or another base that is detectably dissimilar to cytosine in terms of hybridization, or use o f a nucleic acid, comprising at least 16 contiguous nucleotides, preferably at least 50 contiguous nucleotides, of a treated genomic DNA sequence selected from the group consisting of SEQ ID NO: 27, 28, 55, 56, and sequences complementary thereto, wherein said nucleic acid comprises at least one base which has been converted from an unmethylated cytosine base of the genomic DNA sequence to uracil or another base that is detectably dissimilar to cytosine in terms of hybridization, wherein said nucleic acid comprises at least one CpG, TpG or CpA dinucleotide sequence, or use of a nucleic acid, comprising a DNA sequence selected from the group consisting of SEQ ID NO: 27, 28, 55, 56, and sequences complementary thereto, in a method according to any of claims 1 to 8

10. A kit suitable for performing the method according to any of claims 3 to 5, comprising (a) a bisulfite reagent; (b) a container suitable for containing said bisulfite reagent and the biological sample of the patient; (c) at least one set of oligonucleotides containing two oligonucleotides whose sequences in each case are identical, are complementary, or hybridize under stringent or highly stringent conditions to a 9 or more preferably 18 base long segment of a sequence selected from SEQ ID NO: 27, 28, 55, and 56, wherein said kit further comprises at least one set of oligonucleotides containing two oligonucleotides whose sequences in each case are identical, are complementary, or hybridize under stringent or highly stringent conditions to a 9 or more preferably 18 base long segment of a sequence selected from SEQ ID NO: 37 to SEQ ID NO: 42 and SEQ ID NO: 65 to SEQ ID NO: 70, or a kit suitable for performing the method according to claim 8, comprising (a) a methylation sensitive restriction enzyme reagent; (b) a container suitable for containing said reagent and the biological sample of the patient; (c) at least one set of oligonucleotides or one or a plurality of nucleic acids or peptide nucleic acids which are identical, are complementary, or hybridize under stringent or highly stringent conditions to an at least 9 base long segment of a sequence selected from SEQ ID NO: 7; and optionally (d) instructions for use and interpretation of the kit results, wherein said kit further comprises (e) at least one set of oligonucleotides or one or a plurality of nucleic acids or peptide nucleic acids which are identical, are complementary, or hybridize under stringent or highly stringent conditions to an at least 9 base long segment of a sequence selected from SEQ ID NO: 12 to SEQ ID NO: 14.

11. Use of a kit according to claim 10 in the diagnosis of colorectal cancer.

## Patentansprüche

1. Ein Verfahren zur Detektion von Kolorektalkarzinom in einem Subjekt, welches die Bestimmung des Expressionslevels von RASSF2 in einer biologischen Probe umfasst, ausgewählt aus der Gruppe bestehend aus Blutplasma, Blutserum, Vollblut, und Blutzellen, isoliert aus besagtem Subjekt, wobei besagtes Expressionslevel durch die Detektion der Anwesenheit oder Abwesenheit der CpG Methylierung in besagtem Gen bestimmt wird, wobei die Anwesenheit der Methylierung die Anwesenheit des Kolorektalkarzinoms indiziert.

2. Verfahren zur Detektion von Kolorektalkarzinom in einem Subjekt gemäß Anspruch 1, welches weiterhin die Bestimmung des Expressionslevels des Gens Septin 9 umfasst, wobei besagtes Expressionslevel durch die Detektion der Anwesenheit oder Abwesenheit der CpG Methylierung in besagtem Gen bestimmt wird, wobei die Anwesenheit der Methylierung die Anwesenheit des Kolorektalkarzinoms indiziert.

3. Verfahren zur Detektion von Kolorektalkarzinom in einem Subjekt gemäß Anspruch 1, welches das in Kontakt bringen genomischer DNA umfasst, welche aus einer biologischen Probe isoliert ist ausgewählt aus der Gruppe bestehend aus Blutplasma, Blutserum, Vollblut und Blutzellen, und welche von besagtem Subjekt erhalten wurde, mit mindestens einem Reagenz oder einer Serie von Reagenzien, welches zwischen methylierten und nicht-methylierten CpG Dinukleotiden in mindestens einer Zielregion der genomischen DNA unterscheidet, wobei die mindestens eine Zielregion eine Sequenz von mindestens 16 kontinuierlichen Nukleotiden der SEQ ID NO: 7 ist, wobei besagte kontinuierliche Nukleotide mindestens eine CpG Dinukleotidsequenz umfassen und wobei die Detektion des Kolorektalkarzinoms zumindest teilweise gewährleistet ist.

4. Verfahren zur Detektion von Kolorektalkarzinom in einem Subjekt, welches das in Kontakt bringen genomischer DNA umfasst, welche aus einer biologischen Probe isoliert ist, die von besagtem Subjekt erhalten wurde, mit mindestens einem Reagenz oder einer Serie von Reagenzien, welches zwischen methylierten und nicht-methylierten CpG Dinukleotiden in mindestens zwei Zielregionen der genomischen DNA unterscheidet, wobei eine erste Zielregion eine Sequenz von mindestens jeweils 16 kontinuierlichen Nukleotiden ist, die aus mindestens einer Sequenz aus der Gruppe bestehend aus SEQ ID NO: 12 bis SEQ ID NO: 14 ausgewählt ist, und wobei eine zweite Zielregion eine Sequenz von mindestens jeweils 16 kontinuierlichen Nukleotiden von SEQ ID NO: 7 ist, wobei besagte kontinuierliche Nukleotide mindestens eine CpG Dinukleotidsequenz umfassen und wobei die Detektion des Kolorektalkarzinoms zumindest teilweise gewährleistet ist.

5. Verfahren zur Detektion von Kolorektalkarzinom gemäß dem Anspruch 1, umfassend:
a) Extrahieren oder in anderer Weise Isolieren genomischer DNA aus einer biologischen Probe ausgewählt aus der Gruppe bestehend aus Blutplasma, Blutserum, Vollblut, und Blutzellen, welche von einem Subjekt erhalten wurden;
b) Behandeln der genomischen DNA aus a) oder eines Fragments dieser mit einem oder mehreren Reagenzien, vorzugsweise ein Reagenz ausgewählt aus der Gruppe bestehend aus Bisulfit, Hydrogensulfit, Disulfit und Kombinationen dieser, um Cytosinbasen, welche in ihrer 5'-Position unmethyliert sind, in Uracil oder in eine andere Base, welche in Bezug auf die Hybridisierungseigenschaften detektierbar unähnlich zu Cytosin ist, umzuwandeln;
c) in Kontakt bringen der behandelten genomischen DNA oder des behandelten Fragments dieser mit einem Amplifikationsenzym und mindestens einem Primer, welcher eine kontinuierliche Sequenz von mindestens 9 Nukleotiden umfasst, welche komplementär ist oder unter moderat stringenten oder stringenten Bedingungen mit einer Sequenz ausgewählt aus der Gruppe bestehend aus SEQ ID NO: 27, 28, 55, 56 und Komplementärsequenzen davon hybridisiert, wobei die behandelte genomische DNA oder das Fragment dieser entweder amplifiziert wird, um mindestens ein Amplifikat zu produzieren oder nicht ampfliziert wird; und
d) Bestimmen, basierend auf der Anwesenheit oder Abwesenheit oder auf einer Eigenschaft von besagtem Amplifikat, des Methylierungstatus oder -levels von mindestens einem CpG Dinukleotid der SEQ ID NO: 7 oder einen Mittelwert oder einen Wert, welcher einen durchschnittlichen Methylierungsstatus oder -level einer Vielzahl von CpG Dinukleotiden der SEQ ID NO: 7 reflektiert, wobei die Detektion von Kolorektalkarzinom mindestens teilweise gewährleistet ist.

6. Verfahren gemäß Anspruch 5, wobei in Kontakt bringen oder Amplifizieren in c) die Verwendung von mindestens einem Verfahren umfasst, ausgewählt aus der Gruppe, umfassend die Verwendung von einer hitzeresistenten DNA Polymerase als das Amplifizierungsenzym, Verwendung von einer Polymerase, die keine 5'-3' Exonuklease Aktivität aufweist; Verwendung von einer Polymerase Kettenreaktion (PCR); Generieren von einem Amplifikat Nukleinsäuremolekül, welches ein detektierbares Label trägt.

7. Verfahren gemäß Anspruch 5, weiterhin umfassend in Schritt d) die Verwendung von mindestens einem Nukleinsäurenmolekül oder Peptid-Nukleinsäuremolekül, welches in jedem Fall eine kontinuierliche Sequenz von mindestens 9 Nukleotiden in Länge umfasst, welche komplementär ist oder unter moderat stringenten oder stringenten Bedingungen an eine Sequenz, ausgewählt aus der Gruppe bestehend aus SEQ ID NO: 27, 28, 55, 56 und Komplementärsequenzen dieser hybridisiert, wobei besagtes Nukleinsäurenmolekül oder Peptid-Nukleinsäuremolekül die Amplifikation der Nukleinsäure, mit welcher es hybridisiert ist, unterdrückt, oder wobei das Bestimmen in d) die Hybridisierung von mindestens einem Nukleinsäuremolekül oder Peptid-Nukleinsäurenmolekül umfasst, welches in jedem Fall eine kontinuierliche Sequenz von mindestens 9 Nukleotiden in Länge umfasst, welche komplementär ist oder unter moderat stringenten oder stringenten Bedingungen an eine Sequenz ausgewählt aus der Gruppe bestehend aus SEQ ID NO: 27, 28, 55, 56 und Komplementärsequenzen dieser hybridisiert.

8. Verfahren zur Detektion von Kolorektalkarzinom gemäß Anspruch 1, umfassend
a) Extrahieren oder in anderer Weise Isolieren genomischer DNA aus einer biologischen Probe ausgewählt aus der Gruppe bestehend aus Blutplasma, Blutserum, Vollblut und Blutzellen, welche von einem Subjekt erhalten wurde;
b) Verdauen der genomischen DNA aus a) oder eines Fragments dieser mit einem oder mehreren methylierungssensitiven Restriktionsenzymen; in Kontakt bringen des DNA Restriktionsenzymverdaus aus b) mit einem Amplifikationsenzym und mindestens zwei Primern, die zur Amplifikation einer Sequenz geeignet sind, die mindestens ein CpG Dinukleotid von SEQ ID NO: 7 umfasst;
c) Bestimmen, basierend auf der Anwesenheit oder Abwesenheit von einem Amplifikat, wobei die Anwesenheit oder Abwesenheit von einem Amplifikat bevorzugt bestimmt wird durch Mittel der Hybridisierung an mindestens eine Nukleinsäure oder Peptidnukleinsäure, welche identisch ist, komplementär ist, oder unter stringenten oder hoch stringenten Bedingungen an ein mindestens 16 basenlanges Segment von SEQ ID NO: 7 hybridisiert, des Methylierungsstatus oder -levels von mindestens einem CpG Dinukleotid der SEQ ID NO: 7, wobei die Detektion von Kolorektalkarzinom zumindest teilweise gewährleistet ist.

9. Verwendung von einer behandelten Nukleinsäure, abstammend von SEQ ID NO: 7, wobei die Behandlung geeignet ist, mindestens eine unmethylierte Cytosinbase der genomischen DNA Sequenz in Uracil oder eine anderen Base, welche in Bezug auf die Hybridisierung detektierbar unähnlich zu Cytosin ist, umzuwandeln oder Verwendung von einer Nukleinsäure, welche mindestens 16 kontinuierliche Nukleotide, vorzugsweise mindestens 50 kontinuierlichen Nukleotide von einer behandelten genomischen DNA Sequenz umfasst, welche ausgewählt ist aus der Gruppe bestehend aus SEQ ID NO: 27, 28, 55, 56 und hierzu komplementären Sequenzen, wobei besagte Nukleinsäure mindestens eine Base umfasst, welche von einer unmethylierten Cytosin Base der genomischen DNA Sequenz in Uracil oder eine andere Base umgewandelt worden ist, die in Bezug auf die Hybridisierung detektierbar unähnlich zu Cytosin ist, wobei besagte Nukleinsäure mindestens eine CpG, TpG oder CpA Dinukleotidsequenz umfasst, oder Verwendung von einer Nukleinsäure, welche eine DNA Sequenz umfasst, ausgewählt aus der Gruppe bestehend aus SEQ ID NO: 27, 28, 55, 56 und dazu komplementären Sequenzen in einem Verfahren gemäß einem der Ansprüche 1 bis 8.

10. Ein Kit, geeignet das Verfahren gemäß einem der Ansprüche 3-5 durchzuführen, umfassend
(a) ein Bisulfit Reagenz,
(b) einen Behälter, geeignet um besagtes Bisulfit Reagenz und die biologische Probe des Patienten zu beinhalten;
(c) mindestens ein Set von Oligonukleotiden, welches zwei Oligonukleotide beinhaltet, deren Sequenzen in jedem Falle identisch sind oder komplementär sind oder unter stringenten oder hoch stringenten Bedingungen an ein 9, oder bevorzugter an ein 18 Basen langes Segment einer Sequenz ausgewählt aus SEQ ID NO: 27, 28 55 und 56 hybridisiert, wobei besagtes Kit weiterhin mindestens ein Set von Oligonukleotiden umfasst, welches zwei Oligonukleotide, deren Sequenzen in jedem Falle identisch oder komplementär sind oder unter stringenten oder hoch stringenten Bedingungen zu einem 9 oder bevorzugter 18 basenlangen Segment einer Sequenz ausgewählt aus SEQ ID NO: 37 bis SEQ ID NO: 42 und SEQ ID NO: 65 bis SEQ ID NO: 70 hybridisiert, oder ein Kit geeignet zur Durchführung des Verfahrens gemäß Anspruch 8, umfassend
(a) ein methylierungssensitives Restriktionsenzym Reagenz;
(b) einen Behälter, der geeignet ist, um besagtes Reagenz und die biologische Probe des Patienten zu beinhalten;
(c) mindestens ein Set von Oligonukleotiden oder einer oder einer Vielzahl von Nukleinsäuren oder Peptidnukleinsäuren, welche identisch sind oder komplementär sind oder unter stringenten oder hoch stringenten Bedingungen an ein mindestens 9 Basen langes Segment einer Sequenz ausgewählt aus SEQ ID NO: 7 hybridisieren, und optional
(d) Instruktionen zur Verwendung und Interpretation der Ergebnisse des Kits, wobei besagtes Kit weiterhin umfasst
(e) mindestens ein Set von Oligonukleotiden oder einer oder einer Vielzahl von Nukleinsäuren oder Peptidnukleinsäuren, welche identisch sind oder komplementär sind oder unter stringenten oder hoch stringenten Bedingungen an ein mindestens 9 Basen langes Segment einer Sequenz ausgewählt aus SEQ ID NO: 12 bis SEQ ID NO: 14 hybridisieren.

11. Verwendung eines Kits gemäß Anspruch 10 in der Diagnose von Kolorektalkrebs.

## Revendications

1. Procédé pour la détection de carcinome colorectal chez un sujet, comprenant la détermination du niveau d'expression de RASSF2 dans un échantillon biologique choisi dans le groupe consistant en plasma sanguin, sérum sanguin, sang total, et cellules sanguines, isolé à partir dudit sujet, dans lequel ledit niveau d'expression est déterminé par détection de la présence ou de l'absence d'une méthylation de CpG dans ledit gène, tandis que la présence de méthylation indique la présence de carcinome colorectal.

2. Procédé pour la détection de carcinome colorectal chez un sujet selon la revendication 1, comprenant en outre la détermination du niveau d'expression du gène Septine 9, tandis que ledit niveau d'expression est déterminé par détection de la présence ou de l'absence d'une méthylation de CpG dans ledit gène, tandis que la présence de méthylation indique la présence de carcinome colorectal.

3. Procédé pour la détection de carcinome colorectal chez un sujet selon la revendication 1, comprenant la mise en contact d'ADN génomique isolé à partir d'un échantillon biologique choisi dans le groupe consistant en plasma sanguin, sérum sanguin, sang total, et cellules sanguines, obtenu à partir dudit sujet avec au moins un réactif, ou série de réactifs qui fait la distinction entre des dinucléotides CpG méthylés et non-méthylés dans au moins une région cible de l'ADN génomique, tandis que la au moins une région cible est une séquence d'au moins 16 nucléotides contigus selon SEQ ID NO: 7, dans laquelle lesdits nucléotides contigus comprennent au moins une séquence dinucléotidique CpG, et ce par quoi la détection de carcinome colorectal est, au moins en partie procurée.

4. Procédé pour la détection de carcinome colorectal chez un sujet, comprenant la mise en contact d'ADN génomique isolé à partir d'un échantillon biologique obtenu à partir dudit sujet avec au moins un réactif, ou série de réactifs qui fait la distinction entre des dinucléotides CpG méthylés et non-méthylés dans au moins deux régions cibles de l'ADN génomique, tandis qu'une première région cible est une séquence d'au moins 16 nucléotides contigus selon au moins une séquence choisie dans le groupe consistant en SEQ ID NO: 12 à SEQ ID NO: 14 respectivement et tandis qu'une deuxième région cible est une séquence d'au moins 16 nucléotides contigus selon SEQ ID NO: 7 respectivement, lesdits nucléotides contigus comprenant au moins une séquence dinucléotidique CpG, et ce par quoi la détection de carcinome colorectal est, au moins en partie, procurée.

5. Procédé pour la détection de carcinome colorectal selon la revendication 1, comprenant:
a) l'extraction ou l'isolement d'une autre manière d'ADN génomique provenant d'un échantillon biologique choisi dans le groupe consistant en plasma sanguin, sérum sanguin, sang total, et cellules sanguines, obtenu à partir d'un sujet;
b) le traitement de l'ADN génomique issu de a), ou d'un fragment de celui-ci, avec un ou plusieurs réactifs, de préférence un réactif choisi dans le groupe comprenant bisulfite, hydrogéno sulfite, disulfite, et leurs combinaisons, pour convertir les bases cytosine qui sont non-méthylées dans leur position 5 en uracile ou en une autre base qui est détectable comme n'étant pas similaire à la cytosine en termes de propriétés d'hybridation;
c) la mise en contact de l'ADN génomique traité, ou du fragment traité de celui-ci, avec une enzyme d'amplification et au moins une amorce comprenant une séquence contiguë d'au moins 9 nucléotides qui est complémentaire de, ou hybride dans des conditions modérément stringentes ou stringentes à une séquence choisie dans le groupe consistant en SEQ ID NO: 27, 28, 55, 56, et leurs compléments, tandis que l'ADN génomique traité ou le fragment de celui-ci soit est amplifié pour produire au moins un produit d'amplification, soit n'est pas amplifié; et
d) la détermination, fondée sur une présence ou une absence de, ou sur une propriété dudit produit d'amplification, du stade ou niveau de méthylation d'au moins un dinucléotide CpG selon SEQ ID NO: 7, ou d'une moyenne, ou d'une valeur reflétant une moyenne du stade ou niveau de méthylation d'une pluralité de dinucléotides CpG selon SEQ ID NO: 7, ce par quoi la détection de carcinome colorectal est, au moins en partie, procurée.

6. Procédé selon la revendication 5, dans lequel la mise en contact ou l'amplification selon c) comprend l'utilisation d'au moins un procédé choisi dans le groupe comprenant: l'utilisation d'une ADN polymérase résistante à la chaleur à titre d'enzyme d'amplification; l'utilisation d'une polymérase exempte d'activité de 5'-3' exonucléase; l'utilisation d'une amplification en chaîne par polymérase (ACP); la génération d'une molécule d'acide nucléique en tant que produit d'amplification, portant un marqueur détectable.

7. Procédé selon la revendication 5, comprenant en outre dans l'étape d) l'utilisation d'au moins une molécule d'acide nucléique ou une molécule d'acide nucléique peptidique comprenant dans chaque cas une séquence contigue d'au moins 9 nucléotides de long qui est complémentaire de, ou hybride dans des conditions modérément stringentes ou stringentes à une séquence choisie dans le groupe consistant en SEQ ID NO: 27, 28, 55, 56, et leurs compléments, tandis que ladite molécule d'acide nucléique ou molécule d'acide nucléique peptidique empêche l'amplification de l'acide nucléique auquel elle est hybridée, ou tandis que la détermination sous d) comprend l'hybridation d'au moins une molécule d'acide nucléique ou molécule d'acide nucléique peptidique comprenant dans chaque cas une séquence contiguë d'au moins 9 nucléotides de long qui est complémentaire de, ou hybride dans des conditions modérément stringentes ou stringentes à une séquence choisie dans le groupe consistant en SEQ ID NO: 27, 28, 55, 56, et leurs compléments.

8. Procédé pour la détection de carcinome colorectal selon la revendication 1, comprenant
a) l'extraction ou l'isolement d'une autre manière d'ADN génomique provenant d'un échantillon biologique choisi dans le groupe consistant en plasma sanguin, sérum sanguin, sang total, et cellules sanguines, obtenu à partir d'un sujet;
b) la digestion de l'ADN génomique issu de a), ou d'un fragment de celui-ci, avec une ou plusieurs enzymes de restriction sensibles à la méthylation; la mise en contact du produit de digestion de l'enzyme de restriction d'ADN selon b), avec une enzyme d'amplification et au moins deux amorces convenant pour l'amplification d'une séquence comprenant au moins un dinucléotide CpG selon SEQ ID NO: 7;
c) la détermination, fondée sur une présence ou une absence d'un produit d'amplification, tandis que la présence ou l'absence d'un produit d'amplification est de préférence déterminée au moyen de l'hybridation à au moins un acide nucléique ou un acide nucléique peptidique qui est identique à, complémentaire de, ou hybride dans des conditions stringentes ou fortement stringentes à un segment d'au moins 16 bases de long de SEQ ID NO: 7, du stade ou niveau de méthylation d'au moins un dinucléotide CpG selon SEQ ID NO: 7, ce par quoi la détection de carcinome colorectal est, au moins en partie, réalisée.

9. Utilisation d'un acide nucléique traité dérivé de SEQ ID NO: 7, dans laquelle le traitement est approprié pour convertir au moins une base cytosine non-méthylée de la séquence d'ADN génomique en uracile ou en une autre base qui est détectée comme n'étant pas similaire à la cytosine en termes d'hybridation, ou utilisation d'un acide nucléique comprenant au moins 16 nucléotides contigus, de préférence au moins 50 nucléotides contigus, d'une séquence d'ADN génomique traité choisie dans le groupe consistant en SEQ ID NO: 27, 28, 55, 56, et des séquences complémentaires de celles-ci, tandis que ledit acide nucléique comprend au moins une base qui a été convertie à partir d'une base cytosine non-méthylée de la séquence d'ADN génomique en uracile ou en une autre base qui est détectée comme non-similaire à la cytosine en termes d'hybridation, ledit acide nucléique comprenant au moins une séquence de dinucléotide CpG, TpG ou CpA, ou utilisation d'un acide nucléique comprenant une séquence d'ADN choisie dans le groupe consistant en SEQ ID NO: 27, 28, 55, 56, et des séquences complémentaires de celles-ci,
dans un procédé selon l'une quelconque des revendications 1 à 8.

10. Kit convenant pour la mise en oeuvre du procédé selon l'une quelconque des revendications 3 à 5, comprenant (a) un réactif au bisulfite; (b) un récipient approprié pour contenir ledit réactif au bisulfite et l'échantillon biologique du patient; (c) au moins un ensemble d'oligonucléotides contenant deux oligonucléotides dont les séquences dans chaque cas sont identiques, sont complémentaires, ou hybrident dans des conditions stringentes ou fortement stringentes à un segment de 9 ou de préférence de 18 bases de long d'une séquence choisie parmi SEQ ID NO: 27, 28, 55 et 56, tandis que ledit kit comprend en outre au moins un ensemble d'oligonucléotides contenant deux oligonucléotides dont les séquences dans chaque cas sont identiques, sont complémentaires, ou hybrident dans des conditions stringentes ou fortement stringentes à un segment de 9 ou plus préférablement de 18 bases de long d'une séquence choisie parmi SEQ ID NO: 37 à SEQ ID NO: 42 et SEQ ID NO: 65 à SEQ ID NO: 70, ou kit convenant pour la mise en oeuvre du procédé selon la revendication 8, comprenant (a) un réactif à base d'enzyme de restriction sensible à la méthylation; (b) un récipient convenant pour contenir ledit réactif et l'échantillon biologique du patient; (c) au moins un ensemble d'oligonucléotides ou un ou une pluralité d'acides nucléiques ou d'acides nucléiques peptidiques qui sont identiques, sont complémentaires, ou hybrident dans des conditions stringentes ou fortement stringentes à un segment d'au moins 9 bases de long d'une séquence choisie parmi SEQ ID NO: 7; et en option (d) des instructions pour l'utilisation et l'interprétation des résultats du kit, tandis que ledit kit comprend en outre (e) au moins un ensemble d'oligonucléotides ou un ou une pluralité d'acides nucléiques ou d'acides nucléiques peptidiques qui sont identiques, sont complémentaires, ou hybrident dans des conditions stringentes ou fortement stringentes à un segment d'au moins 9 bases de long d'une séquence choisie parmi SEQ ID NO: 12 à SEQ ID NO: 14.

11. Utilisation d'un kit selon la revendication 10 dans le diagnostic du cancer colorectal.
